# EUROPEAN PATENT APPLICATION

(11) **EP 4 692 072 A1**
(43) Date of publication of application: **11.02.2026**
(21) Application number: 23930719.2
(22) Date of filing: 05.07.2023
(51) Int. Cl.: C07D 311/04, C07C 217/76, C07C 217/94, C07D 311/78, C07D 405/04, C07D 407/04, C07D 413/04, C07F 7/10, C09K 9/02, G02C 7/02, G02C 7/10

(54) **PHOTOCHROMIC COMPOUND, PROPARGYL ALCOHOL COMPOUND, CURABLE COMPOSITION, OPTICAL ARTICLE, LENS, AND SPECTACLES**

(30) Priority: 27.03.2023 JP 2023049959; 09.05.2023 JP 2023077412; 17.05.2023 JP 2023081196
(71) Applicant: TOKUYAMA CORPORATION, Yamaguchi 745-8648 (JP)
(72) Inventor: MIYAZAKI, Masayuki, Shunan-shi, Yamaguchi 745-8648 (JP)
(74) Representative: Papula Oy
(86) International application number: PCT/JP2023/024970
(87) International publication number: WO 2024/202087

(57) **Abstract**

The purpose of the present invention is to provide: a photochromic compound having an excellent color fading speed and excellent durability; a propargyl alcohol compound that can serve as an intermediate for this photochromic compound; and a curable composition, an optical article, a lens, and spectacles each including this photochromic compound. Provided according to embodiments is a photochromic compound having a skeleton represented by formula (1). In formula (1), R³ is an aryl group or a heteroaryl group substituted with a group represented by formula (1a). In formula (1a), R¹ is a substituted or unsubstituted aryl group, a substituted or unsubstituted heteroaryl group, or a substituted or unsubstituted fused polycycle in which an aromatic ring or an aromatic heterocycle has been fused to these substituents. R² is a hydrogen atom, a halogen atom, a substituted or unsubstituted alkyl group, a substituted or unsubstituted haloalkyl group, a substituted or unsubstituted cycloalkyl group, a group represented by formula (2a), or the like.

## Description

### TECHNICAL FIELD

The present invention relates to a photochromic compound, a propargyl alcohol compound, a curable composition, an optical article, a lens, and eyeglasses.

### BACKGROUND ART

Photochromic compounds refer to compounds that can reversibly become two types of isomers with different absorption spectra by irradiation with light including ultraviolet ray, such as sunlight or mercury lamp light. Generally, when a colorless compound in a decolored state is irradiated with ultraviolet ray, the compound rapidly changes in color and isomerizes into a colored state (chromogenic reaction). Photochromic compounds have been researched and developed as materials for photochromic lenses.

In applications for such photochromic lenses, photochromic compounds require the following properties in some cases.
(I) Low coloring degree in the visible light region before UV irradiation (hereinafter referred to as "initial coloring").
(II) High rate at which the color optical density reaches saturation after the start of UV irradiation (hereinafter referred to as "color optical density").
(III) High rate at which the color optical density reaches saturation after the start of UV irradiation (hereinafter also referred to as "high chromogenic sensitivity").
(IV) High rate at which the compound returns to its original state after the stop of UV irradiation (hereinafter referred to as "color fading speed").
(V) High durability against the aforementioned repeated reversible actions.
(VI) High solubility in the lens matrix material, and high dispersibility in a cured product.

As photochromic compounds that can satisfy the above properties, various types of chromene compounds have been studied. For example, a chromene compound represented by formula (A) below (Patent Document 1), a chromene compound represented by formula (B) below (Patent Document 2), and a chromene compound represented by formula (C) below (Patent Document 3) are known.

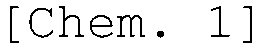

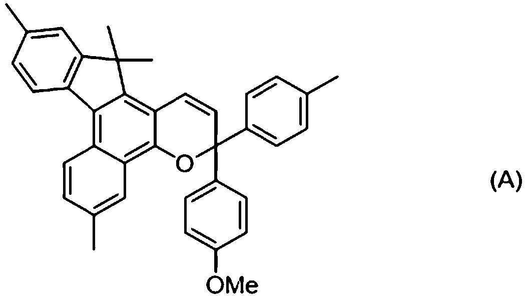

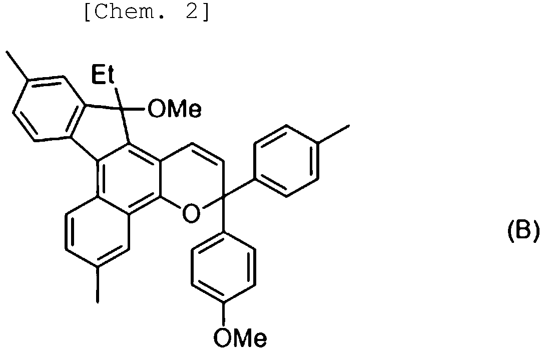

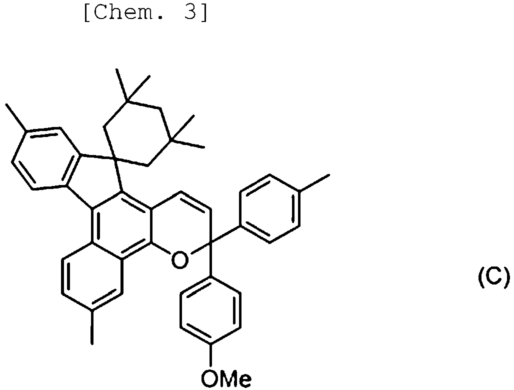

### Citation List

### Patent Documents

Patent Document 1: PCT International Publication No. WO 1996/014596
Patent Document 2: PCT International Publication No. WO 2004/085568
Patent Document 3: PCT International Publication No. WO 2001/060811
Patent Document 4: PCT International Publication No. WO 2015/035325
Patent Document 5: PCT International Publication No. WO 2007/078529
Patent Document 6: PCT International Publication No. WO 2021/075456
Patent Document 7: PCT International Publication No. WO 2018/235771
Patent Document 8: PCT International Publication No. WO 2012/105410
Patent Document 9: PCT International Publication No. WO 2011/053615
Patent Document 10: PCT International Publication No. WO 2013/042800
Patent Document 11: PCT International Publication No. WO 2019/228604
Patent Document 12: PCT International Publication No. WO 2019/013249
Patent Document 13: PCT International Publication No. WO 2016/143910

### Non-Patent Documents

Non-Patent Document 1: Journal of Organic Chemistry, 69(10), pp. 3282-3293; 2004
Non-Patent Document 2: Synthetic Communications, 23(16), pp. 2241-2249 (1993)
Non-Patent Document 3: J. Am. Chem. Soc., 132(41), pp. 14324-14326 (2010)
Non-Patent Document 4: Org. Lett., 16, pp. 6492-6495 (2014)

### DISCLOSURE OF THE INVENTION

### Problems to be Solved by the Invention

An object of the present invention is to provide a photochromic compound excellent in color fading speed and durability, a propargyl alcohol compound that can serve as an intermediate of this photochromic compound, as well as a curable composition, an optical article, a lens, and eyeglasses including this photochromic compound.

### Means for Solving the Problems

The present disclosure relates to a photochromic compound having a skeleton represented by formula (1) below.

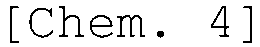

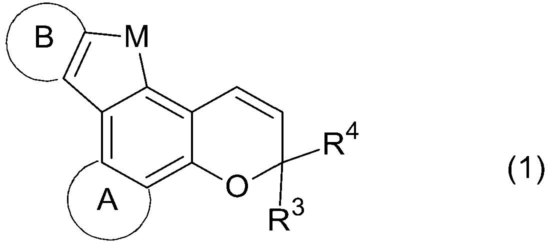

In formula (1) above, M is C, Si, or Ge.

R³ is an aryl group or a heteroaryl group substituted with a group represented by formula (1a) below.

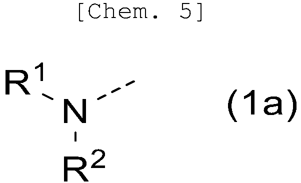

In formula (1a) above, R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents. R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below.

-Q¹-(X¹Q²)a-X²Q³ (2a)

In formula (2a) above, Q¹ is an alkylene group or a haloalkylene group. Q² is an alkylene group or a haloalkylene group. Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group. a is an integer of 0, or 1 or more and 3 or less.

R⁴ is a substituted or unsubstituted aryl group or a substituted or unsubstituted heteroaryl group.

Ring A and ring B are each independently a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Also, the present disclosure relates to a curable composition. The curable composition includes the photochromic compound described above, and at least one selected from a group consisting of a radical polymerizable monomer, a cationic polymerizable monomer, a compound having a polymerizable group, and a (thio)urethane(urea) polymer.

Also, the present disclosure relates to an optical article. The optical article includes the above-described cured product of the curable composition.

Also, the present disclosure relates to a lens. The lens includes the above-described photochromic compound.

Also, the present disclosure relates to eyeglasses. The eyeglasses include the above-described lens.

Also, the present disclosure relates to a propargyl alcohol compound. The propargyl alcohol compound has a skeleton represented by formula (9) below.

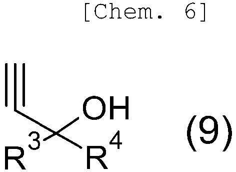

In formula (9), R³ and R⁴ are each independently the same as those in formula (1) above.

### Effects of the Invention

The present invention provides a photochromic compound excellent in color fading speed and durability, a propargyl alcohol compound that can serve as an intermediate of this photochromic compound, as well as a curable composition, an optical article, a lens, and eyeglasses including this photochromic compound.

### PREFERRED MODE FOR CARRYING OUT THE INVENTION

### [Photochromic Compound]

According to the embodiment, a photochromic compound having a skeleton represented by formula (1) below is provided.

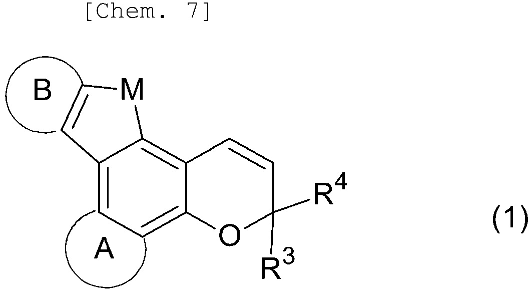

In formula (1), M is C, Si, or Ge.

R³ is an aryl group or heteroaryl group substituted with a group represented by formula (1a) below. R⁴ is a substituted or unsubstituted aryl group or a substituted or unsubstituted heteroaryl group. Rings A and B are each independently a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

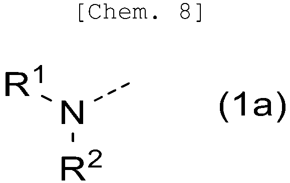

In formula (1a) above, R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents.

R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below.

-Q¹-(X¹Q²)a-X²Q³ (2a)

In formula (2a) above,
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰, PR⁷⁰¹, or P(=O) , R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
a is an integer of 0, or 1 or more and 3 or less,
This photochromic compound is excellent in excellent in color fading speed and durability. Although the reason for this is not yet clear, the inventors consider that the reason is as follows.

First, in the photochromic compound having the skeleton represented by formula (1), R³ is a substituted aryl or substituted heteroaryl. The inventors have found that this substituent significantly affects the color fading speed. That means, the stronger the electron donation from R³, the lower the thermal stability of the color developing structure when the photochromic compound is photoisomerized by light, and therefore the photochromic compound is prone to fading, resulting in a high color fading speed. However, organic compounds with electron-donating substituents generally tend to be susceptible to oxidation due to abundant electrons in their molecules. In particular, photochromic compounds have a tendency that the repetitive durability tends to decrease by oxidation. For example, photochromic compounds having a substituent with a high electron-donating property such as substituted amino groups or nitrogen-containing heterocyclic rings exhibit excellent color fading speed but have low repetitive durability, and therefore it has been difficult for photochromic compounds to achieve both color fading speed and repetitive durability in many cases.

The substituent R³ of the photochromic compound according to the embodiment has the substituent represented by formula (1a) above. Formula (1a) above has a substituted amino group structure with an aryl group or heteroaryl group directly bonded to one bonding hand of a nitrogen atom, and with a group other than aryl groups or heteroaryl groups, or a hydrogen atom, bonded to the other bonding hand. Since an aryl group or heteroaryl group is directly bonded to one bonding hand of a nitrogen atom, it is considered that formula (1a) can be conjugated with the substituted aryl group or heteroaryl group via an orbit p on a nitrogen atom. Compounds having such a structure are considered to have extended conjugation compared to compounds having no amino group with substituted aryl groups or heteroaryl groups. It is considered that, since this extension delocalizes electrons in a color developer because of a resonance structure, oxidation is hardly caused and the repetitive durability can be improved. Moreover, such a compound is expected to be excellent in both color fading speed and repetitive durability because its conjugation is not excessively extended compared to a case that R³ has, as a substituent, an amino group having a diaryl group.

Based on the above description, use of the photochromic compound according to the embodiment makes it possible to achieve a cured product excellent in color fading speed and furthermore repetitive durability. Thus, such a photochromic compound is suitable for optical articles such as photochromic eyeglasses that are used for a long period of time.

The photochromic compound having the skeleton represented by formula (1) will be explained below in detail.

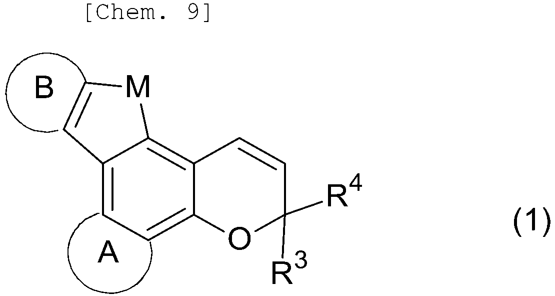

### <M>

In formula (1), M is C, Si, or Ge. Preferably, M is C.

### <Ring A and Ring B>

In formula (1), ring A and ring B are each independently a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Examples of the aromatic hydrocarbon ring include a benzene ring, a cyclotetradecaheptaene ring, and the like.

Examples of the aromatic heterocyclic ring include a furan ring, a thiophene ring, a pyridine ring, and the like.

Examples of the fused polycylic ring include a naphthalene ring, a fluorene ring, an anthracene ring, a phenanthrene ring, a tetracene ring, a pentacene ring, a benzopyrene ring, a chrysene ring, a pyrene ring, a triphenylene ring, a perylene ring, a benzofuran ring, a benzothiophene ring, a quinoline ring, an isoquinoline ring, an indole ring, a pyrimidine ring, a quinazoline ring, a pyridazine ring, a cinnoline ring, a phthalazine ring, a 1,2,3-, 1,2,4-, or 1,3,5-triazine ring, a carbazole ring, a benzoxazole ring, an isothiazole ring, and the like.

Above all, a benzene ring, a naphthalene ring, a fluorene ring, a phenanthrene ring, a pyrene ring, a furan ring, a thiophene ring, or a pyridine ring are preferable, a benzene ring, a naphthalene ring, a fluorene ring, or a phenanthrene ring are more preferable, and a benzene ring is most preferable.

### <R³>

In formula (1), R³ is an aryl group or heteroaryl group substituted with a group represented by formula (1a) below. The dashed line indicates a hand bonded to a carbon atom of the aryl group or heteroaryl group. R³ is preferably an aryl group substituted by one group represented by formula (1a) below, more preferably a phenyl group substituted with one group represented by formula (1a) below.

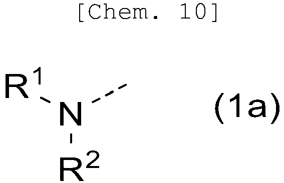

### (R¹)

R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents. The substituent that may be included in a group that may be R¹ will be described later.

The number of carbon atoms in the substituted or unsubstituted aryl group is e.g. 5 or more and 12 or less, and preferably 6 or more and 10 or less. The heteroatom of the substituted or unsubstituted heteroaryl group is at least one selected from a group consisting of oxygen atom, sulfur atom, nitrogen atom, and phosphorus atom. The number of heteroatoms in the substituted or unsubstituted heteroaryl group is e.g. 1 or more and 3 or less, and preferably 1 or 2. The number of carbon atoms in the substituted or unsubstituted heteroaryl group is e.g. 4 or more and 11 or less, and preferably 5 or more and 9 or less.

R¹ is preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group, more preferably a substituted or unsubstituted phenyl group, a substituted or unsubstituted 1-naphthyl group, or a substituted or unsubstituted 2-naphthyl group, particularly preferably, in terms of easiness of production, a substituted or unsubstituted phenyl group.

The substituent that may be included in group R¹ will be described later in detail. The substituent that may be included in group R¹ is preferably at least one selected from a group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of substituents is e.g. 1 or more and 3 or less.

### (R²)

R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below.

R² is preferably a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkylthio group, a substituted or unsubstituted aryloxy group, a substituted or unsubstituted arylthio group, a substituted or unsubstituted aralkyl group, or a group represented by formula (2a) below.

From the viewpoint of enhancing the color fading speed, R² is more preferably a substituted or unsubstituted alkyl group, a substituted or unsubstituted haloalkyl group, a substituted or unsubstituted cycloalkyl group, or a group represented by formula (2a) below.

The halogen atom is preferably a fluorine atom or a chlorine atom.

The alkyl group is preferably an unsubstituted alkyl group having 1 to 20 carbon atoms, more preferably an unsubstituted alkyl group having 1 to 10 carbon atoms. Specific examples of the alkyl group include a methyl group, an ethyl group, a propyl group, a butyl group, a pentyl group, and a hexyl group.

Preferably, the haloalkyl group has 1 to 20 carbon atoms. The number of halogen atoms is preferably 1 to 20, more preferably 2 to 10. The haloalkyl group having 1 to 20 carbon atoms is preferably an alkyl group substituted with a fluorine atom, a chlorine atom, or a bromine atom. Preferably, the haloalkyl group has a perfluoromethyl group on its terminal. Examples of a suitable haloalkyl group include a trifluoromethyl group, a trifluoroethyl group, a trifluoropropyl group, a tetrafluoroethyl group, a chloromethyl group, a 2-chloroethyl group, a bromomethyl group, and the like.

The cycloalkyl group is preferably a cycloalkyl group having 3 to 10 carbon atoms (cycloalkyl group having 3 to 8 carbon atoms constituting a ring). Examples of the cycloalkyl group having 3 to 10 carbon atoms include a cyclopropyl group, a cyclobutyl group, a cyclopentyl group, a cyclohexyl group, and the like. Note that, although the cycloalkyl group may have substituents, the number of carbon atoms (3 to 10 carbon atoms) does not include the number of carbon atoms in the substituents.

The alkoxy group is preferably an alkoxy group having 1 to 6 carbon atoms. Examples of a suitable alkoxy group having 1 to 6 carbon atoms include a methoxy group, an ethoxy group, an n-propoxy group, an isopropoxy group, an n-butoxy group, a sec-butoxy group, a tert-butoxy group, and the like.

Preferably, the aryloxy group has 6 to 12 carbon atoms. Examples of the aryloxy group having 6 to 12 carbon atoms include a phenyloxy group, a naphthyloxy group, and the like.

Preferably, the alkylthio group has 1 to 6 carbon atoms. Examples of the alkylthio group having 1 to 6 carbon atoms include a methylthio group, ethylthio group, an n-propylthio group, an isopropylthio group, an n-butylthio group, a secbutylthio group, a t-butylthio group, and the like.

Preferably, the arylthio group has 6 to 10 carbon atoms. Examples of the arylthio group having 6 to 10 carbon atoms include a phenylthio group, a 1-naphthylthio group, a 2-naphthylthio group, and the like.

Preferably, the aralkyl group has 7 to 11 carbon atoms. Examples of the aralkyl group having 7 to 11 carbon atoms include a benzyl group, a phenylethyl group, a phenylpropyl group, a phenylbutyl group, a naphthylmethyl group, and the like.

The substituent that may be included in group R² will be described later in detail. The substituent that may be included in group R² is preferably at least one selected from a group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, a cycloalkyl group having 4 or more and 8 or less carbon atoms, and a halogen atom. The number of substituents is e.g. 1 or more and 3 or less.

R² is preferably an alkyl group having 1 to 20 carbon atoms, a haloalkyl group having 1 to 20 carbon atoms, an alkoxy group having 1 to 10 carbon atoms, an alkoxyalkyl group having 1 to 10 carbon atoms, a cycloalkyl group having 3 to 10 carbon atoms, or a fluorine atom, more preferably an alkyl group having 1 to 10 carbon atoms, a haloalkyl group having 1 to 10 carbon atoms, or a cycloalkyl group having 5 to 8 carbon atoms. The haloalkyl group having 1 to 10 carbon atoms is preferably a fluoroalkyl group, particularly preferably a fluoroalkyl group having a perfluoromethyl group on its terminal.

### (Group Represented by Formula (2a))

-Q¹-(X¹Q²)a-X²Q³ (2a)

In formula (2a), Q¹ is an alkylene group or a haloalkylene group.

The number of carbon atoms in the alkylene group is preferably 1 or more and 20 or less, more preferably 1 or more and 12 or less, even more preferably 1 or more and 7 or less, and most preferably 2 or more and 6 or less. As the halogen atom in the haloalkylene group, at least one selected from a group consisting of I, Cl, Br, and F may be used. The halogen atom is preferably at least one of Cl and F, more preferably F. In the haloalkylene group, it is preferable that the halogen atom is bonded to the terminal carbon atom, and it is more preferable that the terminal group is a perfluoromethyl group.

Q² is an alkylene group or a haloalkylene group. The preferable aspect of the alkylene group or haloalkylene group is the same as that described for Q¹. The number of carbon atoms in the alkylene group or haloalkylene group of Q² may be the same as or different from that in the alkylene group or haloalkylene group of Q¹.

Q³ is an alkyl group or a haloalkyl group, which may be linear or branched, but is preferably linear.

The carbon number in the alkyl group is preferably 1 or more and 20 or less, more preferably 1 or more and 12 or less, and most preferably 1 or more and 7 or less. As the halogen atom of the haloalkyl group, at least one selected from a group consisting of I, Cl, Br, and F may be used. The halogen atom is preferably at least one of Cl and F, more preferably F.

Q³ is preferably a linear alkyl group.

X¹ and X² are each independently O, S, NR⁷⁰⁰, PR⁷⁰¹, or P(=O). X¹ and X² are preferably O, S, or NR⁷⁰⁰, most preferably O.

R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.

R⁷⁰⁰ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

R⁷⁰¹ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

a is an integer of 0, or 1 or more and 3 or less.

Formula (2a) is preferably an alkylenealkoxy group, an alkylenethioalkyl group, or an alkyleneoxyalkylenealkoxy group.

Specific examples of formula (2a) include -CH₂OCH₃, - CH₂SCH₃, -CH₂CH₂OCH₃, -CH₂CH₂OCH₂CH₃, -CH₂CH₂SCH₃, -CH₂CH₂CH₂OCH₃, - CH₂CH₂CH₂SCH₃, -CH₂CH₂OCH₂CH₂OCH₃, and -CH₂CH₂OCH₂CH₂OCH₂CH₃.

The group represented by formula (2a) is preferably - CH₂OCH₃, -CH₂CH₂OCH₃, -CH₂CH₂OCH₂CH₃, -CH₂CH₂CH₂OCH₃, - CH₂CH₂OCH₂CH₂OCH₃, or -CH₂CH₂OCH₂CH₂OCH₂CH₃.

### (Specific Example of Formula (1a))

Examples of suitable formula (1a) are as follows.

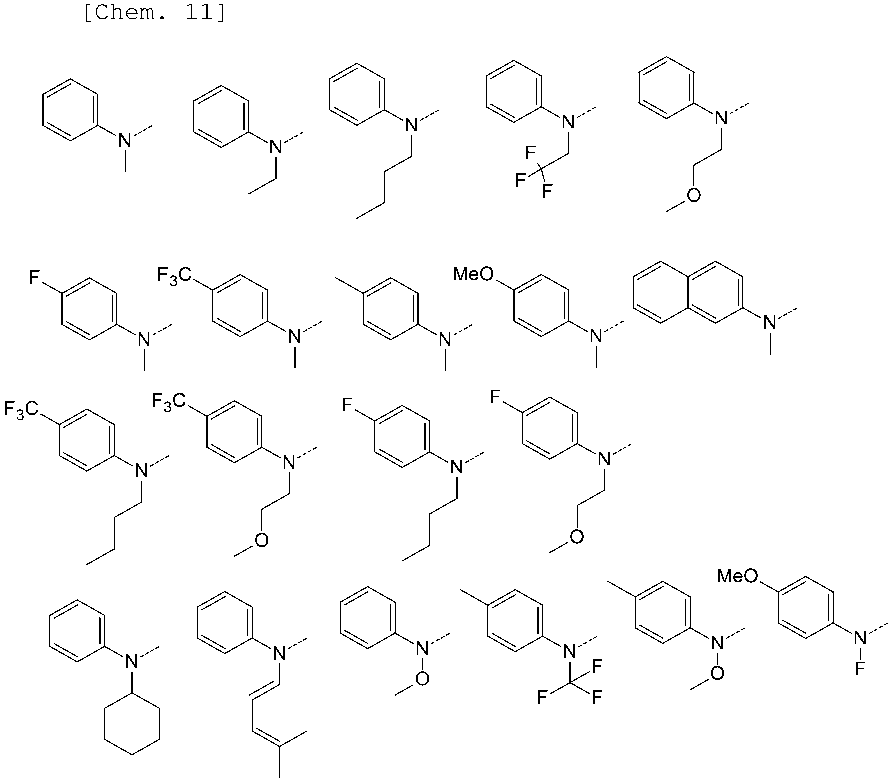

### <R⁴>

In formula (1), R⁴ is a substituted or unsubstituted aryl group or a substituted or unsubstituted heteroaryl group.

The number of carbon atoms in the substituted or unsubstituted aryl group is e.g. 5 or more and 12 or less, and preferably 6 or more and 10 or less. The heteroatom of the substituted or unsubstituted heteroaryl group is at least one selected from a group consisting of oxygen atom, sulfur atom, nitrogen atom, and phosphorus atom. The number of heteroatoms in the substituted or unsubstituted heteroaryl group is e.g. 1 or more and 3 or less, and preferably 1 or 2. The number of carbon atoms in the substituted or unsubstituted heteroaryl group is e.g. 4 or more and 11 or less, and preferably 5 or more and 9 or less.

R⁴ is preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group, more preferably a substituted or unsubstituted phenyl group, a substituted or unsubstituted 1-naphthyl group, or a substituted or unsubstituted 2-naphthyl group, particularly preferably a substituted phenyl group.

### <Skeleton Represented by Formula (2)>

Preferably, the photochromic compound according to the embodiment has a naphthopyran skeleton represented by formula (2) below. In formula (2) below, M, R³, and R⁴ are each the same as those in formula (1).

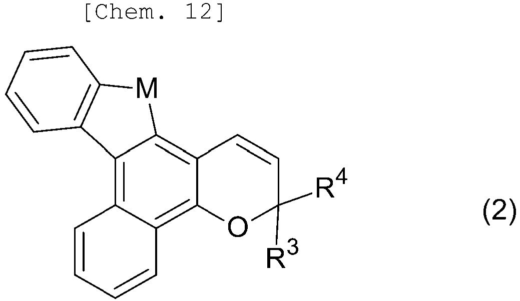

### <Skeleton Represented by Formula (3)>

Preferably, the photochromic compound according to the embodiment has a skeleton represented by formula (3) below. In formula (3) below, M, R³, and R⁴ are each the same as those in formula (1).

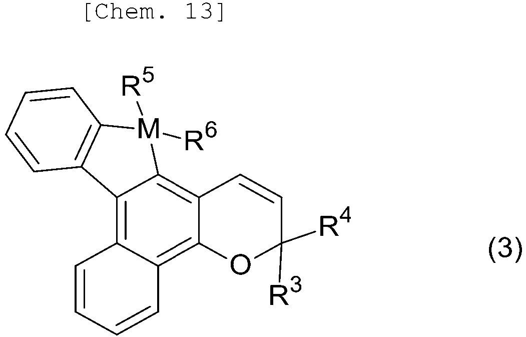

### <R⁵ and R⁶>

R⁵ and R⁶ are each independently a hydrogen atom, a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) below.

The same groups as those listed for R¹ or R² as examples may be used as the substituted or unsubstituted alkyl group, the haloalkyl group, the group represented by formula (2a), the substituted or unsubstituted cycloalkyl group, the substituted or unsubstituted alkoxy group, the halogen atom, the substituted or unsubstituted alkylthio group, the substituted or unsubstituted aryloxy group, substituted or unsubstituted arylthio group, the substituted or unsubstituted aralkyl group, the substituted or unsubstituted aryl group, and the substituted or unsubstituted heteroaryl group.

The amino group may be a primary amino group (-NH₂), or a secondary or tertiary amino group with substituents that have been substituted for one or two hydrogen atoms. The amino group may be the group represented by formula (1a) above. Examples of the substituents in the substituted amino group include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like. Examples of a suitable amino group include an amino group, a methylamino group, a dimethylamino group, an ethylamino group, a diethylamino group, a methylphenylamino group, a diphenylamino group, and the like.

Preferably, the heterocyclic group is constituted by 3 to 10 atoms. The heteroatom of the heterocyclic group is preferably at least one selected from a group consisting of oxygen atom, nitrogen atom, sulfur atom, and phosphorus atom. The number of heteroatoms is e.g. 1 or more and 5 or less, and preferably 1 or 2. Specific examples of the heterocyclic group include an aliphatic heterocyclic group such as a morpholino group, a piperidino group, a pyrrolidinyl group, a piperazino group, and an N-methylpiperazino group, and an aromatic heterocyclic group such as an indolinyl group. The heterocyclic group may also be a 2,6-dimethylmorpholino group, a 2,6-dimethylpiperidino group, and a 2,2,6,6-tetramethylpiperidino group.

Preferably, the alkylcarbonyl group has 2 to 7 carbon atoms. Examples of the alkylcarbonyl group having 2 to 7 carbon atoms include an acetyl group and an ethylcarbonyl group.

Preferably, the alkoxycarbonyl group has 2 to 7 carbon atoms. Examples of the alkoxycarbonyl group having 2 to 7 carbon atoms include a methoxycarbonyl group and an ethoxycarbonyl group.

Preferably, the aralkoxy group has 7 to 11 carbon atoms. Examples of the aralkoxy group having 7 to 11 carbon atoms include a benzyloxy group, a naphthylmethoxy group, and the like.

Preferably, the alkoxyalkylthio group has 2 to 9 carbon atoms. Examples of the alkoxyalkylthio group having 2 to 9 carbon atoms include a methoxymethylthio group, a methoxyethylthio group, a methoxy-n-propylthio group, a methoxy-n-butylthio group, an ethoxyethylthio group, an n-propoxypropylthio group, and the like.

Preferably, the haloalkylthio group has 1 to 6 carbon atoms. Examples of the haloalkylthio group having 1 to 6 carbon atoms include a trifluoromethylthio group, a tetrafluoroethylthio group, a chloromethylthio group, a 2-chloroethylthio group, a bromomethylthio group, and the like.

Preferably, the cycloalkylthio group has 3 to 8 carbon atoms. Examples of the cycloalkylthio group having 3 to 8 carbon atoms include a cyclopropylthio group, a cyclobutylthio group, a cyclopentylthio group, a cyclohexylthio group, and the like. Note that, although the cycloalkylthio group may have substituents, the number of carbon atoms (3 to 8 carbon atoms) does not include the number of carbon atoms in the substituents.

The silyl group may have substituents. Examples of the substituents of the substituted silyl group include, but are not particularly limited to, an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like.

The oxysilyl group may have substituents. Examples of the substituents of the substituted oxysilyl group include, but are not particularly limited to, an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like.

The cycloalkyl group, the arylthio group, the aralkyl group, the aralkoxy group, the aryloxy group, the aryl group, the heteroaryl group, and the cycloalkylthio group may be unsubstituted. However, when these groups have substituents, it is preferable that 1 to 8 hydrogen atoms, particularly preferably 1 to 4 hydrogen atoms in groups constituting the ring are substituted with substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom.

R⁵ and R⁶ may be a hydrogen atom, a substituted or unsubstituted alkyl group, a haloalkyl group, a group represented by formula (2a), a substituted or unsubstituted cycloalkyl group, a substituted or unsubstituted alkoxy group, a hydroxycarbonyl group, a substituted or unsubstituted alkylcarbonyl group, a substituted or unsubstituted alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, or formula (X3) below.

R⁵ and R⁶, together with M, may collectively constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with an aliphatic ring, a substituted or unsubstituted heterocyclic ring having 3 to 20 ring-membered atoms, or a substituted or unsubstituted fused polycylic ringin in which an aromatic ring or an aromatic heterocyclic ring is fused with a heterocyclic ring.

R⁵ and R⁶ preferably constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with an aliphatic ring, a substituted or unsubstituted heterocyclic ring having 3 to 20 ring-membered atoms, more preferably constitute, together with M, a ring selected from a cyclopentane ring, a cyclohexane ring, a cycloheptane ring, a cyclooctane ring, a cyclononane ring, a cyclodecane ring, a cycloundecane ring, a cyclododecane ring, and a spirodicyclohexane ring. The ring may have 1 to 10 alkyl groups having 1 to 5 carbon atoms or cycloalkyl groups having 5 to 7 carbon atoms as substituents, and a cycloalkyl group having 5 to 7 carbon atoms may be fused. Specifically, it is preferable that rings represented by formulas below are formed.

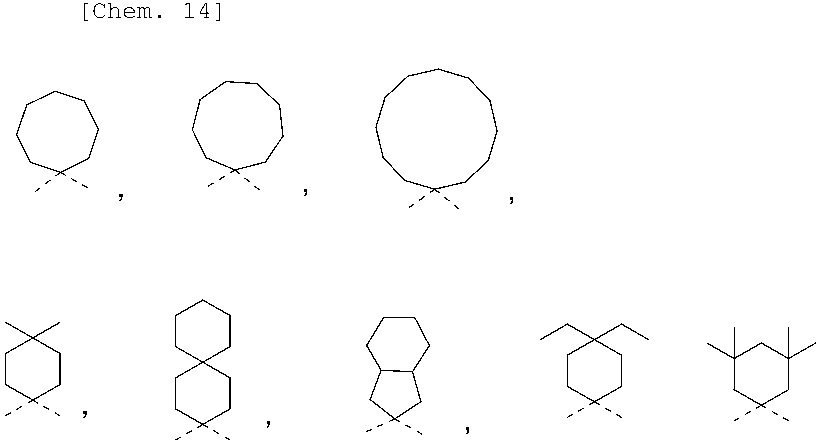

The substituents that may be included in groups R⁵ and R⁶ will be described later in detail. A substituent that may be included in this group is preferably a linear or branched alkyl group having 1 or more and 6 or less carbon atoms.

### <Group Represented by Formula (X)>

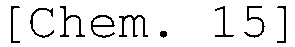

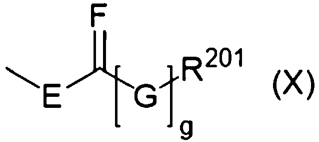

In formula (X), E is an oxygen atom or NR¹⁰¹. R¹⁰¹ is a hydrogen atom or an alkyl group. E is NR¹⁰¹, and R¹⁰¹ is preferably a hydrogen atom or an alkyl group having 1 to 6 carbon atoms.

F is an oxygen atom or a sulfur atom. F is preferably an oxygen atom.

G is an oxygen atom, a sulfur atom, or NR²⁰². R²⁰² is a hydrogen atom, an alkyl group, a cycloalkyl group, an aryl group, or a heteroaryl group. G is preferably NH.

g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than a hydrogen atom. R²⁰¹ is preferably an alkyl group having 1 to 6 carbon atoms, an alkoxy group having 1 to 6 carbon atoms, or an aryl group having 6 to 12 carbon atoms.

Suitable groups represented by formula (X) are as follows.

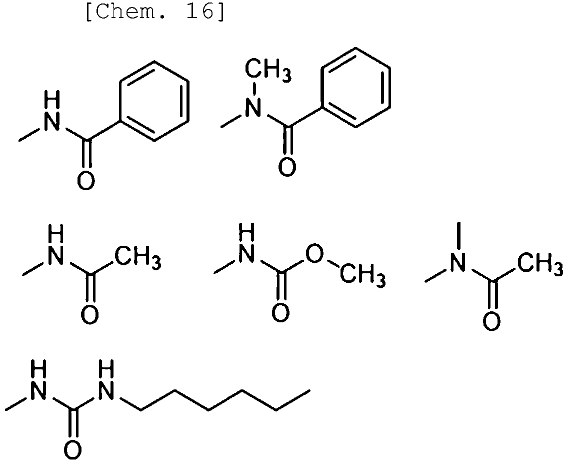

### <Group Represented by Formula (X3)>

L¹-R⁴⁰⁰ (X3)

In formula (X3),
R⁴⁰⁰ is a hydrogen atom, an alkyl group, an aryl group, a polymerizable group, a photochromic group, or a silyl group having, as a substituent, an alkyl group, an alkoxy group, or an aryl group.

L¹ is a group represented by formula (X2) below.

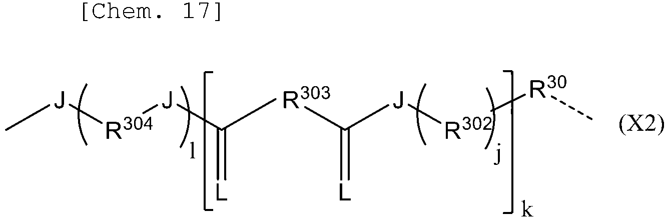

In formula (X2), R³⁰ is a group represented by formula (X2a) below.

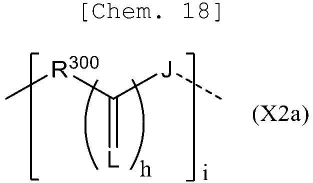

In formula (X2) and formula (X2a), J represents a divalent group. A plurality of J groups is each independently directly bonded, a substituted or unsubstituted methylene group, an oxygen atom, a sulfur atom, or NR³⁰¹. R³⁰¹ is a hydrogen atom or an alkyl group. R³⁰¹ is preferably an alkyl group having 1 to 20 carbon atoms. Preferably, this alkyl group has, as a substituent, a silyl group having an alkyl group with 1 to 10 carbon atoms, a polymerizable group, or a photochromic group.

Examples of the polymerizable group include radical polymerizable groups such as a vinyl group, a 1-chlorovinyl group, an allyl group, a styryl group, a (meth)acrylic group, a 2-(methacryloxy)ethylcarbamyl group, a 2-(methacryloxy)ethoxycarbonyl group, and a crotyl group. Other examples thereof include an epoxy group, an episulfide group, a thietanyl group, OH group, SH group, NH₂ group, COOH group, NCO group, NCS group, or the like. The polymerizable group is preferably at least one selected from a group consisting of a (meth)acrylic group, a 2-(methacryloxy)ethylcarbamyl group, a 2-(methacryloxy)ethoxycarbonyl group, an epoxy group, OH group, SH group, NH₂ group, and COOH group.

The photochromic group includes a photochromic site. Representative examples of the photochromic group include naphthopyran, spirooxazine, spiropyran, fulgide, fulgimide, and diarylethene. As the photochromic group, indenonaphthopyran is preferable because it can express excellent photochromic properties, and above all, indeno[2,1-f]naphtho[1,2-b]pyran is particularly preferable.

Indeno[2,1-f]naphtho[1,2-b]pyran is preferably a group represented by formula (X₄) below.

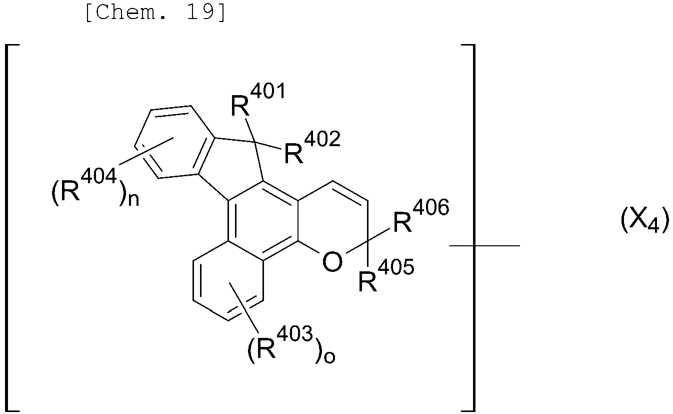

In formula (X₄), R⁴⁰¹ and R⁴⁰² may be the same groups as those used for R⁵ and R⁶ described above.

R⁴⁰³ and R⁴⁰⁴ may be each independently the same groups as those used for R⁵ and R⁶ described above.

R⁴⁰⁵ and R⁴⁰⁶ are each independently a substituted or unsubstituted aryl group, or a substituted or unsubstituted heteroaryl group. R⁴⁰⁵ and R⁴⁰⁶ may be the same as the groups used for R⁴ described above.

In formula (X₄), o is an integer of 0 to 4.

n is an integer of 0 to 4. When o is 2 to 4, a plurality of R⁴⁰³ groups may be the same as or different from each other. When n is 2 to 4, a plurality of R⁴⁰⁴ groups may be the same as or different from each other.

At least one of the substituents on the aryl or heteroaryl group of R⁴⁰¹, R⁴⁰², R⁴⁰³, R⁴⁰⁴, and R⁴⁰⁵ binds to L¹.

A particularly suitable group represented by formula (X2) is represented by formulas below.

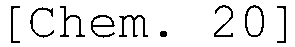

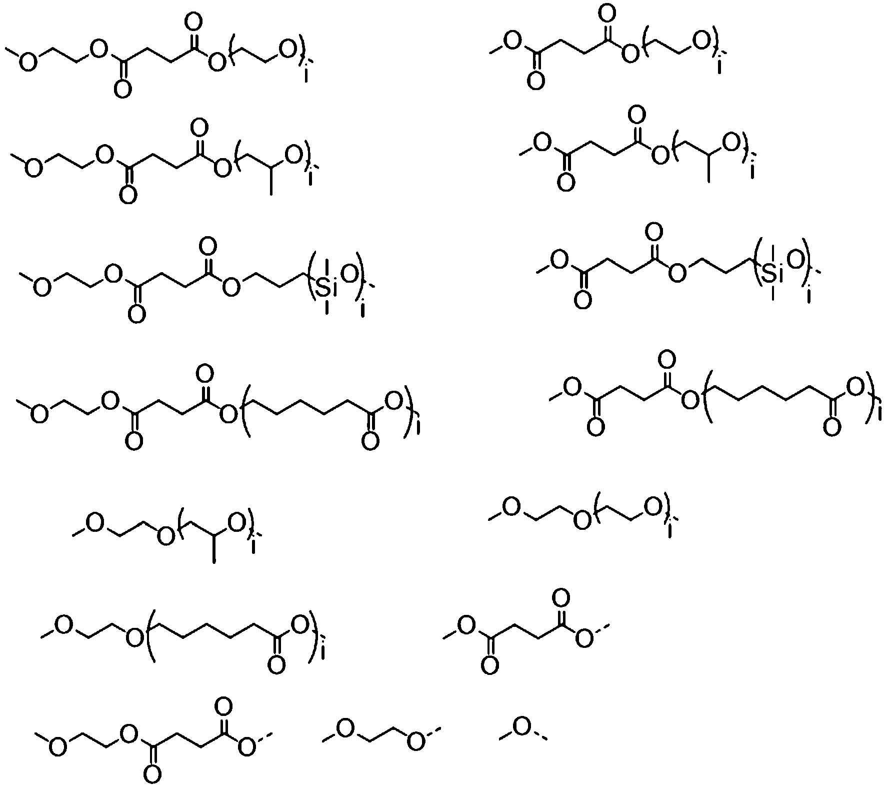

In formula (X2) and formula (X2a), L is an oxygen atom or a sulfur atom.

R³⁰⁰ is an alkylene group or a silylene group having an alkyl group or an aryl group as a substituent. R³⁰⁰ is preferably a silylene group having an alkylene group having 1 to 6 carbon atoms, or a silylene group with an alkyl group having 1 to 6 carbon atoms as a substituent.

R³⁰², R³⁰³, and R³⁰⁴ are each independently an alkylene group. R³⁰² is preferably an alkylene group having 1 to 6 carbon atoms. R³⁰³ is preferably an alkylene group having 1 to 6 carbon atoms. R³⁰⁴ is preferably an alkylene group having 1 to 6 carbon atoms.

h, j, k, and 1 are each independently an integer of 0 or 1.

In formula (X2a), i is an integer of 1 to 200. When i is 2 or more, the structures of a plurality of R³⁰ may be the same as or different from each other.
i is preferably a numerical number within a range of 5 to 100, more preferably 8 to 75, most preferably 10 to 70.

The dashed lines indicate bonds with R⁴⁰⁰,

### <Photochromic Compound Represented by Formula (4)>

The photochromic compound according to the embodiment is preferably a compound represented by formula (4) below.

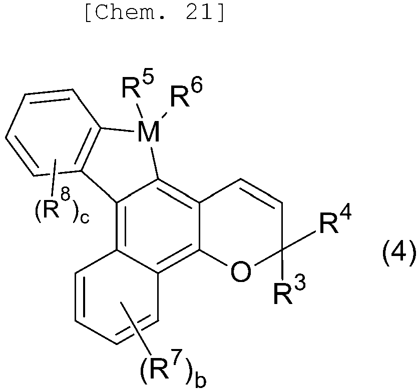

In formula (4), R³, R⁴, R⁵, R⁶, and M are each the same as those in formula (3).

### <R⁷ and R⁸>

R⁷ and R⁸ are each independently a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted haloalkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) above, or a group represented by formula (X3) above. As these groups, the same groups as those listed for R¹ to R⁶ as examples may be used.

b is an integer of 0 to 4, and c is an integer of 0 to 4, when b is 2 to 4, a plurality of R⁷ groups may be the same as or different from each other,
when c is 2 to 4, a plurality of R⁸ groups may be the same as or different from each other,
when b is 2 to 4 and there are R⁷ groups adjacent to each other, the two adjacent R⁷ groups, together with carbon atoms to which the R⁷ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ringin in which an aromatic ring or an aromatic heterocyclic ring is fused with these rings. The combined adjacent R⁷ groups are located at the position 5 and the position 6, the position 6 and the position 7, or the position 7 and the position 8 of the chromene compound.

When c is 2 to 4, a plurality of R⁸ groups may be the same as or different from each other. When c is 2 to 4 and there are R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ringin in which an aromatic ring or an aromatic heterocyclic ring is fused with these rings. The combined adjacent R⁸ groups are located at the position 9 and the position 10, the position 10 and the position 11, or the position 11 and the position 12 of the chromene compound.

R⁷ or R⁸ may constitute a ring constituted by 5 to 8 atoms including carbon atoms to which R⁷ or R⁸ bind. The ring may also have a substituent. The substituent includes a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, and a halogen atom. Specific examples of these substituents will be described later.

A suitable ring is exemplified by a ring represented by formula (X5) below.

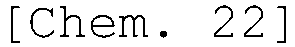

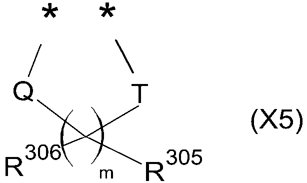

In formula (X5), Q and T are each independently a sulfur atom, a substituted or unsubstituted methylene group, an oxygen atom, or a group represented by NR³⁰⁷. R³⁰⁷ is a hydrogen atom, a hydroxy group, an alkyl group, a haloalkyl group, a cycloalkyl group, an alkoxy group, a substituted or unsubstituted aryl group, a substituted or unsubstituted heteroaryl group, or a group represented by formula (2a).

Preferably, R³⁰⁵ and R³⁰⁶ are each independently a hydroxy group, an alkyl group, a haloalkyl group, a cycloalkyl group, an alkoxy group, an amino group, a substituted amino group, a substituted or unsubstituted heterocyclic group, a cyano group, a nitro group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryl group, a thiol group, an alkylthio group, an alkoxyalkylthio group, a haloalkylthio group, a cycloalkylthio group, or a substituted or unsubstituted arylthio group.

R³⁰⁵ and R³⁰⁶ may constitute a substituted or unsubstituted aliphatic ring together with carbon atoms to which R³⁰⁵ and R³⁰⁶ bind. Specific examples of the aliphatic ring include a cyclopentane ring, a cyclohexane ring, and the like. In the aliphatic ring, 1 to 8 hydrogen atoms, particularly preferably 1 to 4 hydrogen atoms may be substituted with at least one group selected from a group consisting of a hydroxy group, an alkyl group, a haloalkyl group, a cycloalkyl group, an alkoxy group, an amino group, a substituted amino group, a heterocyclic group, a cyano group, a nitro group, and a halogen atom. Specific examples of these substituents will be described later.

In formula (X5), m is an integer of 1 to 4.

### <Photochromic Compound Represented by Formula (5)>

A suitable chromene compound is exemplified by a compound represented by formula (5) below.

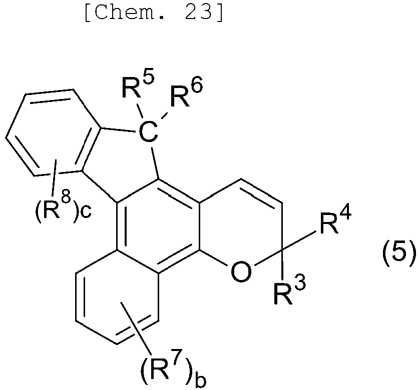

In formula (5),
R³, R⁴, R⁵, R⁶, R⁷, R⁸, b, and c are each independently the same as those in formula (4).

### <Photochromic Compound Represented by Formula (6)>

A more preferable photochromic compound is exemplified by a compound represented by formula (6) below.

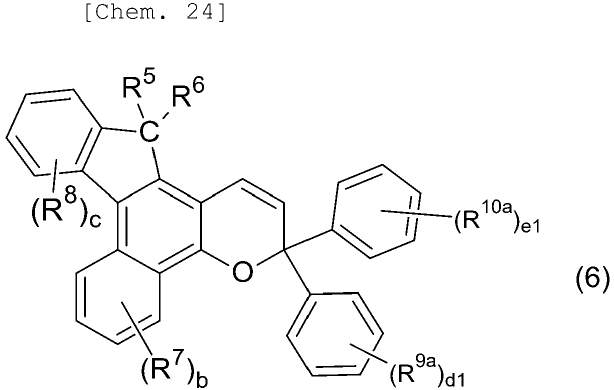

In formula (6), R⁵, R⁶, R⁷, R⁸, b, and c are each independently the same as those in formula (5).

### <R^{9a}>

R^{9a} is a group represented by formula (1a) above, a hydrogen atom, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above. As these groups, the same groups as those listed for R¹ to R⁸ as examples may be used.

d1 represents the number of R^{9a} and is an integer of 1 to 5.

When d1 is 1, R^{9a} is a group represented by formula (1a) above.

When d1 is 2 to 5, at least one of a plurality of R^{9a} groups is a group represented by formula (1a) above, and a plurality of R^{9a} groups may be the same as or different from each other.

When there are R^{9a} groups adjacent to each other, except for the group represented by formula (1a) above, the two adjacent R^{9a} groups, together with carbon atoms to which the R^{9a} groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ringin in which an aromatic ring or an aromatic heterocyclic ring is fused with these rings. Two R^{9a} groups adjacent to each other can collectively constitute a ring group that may include at least one selected from a group consisting of an oxygen atom, a sulfur atom, a carbon atom, and a nitrogen atom. This ring group is preferably, but not particularly limited to, a ring constituted by 5 to 8 atoms including carbon atoms to which R^{9a} groups bind. The ring may also have a substituent. Examples of the substituent include substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents are the same as those described above. As for the ring, it is preferable that R^{9a} groups collectively constitute a ring represented by formula (X5). However, as described above, the group represented by formula (1a) above does not constitute the above-described ring even if there are other adjacent R^{9a} groups.

### <R^{10a}>

R^{10a} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above. As these groups, the same groups as those listed for R¹ to R⁸ as examples may be used.

e1 represents the number of R^{10a} and is an integer of 0 to 5.

When e1 is 2 or larger, a plurality of R^{10a} groups may be the same as or different from each other.

When e1 is 2 to 5 and there are R^{10a} groups adjacent to each other, the two adjacent R^{10a} groups, together with carbon atoms to which the R^{10a} groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ringin in which an aromatic ring or an aromatic heterocyclic ring is fused with these rings.

The two adjacent R^{10a} groups can collectively constitute a ring group that may include at least one selected from a group consisting of an oxygen atom, a sulfur atom, a carbon atom, and a nitrogen atom. This ring group is preferably, but not particularly limited to, a ring constituted by 5 to 8 atoms including carbon atoms to which R^{10a} groups bind. The ring may also have a substituent. Examples of the substituent include substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents are the same as those described above. As for the ring, it is preferable that R^{10a} groups collectively constitute a ring represented by formula (X5).

### <Photochromic Compound Represented by Formula (7)>

A photochromic compound having superior color fading speed and repetitive durability is exemplified by a compound represented by formula (7) below.

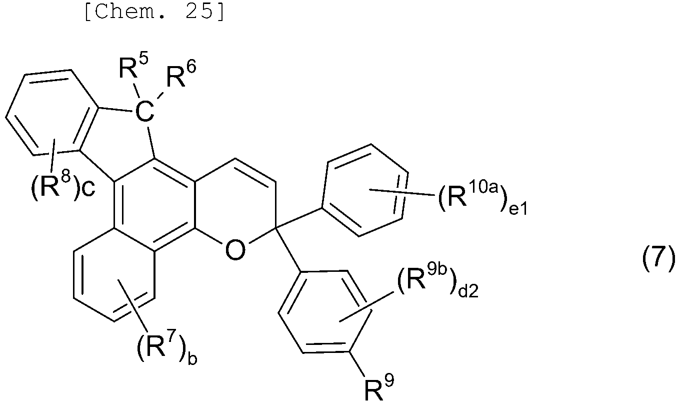

In formula (7), R⁵, R⁶, R⁷, R⁸, R^{10a}, b, c, and e1 are each the same as those in formula (6), and R⁹ is a group represented by formula (1a) above.

### <R^{9b}>

R^{9b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above. As these groups, the same groups as those listed for R¹ to R⁸ as examples may be used.

d2 represents the number of R^{9b} and is an integer of 0 to 4.

When d2 is 2 or larger, a plurality of R^{9b} groups may be the same as or different from each other.
when d2 is 2 to 4 and there are R^{9b} groups adjacent to each other, the two adjacent R^{9b} groups, together with carbon atoms to which the R^{9b} groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ringin in which an aromatic ring or an aromatic heterocyclic ring is fused with these rings.

Two R^{9b} groups adjacent to each other can collectively constitute a ring group that may include at least one selected from a group consisting of oxygen atom, sulfur atom, carbon atom, and nitrogen atom. This ring group is preferably, but not particularly limited to, a ring constituted by 5 to 8 atoms including carbon atoms to which R^{9b} groups bind. The ring may also have a substituent. Examples of the substituent include substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents are the same as those described above. As for the ring, it is preferable that R^{9b} groups collectively constitute a ring represented by formula (X5).

### <Photochromic Compound Represented by Formula (8)>

A photochromic compound having particularly excellent color fading speed and repetitive durability is exemplified by a compound represented by formula (8) below.

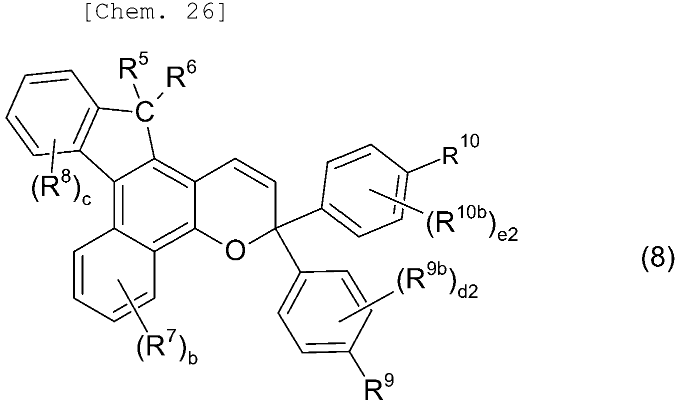

In formula (8), R⁵, R⁶, R⁷, R⁸, R⁹, R^{9b}, b, c, and d2 are each the same as those in formula (7).

### <R¹⁰>

R¹⁰ is an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a substituted or unsubstituted silyl group, a substituted or unsubstituted oxysilyl group, a group represented by formula (2a) above, or a group represented by formula (X3) above. As these groups, the same groups as those listed for R¹ to R⁸ as examples may be used.

### <R^{10b}>

R^{10b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above. As these groups, the same groups as those listed for R¹ to R⁸ as examples may be used.

e2 represents the number of R^{10b} and is an integer of 0 to 4.

When e2 is 2 or larger, a plurality of R^{10b} groups may be the same as or different from each other.

When e2 is 2 to 4 and there are R^{10b} groups adjacent to each other, the two adjacent R^{10b} groups, together with carbon atoms to which the R^{10b} groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Two R^{10b} groups adjacent to each other can collectively constitute a ring group that may include at least one selected from a group consisting of oxygen atom, sulfur atom, carbon atom, and nitrogen atom. This ring group is preferably, but not particularly limited to, a ring constituted by 5 to 8 atoms including carbon atoms to which R^{10b} groups bind. The ring may also have a substituent. Examples of the substituent include substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents are the same as those described above. As for the ring, it is preferable that R^{10b} groups collectively constitute a ring represented by formula (X5).

### <Detailed Description of Substituents etc.>

Examples of the substituents that may be included in the groups R¹ to R^{10b} described above include a hydroxy group, a cyano group, a halogen atom, a nitro group, a formyl group, a hydroxycarbonyl group, a thiol group, a group represented by formula (2a), a group represented by formula (X), a group represented by formula (X3), an alkyl group, a haloalkyl group, a cycloalkyl group, an alkoxy group, an amino group, a substituted amino group, a heterocyclic group, a halogen atom, an alkylthio group, an arylthio group, an alkylcarbonyl group, an alkoxycarbonyl group, an aralkyl group, an aralkoxy group, an aryloxy group, an aryl group, a heteroaryl group, an alkoxyalkylthio group, a haloalkylthio group, a cycloalkylthio group, a silyl group, and an oxysilyl group. As these groups, the same groups as those described in detail for R¹ to R⁸ may be used.

### <Specific Examples of Suitable Photochromic Compound>

A particularly suitable photochromic compound is specifically exemplified by photochromic compounds represented by the following formulas.

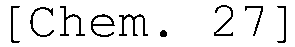

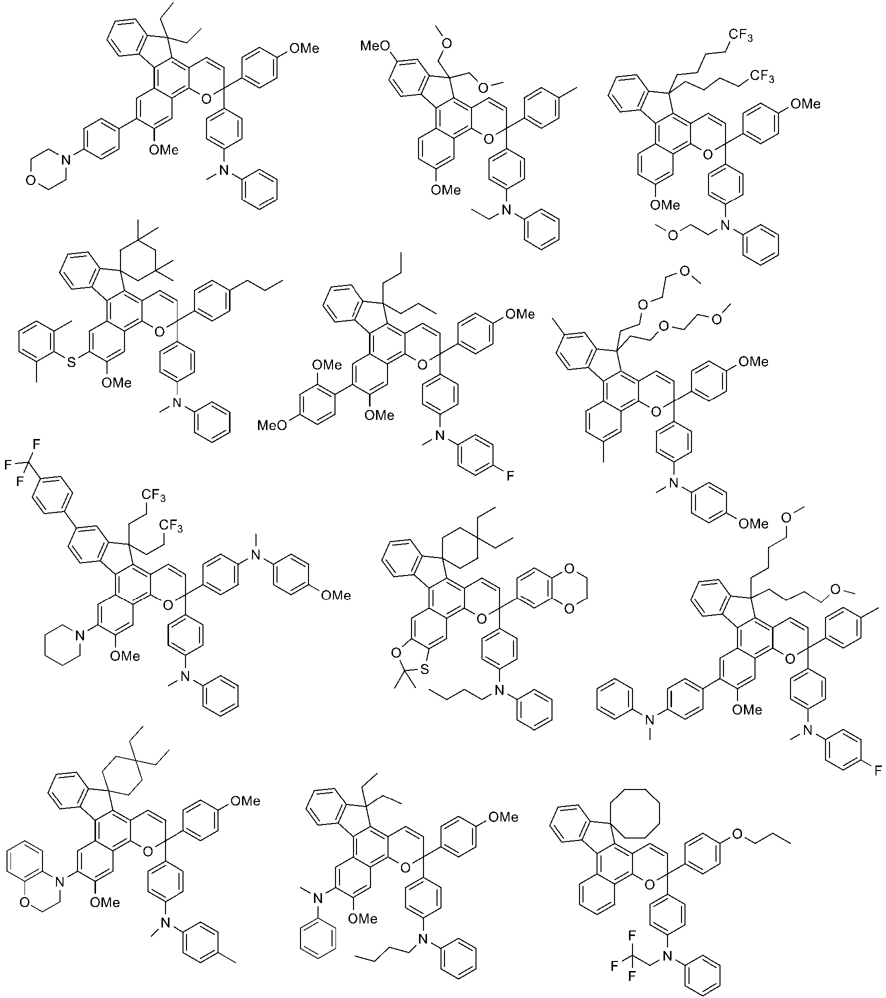

### [Propargyl Alcohol Compound]

The propargyl alcohol compound according to the embodiment has a skeleton represented by formula (9) below. This propargyl alcohol compound may be used as an intermediate for synthesizing the photochromic compounds having the skeleton represented by formulas (1), (2), (3), (4), and (5) above.

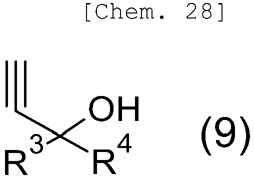

In formula (9), R³ and R⁴ are each independently the same as those in formula (1) above.

### <Propargyl Alcohol Compound Represented by Formula (10)>

The propargyl alcohol compound represented by formula (10) may be used as an intermediate for synthesizing the photochromic compound represented by formula (6) above.

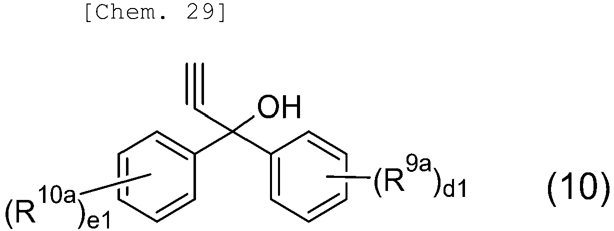

In formula (10), R^{9a}, R^{10a}, d1, and e1 are each independently the same as those in formula (6) above.

### <Propargyl Alcohol Compound Represented by Formula (11)>

The propargyl alcohol compound represented by formula (11) may be used as an intermediate for synthesizing the photochromic compound represented by formula (7) above.

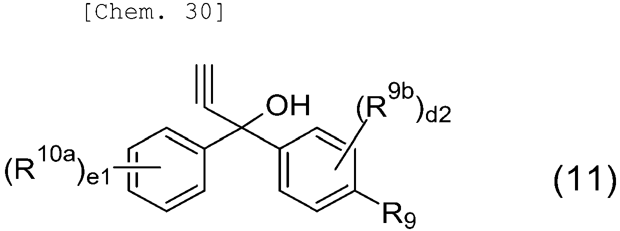

In formula (10), R^{10a}, R⁹, R^{9b}, e1, and d2 are each independently the same as those in formula (7) above.

### <Propargyl Alcohol Compound Represented by Formula (12)>

The propargyl alcohol compound represented by formula (12) may be used as an intermediate for synthesizing the photochromic compound represented by formula (8) above.

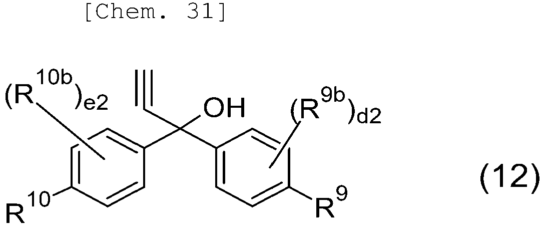

In formula (10), R⁹, R^{9b}, R¹⁰, R^{10b}, d2, and e2 are each independently the same as those in formula (8) above.

### <Specific Examples of Naphthol Derivative>

Specific examples of the naphthol derivative according to the embodiment include compounds represented by the following formulas.

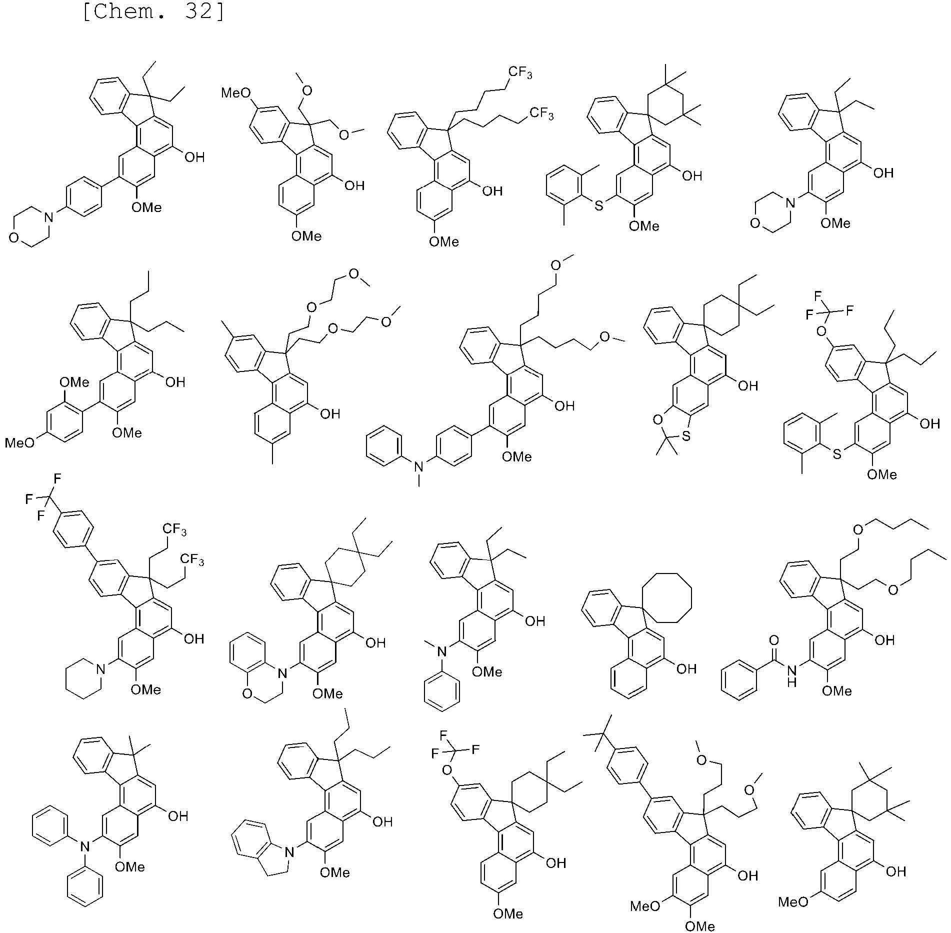

### [Method for Producing Photochromic Compound]

The photochromic compound according to the embodiment may be produced by any synthetic method. A representative example of the method for producing the photochromic compound will be explained, but the present invention is not limited to this method. Note that, in the following description, unless otherwise specified, the symbols in each formula have the same meanings as explained for the formulas described above.

The photochromic compound can be produced by a method of reacting a naphthol derivative represented by formula (13) below with a propargyl alcohol compound represented by formula (9) below in the presence of an acid catalyst.

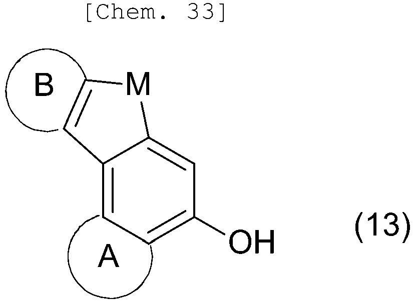

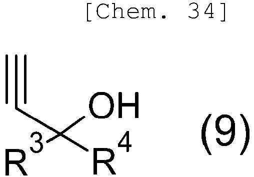

A reaction ratio of the naphthol compound and the propargyl alcohol compound is preferably selected from a range of 1:10 to 10:1 (molar ratio). As an acid catalyst, for example, sulfuric acid, benzenesulfonic acid, p-toluenesulfonic acid, acidic alumina and the like are used. The acid catalyst is used preferably in a range of 0.1 to 10 parts by weight per 100 parts by weight of the sum of the naphthol compound and the propargyl alcohol compound. The reaction temperature is preferably 0 to 200°C. As the solvent, it is preferable to use an aprotic organic solvent such as N-methylpyrrolidone, dimethylformamide, tetrahydrofuran, benzene, and toluene. A method for purifying a product obtained by this reaction is not particularly limited. For example, the product can be purified by a silica gel column purification followed by recrystallization.

### <Method for Synthesizing Naphthol Derivative>

The naphthol derivative can be synthesized e.g. based on reaction methods described in Non-Patent Document 1, Non-Patent Document 2, Patent Document 3, and other articles. As an example, a method for synthesizing a naphthol derivative represented by formula (14) below is not particularly limited, but, for example, when M is a carbon atom, the naphthol derivative can be synthesized as follows. Note that, in formula (14), R⁵, R⁶, R⁷, R⁸, b, and c are the same as those in formula (4) above.

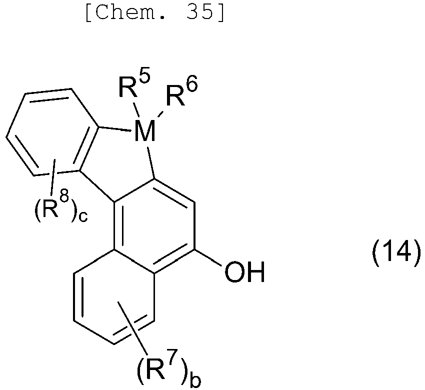

First, a benzophenone compound represented by formula (15) below is synthesized by reacting a benzene compound with an acid chloride using Friedel-Crafts reaction.

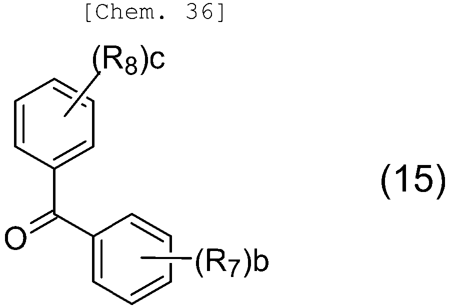

Note that, in formula (15), R⁷, R⁸, b, and c are the same as those in formula (4). Furthermore, the benzophenone compound represented by formula (15) is subjected to a Stobbe reaction, a cyclization reaction, a hydrolysis reaction using an alkali or acid, benzyl protection, or a debenzylization by a hydrolysis reaction using an alkali or acid to obtain a carboxylic acid with a hydroxy group protected by benzyl (Bn), represented by formula (16) below.

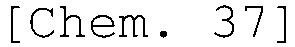

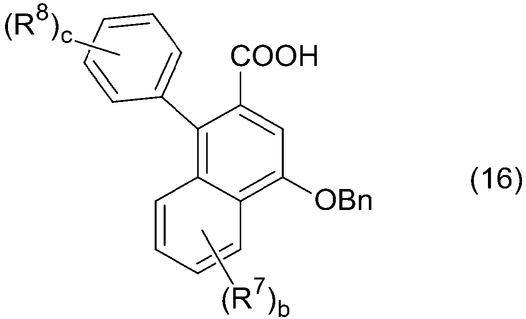

Subsequently, the benzyl-protected carboxylic acid represented by formula (16) is converted into an amine by Curtius rearrangement, Hofmann rearrangement, Lossen rearrangement, or the like to prepare a diazonium salt from this amine. This diazonium salt is converted into a halide such as a bromide or an iodide by a Sandmeyer reaction or the like to obtain a halide represented by formula (17) below (in the formula, Hal represents a halogen).

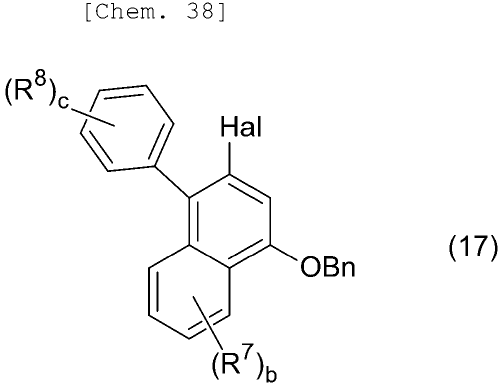

The resulting halide is reacted with magnesium, lithium, or the like to prepare an organometallic reagent. This organometallic reagent is reacted with a ketone represented by formula (18) below (in the formula, R⁵ and R⁶ are the same as those in formula (4)) in an organic solvent at -100 to 70°C to obtain a compound represented by formula (19) below.

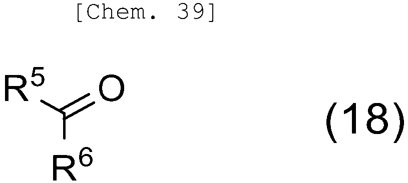

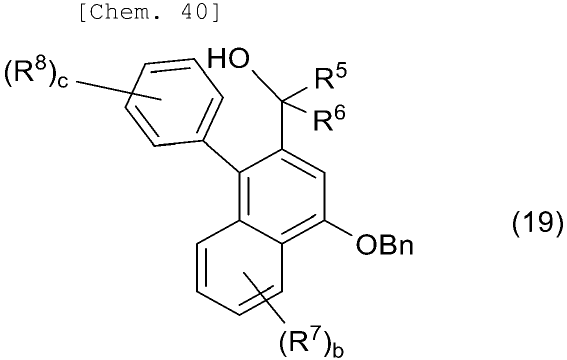

The resulting compound (19) is debenzylized and then reacted under a neutral to acidic conditions at 10 to 120°C for 10 minutes to 2 hours to spiroannulate the alcohol, so that a desired naphthol derivative represented by formula (14) can be synthesized. In this reaction, a reaction ratio between the organometallic reagent and the ketone represented by a formula (19) can be adopted from a wide range, but is preferably selected from a range of 1:10 to 10:1 (molar ratio). The reaction temperature is preferably -100 to 70°C. As the solvent, an aprotic organic solvent such as diethyl ether, tetrahydrofuran, benzene, and toluene is preferably used. The spiroannulation of the alcohol under a neutral to acidic condition is preferably performed in the presence of an acid catalyst. As the acid catalyst, for example, acetic acid, hydrochloric acid, sulfuric acid, benzenesulfonic acid, p-toluenesulfonic acid, and acidic alumina are used. Suitably, such an acid catalyst is used in a range of 0.1 to 10 parts by weight per 100 parts by weight of the alcohol. Preferably, the spiroannulation is performed in the presence of a solvent such as tetrahydrofuran, benzene, and toluene.

### <Method for Synthesizing Naphthol Derivative Including Si and Ge>

An example of a method for producing the naphthol derivative represented by formula (14) with M being Si or Ge will be explained below.

First, the halide represented by formula (17) is reacted with magnesium, lithium, or the like to prepare an organometallic reagent. This organometallic reagent is reacted with a monohalide represented by formula (20) below (in the formula, R⁵ and R⁶ are the same as those in formula (4)) at - 100 to 70°C in an organic solvent to obtain a compound represented by formula (21) below.

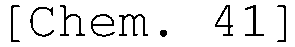

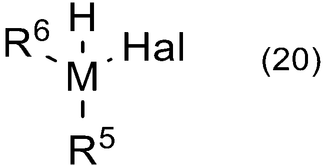

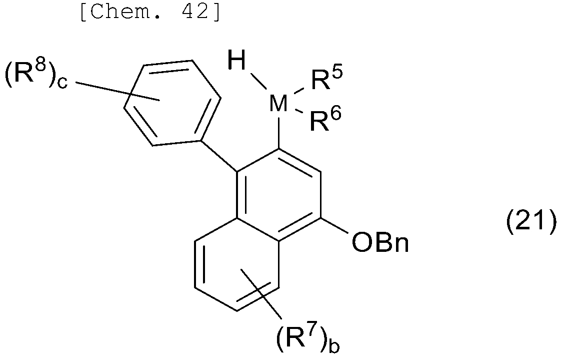

The resulting compound (21) is subjected to a reaction by referring to reaction methods described in Non-Patent Document 3, Non-Patent Document 4, Patent Document 4, and the like, to obtain a cyclized group represented by formula (22) below.

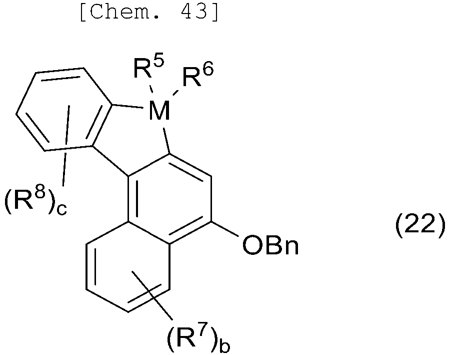

The resulting cyclized group can be debenzylized to obtain the naphthol derivative represented by formula (14) above.

### <Method for Synthesizing Propargyl Alcohol Compound>

The propargyl alcohol compound can be synthesized e.g. based on reaction methods described in Patent Document 5 and Patent Document 6. As an example, the propargyl alcohol compound represented by formula (11) above can be synthesized as follows. First, a benzene compound represented by formula (23) below is reacted with an acid chloride compound represented by formula (24) below to obtain a benzophenone compound represented by formula (25) below (in the formula, R⁹ is the same as that in formula (1a) above).

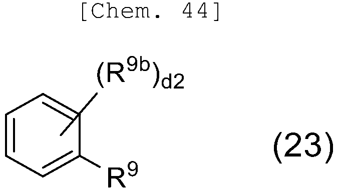

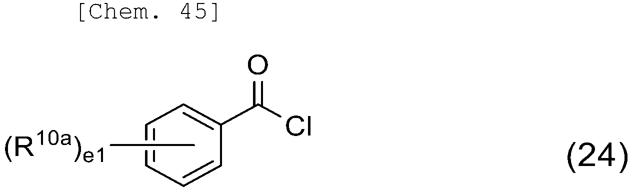

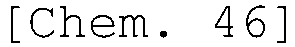

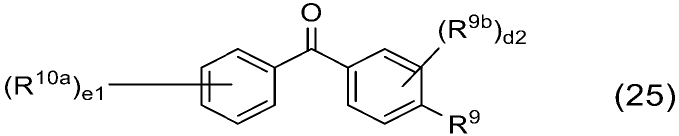

The benzophenone compound represented by formula (25) above can be synthesized by another method in which a benzophenone substituted with a halogen atom as presented in formula (26) below (in the formula, Hal represents a halogen atom) is synthesized, and then the halogen atom is substituted with an amino compound represented by formula (27) below.

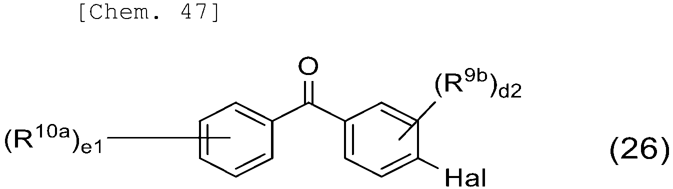

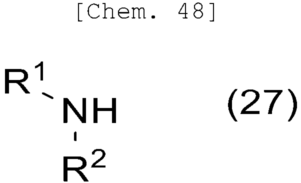

The obtained benzophenone compound can be reacted with a metal acetylene such as sodium acetylide and lithium acetylide, or an acetylene derivative such as acetylene Grignard, to synthesize the propargyl alcohol compound represented by formula (11) above.

Also, the photochromic compound according to the present invention can be obtained by another method in which a propargyl alcohol represented by formula (28) below synthesized from the benzophenone compound represented by formula (26) above is reacted with the naphthol derivative represented by formula (14) above to synthesize a photochromic compound having a halogen atom represented by formula (29) below, and then the halogen atom is substituted with the amino compound represented by formula (27) above.

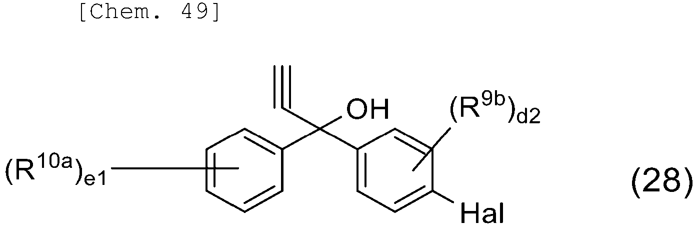

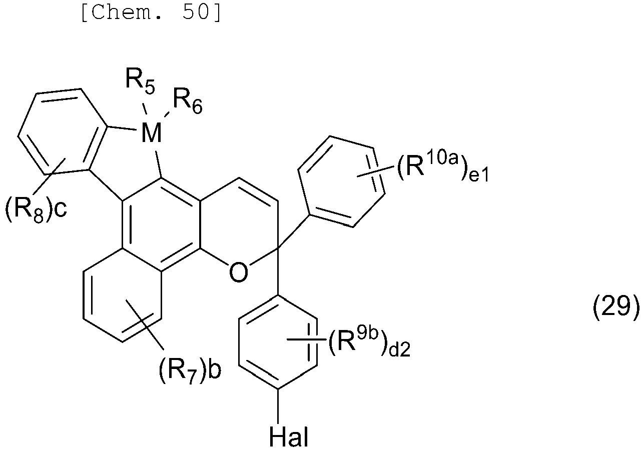

### <Identification of Photochromic Compound>

The photochromic compound according to the embodiment is, for example, a solid or viscous liquid at room temperature and atmospheric pressure. From this solid or liquid, a photochromic compound can be isolated by a separation operation such as a thin-layer chromatography, a silica gel column chromatography, a high-performance liquid chromatography, and a gas chromatography. Furthermore, it can be confirmed that the isolate does not include by-products other than the photochromic compound, such as raw material compounds and coloring components.

In a proton nuclear magnetic resonance spectroscopy (¹H-NMR), the obtained photochromic compound showed peaks attributed to aromatic protons and protons of an alkene at around δ: 5.0 to 9.0 ppm, and peaks attributed to protons of an alkyl group and an alkylene group at around δ: 1.0 to 4.0 ppm. When the intensities of each spectrum are relatively compared, the number of protons corresponding to each bond group can be determined. This makes it possible to identify the skeleton, substituents, and the like of the photochromic compound.

When the photochromic compound is contained in a cured product such as a resin, this resin is dissolved and subjected to the above-described separation methods to isolate the photochromic compound.

### <Photochromic Composition>

The photochromic compound according to the embodiment is soluble in general organic solvents such as toluene, chloroform, and tetrahydrofuran. The photochromic compound having the skeleton represented by formula (1) is dissolved in such a solvent to obtain a colorless transparent solution. When this solution is exposed to sunlight or ultraviolet ray, it rapidly develops color, and when it is blocked from sunlight or the like, it reversibly and rapidly returns to its original colorless state and exhibits a good photochromic function.

Furthermore, the photochromic compound according to the embodiment can be used in combination with other photochromic compounds having other structures, depending on an intended purpose. For example, the photochromic compound can be used in combination with other photochromic compounds for the purpose of achieving various color tones required for photochromic lenses. The photochromic compounds to be used in combination can be any known compound with no limitation. Examples of the photochromic compounds to be used in combination include indenonaphthopyran, naphthopyran, spirooxazine, spiropyran, fulgide, fulgimide, diarylethene, and the like. Above all, an indenonaphthopyran compound is particularly preferable because it can maintain uniform color tones during color development/fading, suppress color shift during color development accompanying degradation of the photochromic properties, and further reduce the initial coloring. It is preferable to adjust the color tone by using plural kinds of photochromic compounds in combination, because the combination can achieve both a high color optical density and a high color fading speed particularly at a high temperature and in addition has an excellent durability.

When the photochromic composition includes the photochromic compound according to the embodiment and other photochromic compounds, the compounding ratios of each photochromic compound are determined as appropriate depending on a desired color tone.

### <Photochromic Curable Composition>

The photochromic curable composition as the curable composition according to the embodiment contains the photochromic compound according to the embodiment, and at least one selected from a group consisting of a radical polymerizable monomer, a cationic polymerizable monomer, a compound having a polymerizable group, and a (thio) urethane (urea) polymer. In this case, the (thio)urethane (urea) polymer includes at least one selected from a group consisting of a urethane polymer, a thiourethane polymer, a urethaneurea polymer, and a thiourethaneurea polymer.

Preferably, the photochromic compound and photochromic composition according to the embodiment are used as a photochromic curable composition in combination with a polymerizable compound.

The photochromic curable composition depends on a color development intensity of the photochromic compound, selected lens materials, and a lens thickness, and therefore it is preferable that, although it is not always the case, the photochromic compound (or photochromic composition) is used in an amount of 0.001 to 10 parts by mass based on 100 parts by mass of the polymerizable compound. An optimal compounding ratio of this photochromic compound varies depending on an intended purpose. For example, there is a difference as described below between a case that the photochromic curable composition is used as a thin-film optical article and a case that the photochromic curable composition is used as a thick-film optical article.

### (Use as Thin-Film Optical Article)

For example, when the photochromic curable composition is formed into a thin film having a thickness of 10 µm or more and less than 1000 um, e.g. about 100 µm (polymer film with the polymerized photochromic curable composition), it is preferable that the color tone is adjusted by blending the photochromic compound (or photochromic composition) in an amount of 0.001 to 10 parts by mass based on 100 parts by mass of other polymerizable monomers.

### (Use as Thick-Film Optical Article)

When the photochromic curable composition is formed into a thick cured product (polymer formed product with the polymerized photochromic curable composition) having a thickness of e.g. 1 mm or more, it is preferable that the color tone is adjusted by blending the photochromic compound (or photochromic composition) according to the present invention in an amount of 0.001 to 1 part by mass based on 100 parts by mass of the thick cured product or other polymerizable monomers capable of providing a thick cured product.

### <Polymerizable Compound>

As described above, the photochromic compound is preferably used in combination with a polymerizable compound, as a photochromic curable composition. Examples of the polymerizable compound include a urethane or urea-based polymerizable compound capable of forming a urethane bond, a urea bond, or the like, a compound having a polymerizable group, a radical-polymerizable compound, an epoxy-based polymerizable compound, and the like. These polymerizable compounds are not particularly limited, but, for example, the polymerizable compound described in Patent Document 7 can be suitably used.

Above all, the following polymerizable compounds are particularly suitable for use.

### <Compound Having Polymerizable Group>

The compound having a polymerizable group is exemplified by a compound having an iso(thio)cyanate group. The iso(thio)cyanate compound is a compound having an isocyanate group or an isothiocyanate group, and may have both the isocyanate group and isothiocyanate group. Suitably, this compound is used in combination with a compound including active hydrogen described later. Examples of such an iso(thio)cyanate compound include, but are not limited to, the following compounds.

### (Polyiso(thio)cyanate)

Polyiso(thio)cyanates are compounds having at least two or more iso(thio)cyanate groups in one molecule. Examples of the polyiso(thio)cyanate include an aromatic polyiso(thio)cyanate having an aromatic ring, such as m-xylene diisocyanate and 4,4'-diphenylmethane diisocyanate, and an aliphatic polyiso(thio)cyanate such as norbornane diisocyanate and dicyclohexylmethane-4,4'-diisocyanate.

### (Compound Having Active Hydrogen)

The compound containing active hydrogen is preferably, but not limited to, a compound having a hydroxy group and/or a thiol group, particularly preferably a polyfunctional compound having two or more active hydrogen atoms in one molecule. Specific examples of the compound having active hydrogen include: a polyfunctional thiol compound such as pentaerythritol tetrakis(3-mercaptopropionate) and 4-mercaptomethyl-3,6-dithia-octanedithiol, and a polyfunctional alcohol such as trimethylolpropane and pentaerythritol.

### (Radical-Polymerizable Compound)

The radical-polymerizable compound includes polyfunctional radical-polymerizable compounds and monofunctional radical-polymerizable compounds. These compounds can be used alone or in combination with others. Examples of radical-polymerizable substituents include a group having an unsaturated double bond, i.e. a vinyl group (including a styryl group, a (meth)acrylic group, an allyl group, and the like).

The polyfunctional radical-polymerizable compound has two or more radical-polymerizable substituents in one molecule. This polyfunctional radical-polymerizable compound includes a first polyfunctional radical-polymerizable compound having 2 to 10 radical-polymerizable substituents and a second polyfunctional radical-polymerizable compound having more than 10 radical-polymerizable substituents.

The first polyfunctional radical-polymerizable compound is not particularly limited, but, more preferably, it has 2 to 6 radical-polymerizable substituents. Specific examples thereof are as follows.

(Polyfunctional (Meth)acrylic Acid Ester Compound) Ethylene glycol di(meth)acrylate, diethylene glycol di(meth)acrylate, triethylene glycol di(meth)acrylate, tetraethylene glycol di(meth)acrylate, ethylene glycol bisglycidyl(meth)acrylate, bisphenol A di(meth)acrylate, 2,2-bis(4-(meth)acryloyloxyethoxyphenyl)propane, 2,2-bis(3,5-dibromo-4-(meth)acryloyloxyethoxyphenyl)propane.

### (Polyfunctional Allyl Compound)

Diallyl phthalate, diallyl terephthalate, diallyl isophthalate, diallyl tartrate, diallyl epoxy succinate, diallyl fumarate, diallyl chlorendate, diallyl hexaphthalate, diallyl carbonate, allyl diglycol carbonate, trimethylol propane triallyl carbonate.

(Polyfunctional Thio(meth)acrylic Acid Ester Compound) 1,2-bis(methacryloylthio)ethane, bis(2-acryloylthioethyl)ether, 1,4-bis(methacryloylthiomethyl)benzene.

### (Vinyl Compound)

Divinyl benzene.

Examples of the second polyfunctional radically-polymerizable compound having more than 10 radical-polymerizable substituents include relatively high-molecular-weight compounds such as a silsesquioxane compound having a radical-polymerizable substituent and a polyrotaxane compound having a radical-polymerizable substituent.

The monofunctional radical-polymerizable compound has one radical-polymerizable substituent in its molecule, and specific examples thereof include, but are not limited to, the following compounds.

### (Unsaturated Carboxylic Acid)

Acrylic acid, methacrylic acid, maleic anhydride.

### ((Meth)acrylic Acid Ester)

Methyl (meth)acrylate, benzyl methacrylate, phenyl methacrylate.

2-hydroxyethyl methacrylate, glycidyl (meth)acrylate, β-methylglycidyl (meth)acrylate, bisphenol A-monoglycidyl ether-methacrylate, 4-glycidyl oxymethacrylate, 3-(glycidyl-2-oxyethoxy)-2-hydroxy, propyl methacrylate, 3-(glycidyloxy-1-isopropyloxy)-2-hydroxypropyl acrylate, 3-(glycidyloxy-2-hydroxypropyloxy)-2-hydroxypropyl acrylate.

### (Fumaric Acid Ester)

Diethyl fumarate, diphenyl fumarate.

### (Thio(meth)acrylic Acid)

Methyl thioacrylate, benzyl thioacrylate, benzyl thiomethacrylate.

### (Vinyl Compound)

Styrene, chlorostyrene, thylstyrene, vinyl naphthalene, α-methylstyrene dimer, bromostyrene.

The radical-polymerizable compound may be used alone or in combination of two or more types. In this case, based on 100 parts by mass of the total amount of the radical-polymerizable compounds, the amounts of the polyfunctional radical-polymerizable compound and the monofunctional radical-polymerizable compound are preferably 80 to 100 parts by mass and 0 to 20 parts by mass respectively, more preferably 90 to 100 parts by mass and 0 to 10 parts by mass respectively. Furthermore, based on 100 parts by mass of the total amount of the radical-polymerizable compound, the amounts of the first polyfunctional radical-polymerizable compound, the second polyfunctional radical-polymerizable compound, and the monofunctional radical polymerizable compound are preferably 80 to 100 parts by mass, 0 to 20 parts by mass, and 0 to 20 parts by mass respectively, more preferably 85 to 100 parts by mass, 0 to 10 parts by mass, and 0 to 10 parts by mass respectively.

### (Various Compounding Agents)

The curable composition may contain various known copounding agents unless the effects of the composition are impaired. Examples of compounding agents include various stabilizers such as release agents, ultraviolet absorbers, infrared absorbers, ultraviolet stabilizers, antioxidants, coloring inhibitors, antistatic agents, fluorescent dyes, dyes, pigments, and fragrances. Also, solvents and leveling agents may be blended. Thiols such as t-dodecylmercaptan may be blended as polymerization modifier.

Among the above compounding agents, ultraviolet stabilizers are suitable because they can improve the durability of the photochromic site. As such ultraviolet stabilizers, hindered amine light stabilizers, hindered phenol antioxidants, sulfur-based antioxidants, and the like are known. Particularly suitable ultraviolet stabilizers are as follows.

Bis(1,2,2,6,6-pentamethyl-4-piperidyl) sebacate; ADEKA Corporation's ADK STAB LA-52, LA-57, LA-62, LA-63, LA-67, LA-77, LA-82, LA-87; 2,6-di-tert-butyl-4-methylphenol, ethylene bis(oxyethylene) bis[3-(5-tert-butyl-4-hydroxy-m-tolyl) propionate]; BASF Japan's IRGANOX 1010, 1035, 1075, 1098, 1135, 1141, 1222, 1330, 1425, 1520, 259, 3114, 3790, 5057, 565. An amount used of such an ultraviolet stabilizer is not particularly limited unless the effects of the stabilizer are impaired, but is typically in a range of 0.001 to 10 parts by mass, particularly 0.01 to 1 part by mass based on 100 parts by mass of the photochromic curable composition.

In addition to the ultraviolet stabilizer, an ultraviolet absorber may also be used. As the ultraviolet absorber, a known ultraviolet absorber such as a benzophenone compound, a benzotriazole compound, a cyanoacrylate compound, a triazine compound, and a benzoate compound may be used. Above all, a cyanoacrylate compound and a benzophenone compound are preferable. Preferably, the above ultraviolet stabilizer is used in a range of 0.001 to 5 parts by mass based on 100 parts by mass of the photochromic curable composition containing a photochromic compound and a polymerizable compound.

### <Method for Using Photochromic Curable Composition; Optical Article>

A photochromic cured product is obtained by curing the photochromic curable composition. The polymerization curing for preparing the photochromic cured product is performed by irradiation with active energy rays such as ultraviolet ray, α-ray, β-ray, and γ-ray, heating, or a combination thereof, through a radical polymerization, a ring-opening polymerization, an anion polymerization, or a condensation polymerization. That means, an appropriate polymerization means should be adopted depending on the types of the polymerizable compound or polymerization curing promoter and the form of the photochromic cured product to be formed.

When thermally polymerizing a curable composition containing a polymerizable compound, the temperature affects the properties of the resulting photochromic cured product.

This temperature condition is affected by the type and amount of the thermal polymerization initiator and the type of the polymerizable compound, and therefore, although it cannot be generally limited, it is generally preferable to initiate the polymerization at a relatively low temperature and gradually increase the temperature. Since the polymerization time also varies depending on various factors like the temperature, it is preferable to previously determine the optimal time in accordance with these conditions. However, generally, it is desirable to select a polymerization condition such that the polymerization is completed within 2 to 48 hours. When obtaining a photochromic laminated sheet, it is preferable that the polymerization is performed at a temperature at which the reaction between polymerizable functional groups proceeds, and, at this time, the optimal temperature and time are determined such that the molecular weight is a desired weight.

Moreover, when photopolymerizing the curable composition, particularly the UV intensity among the polymerization condition affects the properties of the resulting photochromic cured product. This illuminance condition is affected by the type and amount of the photoinitiator and the type of the polymerizable monomer, and therefore, although it cannot be generally limited, it is generally preferable to select a condition such that UV light at a wavelength of 365 nm having an intensity of 50 to 500 mW/cm² is emitted for 0.5 to 5 minutes.

### [Optical Article]

The photochromic compound according to the embodiment can be widely used as a photochromic material, e.g. as various memory materials that are alternatives for silver halide photosensitive materials, copying materials, printing photoreceptors, cathode ray tube memory materials, laser photosensitive materials, and various memory materials such as holography photosensitive materials. Photochromic materials can also be used as photochromic lens materials, optical filter materials, display materials, as well as materials for actinometers, decoration, fabric, string, and the like.

The photochromic compound according to the embodiment is suitable particularly for photochromic lens applications. Photochromic lenses are suitable for eyeglasses lenses such as sunglass lenses. As the method for producing the photochromic lens, any known method can be adopted as long as a uniform dimming performance can be achieved.

When photochromic properties are exhibited by a kneading method, the above-described curable composition is injected into between glass molds held by an elastomer gasket or spacer, and then cast-polymerized by heating in an air furnace or irradiation with an active energy ray such as ultraviolet ray depending on the types of the polymerizable compound and polymerization curing promoter, resulting in a photochromic cured product formed into an optical material such as a lens.

When exhibiting photochromic properties by a lamination method, the curable composition is dissolved in an appropriate organic solvent to prepare a coating liquid, which is then applied on a surface of an optical substrate such as a lens substrate by spin coating, dipping, or the like. The coating liquid is dried to remove the organic solvent, and then cured by polymerization with UV irradiation, heating, or the like in an inert gas such as nitrogen, to form a photochromic layer consisting of the photochromic cured product on the surface of the optical substrate (coating method).

Alternatively, an optical substrate such as a lens substrate is arranged so as to face a glass mold such that predetermined voids are formed, and a curable composition is injected into these voids. In this state, a photochromic layer consisting of a photochromic cured product can be formed on the surface of the optical substrate also by a cast polymerization using an inner mold in which polymerization curing is performed by UV irradiation, heating, or the like (cast polymerization method).

When forming a photochromic layer on a surface of an optical substrate using the lamination method as described above (coating method and cast polymerization method), the adhesiveness between the photochromic layer and the optical substrate can be improved by previously subjecting a surface of the optical substrate to a chemical treatment using an alkaline solution, an acidic solution, or the like, or a physical treatment using corona discharge, plasma discharge, polishing, or the like. Needless to say, it is also possible to provide a transparent adhesive resin layer on the surface of the optical substrate.

Furthermore, when acquiring the photochromic properties by a binder method, a photochromic sheet is prepared by sheet forming using a curable composition, this sheet is inserted into between two transparent sheets (optical sheets) and then cured by polymerization as described above, to obtain a photochromic binder sheet having a photochromic layer as an adhesive layer.

In this case, for the preparation of the photochromic sheet, it is also possible to adopt a method in which a curable composition is dissolved in an organic solvent to prepare a coating liquid, and this coating liquid is used for coating. An adhesive layer may be provided between the photochromic sheet and the optical sheet.

For example, the binder sheet prepared in this manner is placed in a mold, onto which a thermoplastic resin (e.g. polycarbonate) for an optical substrate such as lens is injection-formed, to obtain an optical substrate such as lens in a predetermined shape having photochromic properties.

Moreover, this binder sheet can be bonded to the surface of the optical substrate using an adhesive or the like to obtain a photochromic lens.

In addition, the photochromic layer or photochromic cured product prepared from the curable composition can be subjected to post-processings e.g. dying using dyes such as dispersant dyes, formation of a hard coat film using a silane coupling agent, or a hard coating agent predominantly containing a sol of silicon, zirconium, antimony, aluminum, tin, tungsten, etc., formation of a thin film by vapor deposition of metal oxides such as SiO₂, TiO₂, and ZrO₂, antireflection treatment using a thin film formed by application of organic polymers, anti-static treatment, and protective coating treatment with a urethane resin or the like.

### EXAMPLES

The present invention will be explained in more detail with reference to Examples below. These Examples are intended to merely explain the present invention, and the spirit and scope of the present invention are not limited to these Examples.

### (Example 1)

### First Step

95.4 g (500 mmol) of p-toluenesulfonic acid monohydrate and 1000 mL of toluene were mixed, the mixture was subjected to azeotropic dehydration, and it was confirmed that a water content was 202 ppm.

By referring to the method described in Patent Document 8, 37.3 g (100.0 mmol) of a carboxylic acid compound represented by formula (30) below synthesized from 3-bromo-4-methoxybenzophenone was added to the mixture, which was reacted while performing azeotropic dehydration. After confirming that the raw materials had been consumed, the mixture was cooled to room temperature, and then the resulting solid was filtrated to obtain a carbonyl compound represented by formula (31) below with a yield of 90%.

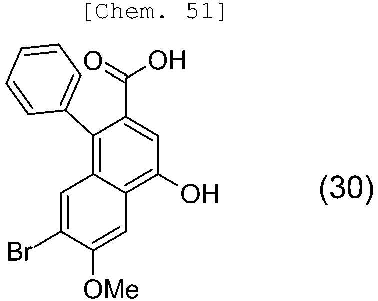

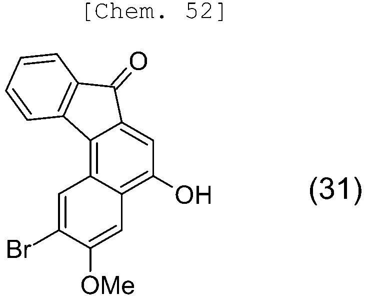

### Second Step

By referring to the method described in Patent Document 9, a reaction was performed using the carbonyl compound represented by formula (31) above to obtain a naphthol derivative represented by formula (32) below with a yield of 83%.

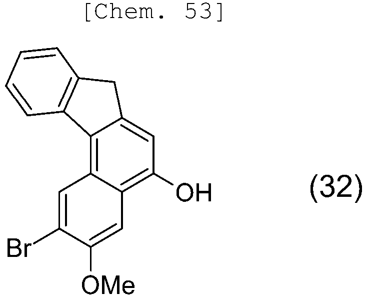

### Third Step

To 17.1 g (49.9 mmol) of the naphthol derivative represented by formula (32) above, 11.2 g (54.3 mmol) of 4-morpholinophenylboronic acid, 11.5 g (108.7 mmol) of sodium carbonate, 103.5 mL of water, 121.5 mL of 1,2-dimethoxyethane, and 12.2 mL of ethanol were added, and stirred while carrying out nitrogen bubbling. The nitrogen bubbling was continued for about 20 minutes, then 142.7 mg (0.1 mmol) of Pd(PPh₃)₄ was added to the mixture, which was reacted at 75°C for 2 hours. After the reaction, the mixture was cooled to room temperature, to which 650 mL of THF was added, and the mixture was cooled to 0 to 5°C, then adjusted to pH 1 by adding concentrated hydrochloric acid, and subjected to liquid separation. The mixture was washed twice with 500 mL of water to remove the solvent. The mixture was purified by reslurry with 300 mL of methanol to obtain a naphthol derivative represented by formula (33) below with a yield of 96%.

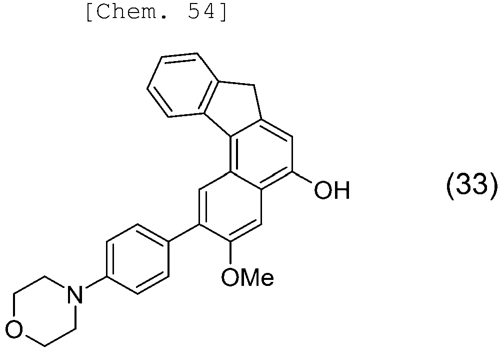

### Fourth Step

To 50 mL of toluene, 2.91 g (10.0 mmol) of 4-bromo-4'-methoxybenzophenone, 1.50 g (14.0 mmol) of N-methylaniline, and 0.29 g (30.0 mmol) of sodium t-butoxide were added, and the mixture was subjected nitrogen bubbling for 20 minutes. To this mixture, 46 mg (0.05 mmol) of tris(dibenzylideneacetone)dipalladium(0), 95 mg (0.20 mmol) of 2-dicyclohexylphosphino-triisopropylbiphenyl were added, and the mixture was reacted at 80°C for 3 hours. After completion of the reaction, the mixture was cooled to room temperature, and then 30 mL of THF was added to the mixture, which was filtrated. The mixture was cooled to 0 to 5°C, and 10% hydrochloric acid was added to the mixture until the pH reached 6 to 7. The mixture was subjected to liquid separation, and washed three times with 100 mL of water. From the obtained organic layer, the solvent was removed, and the organic layer was purified by a chromatography on silica gel to obtain a benzophenone represented by formula (34) below with a yield of 92%.

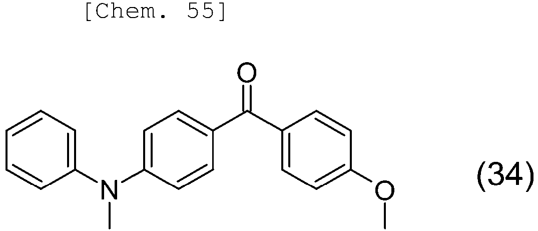

By referring to the method described in Patent Document 6, the obtained benzophenone was used to obtain a propargyl alcohol represented by formula (35) below with a yield of 86%.

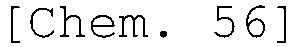

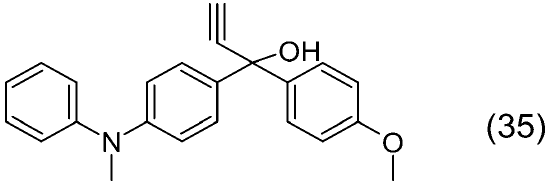

### Fifth Step

First, 2.80 g (6.6 mol) of the naphthol derivative represented by formula (33) above, 5.60 g of WAKOGEL C300, and 80 mL of toluene were mixed, and heated to 100°C. To this mixture, 2.71 g (7.9 mmol) of the propargyl alcohol represented by formula (35) above dissolved in 20 mL of toluene was added, and heated at 100°C. After consuming the naphthol derivative as the raw material, the mixture was cooled to room temperature, and 40 mL of water was added to the mixture, which was subjected to liquid separation. From the obtained organic layer, the solvent was removed, and the organic layer was purified by a chromatography on silica gel to obtain a photochromic compound represented by formula (36) below with a yield of 87%.

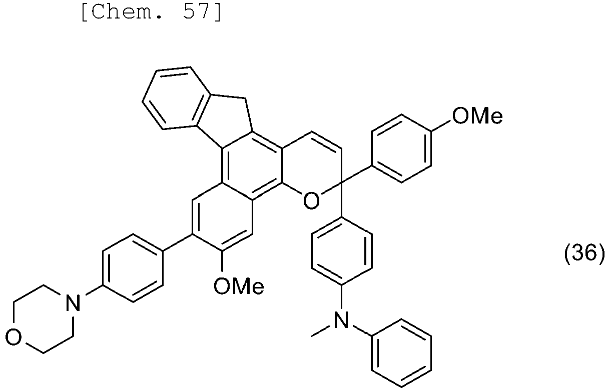

### Sixth Step

4.1 g (5.5 mmol) of the photochromic compound represented by formula (36) above, 3.9 g (27.5 mmol) of iodomethane, and 60 mL of THF were mixed, and cooled with ice. To the mixture, 1.8 g (16.5 mmol) of tBuOK was added in four batches while maintaining the temperature at 0 to 5°C. After confirming that the raw materials had been consumed, the mixture was neutralized with 10% hydrochloric acid, to which 30 mL of toluene was added, and the mixture was subjected to liquid separation. From the obtained organic layer, the solvent was removed, and then the organic layer was purified by a chromatography on silica gel to obtain a photochromic compound represented by formula (37) below with a yield of 89%.

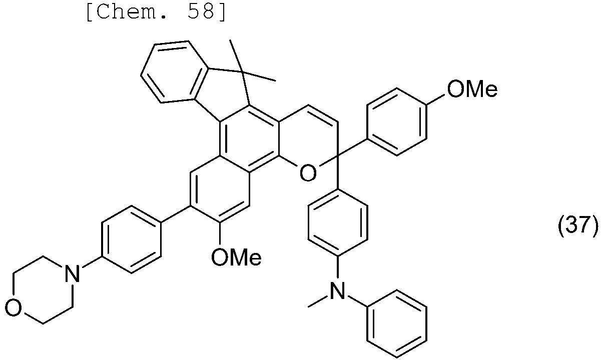

The photochromic compound represented by formula (37) above had elemental analysis values of C: 81.93%, H: 6.21%, and N: 3.62%, which were considerably consistent with the C₅₃H₄₈N₂O₄ calculated values of C: 81.93%, H: 6.23%, and N: 3.61%.

In a proton nuclear magnetic resonance spectroscopy, the photochromic compound represented by formula (37) showed 13H peaks attributed to a methyl group and a morpholino group at around δ0.5 to 3.0 ppm, 10H peaks attributed to a methoxy group and a morpholino group at around δ3.0 to 5.0 ppm, and 25H peaks attributed to aromatic protons and protons of an alkene at around δ5.0 to 9.0 ppm.

Furthermore, in a ¹³C-nuclear magnetic resonance spectroscopy, the photochromic compound represented by formula (37) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 2)

### First Step

By referring to the method described in Patent Document 8, 31.8 g (68.6 mmol) of compound represented by formula (38) below synthesized from the carboxylic acid compound represented by formula (30) above, 550 mL of toluene, 14.79 g (87.4 mmol) of diphenyl amine, and 30.8 g (274.6 mmol) of t-butoxy sodium were mixed, and stirred under reduced pressure, from which dissolved oxygen was removed. Subsequently, 0.57 g (0.6 mmol) of Pd₂(dba)₃ and 1.19 g (2.5 mmol) of X-phos were added to the reaction liquid, which was heated to 80°C. The heating was continued until the raw materials became invisible, and after completion of the reaction, the reaction liquid was cooled to room temperature and then filtrated. To the resulting filtrate, 500 mL of tetrahydrofuran and 10% hydrochloric was added, which was neutralized and then subjected to liquid separation. The resulting organic layer was concentrated and then purified by reslurry with 200 mL of methanol to obtain a carboxylic acid compound represented by formula (39) below with a yield of 87%.

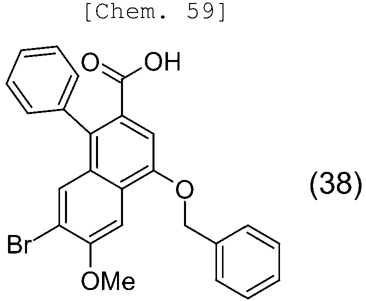

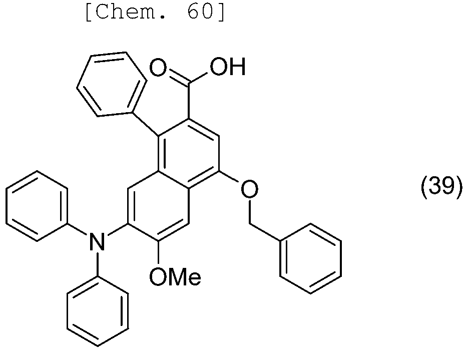

### Second Step

By referring to the method described in Patent Document 8 except that the carboxylic acid compound obtained in the first step was used, an iodine compound represented by formula (40) below was obtained with a yield of 75%.

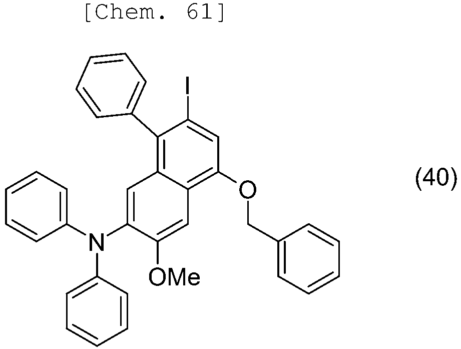

### Third Step

To 28.4 g (44.8 mmol) of the compound represented by formula (40) above obtained in the second step, 400 mL of toluene was added, and subjected to azeotropic dehydration until the water content in toluene decreased to no more than 100 ppm. After the azeotropic dehydration, the mixture was slowly cooled to - 20°C, into which 33.6 mL of n-BuLi (1.6 mol/L hexane solution) was slowly dripped while maintaining the temperature at -15 to -20°C. After confirming that the raw materials had been consumed, 8.64 g (56.0 mmol) of 4,4-diethylcyclohexanone was slowly dripped into the mixture while maintaining the temperature at -5 to -20°C. After the dripping, the temperature was slowly raised to room temperature. After the temperature rising, 200 mL of water was added to the mixture, which was subjected to liquid separation. The mixture was repeatedly washed with water until a pH of a water layer reached 7 to 8. From the obtained organic layer, the solvent was removed, and the organic layer was purified by a chromatography on silica gel to obtain a compound represented by formula (41) below with a yield of 86%.

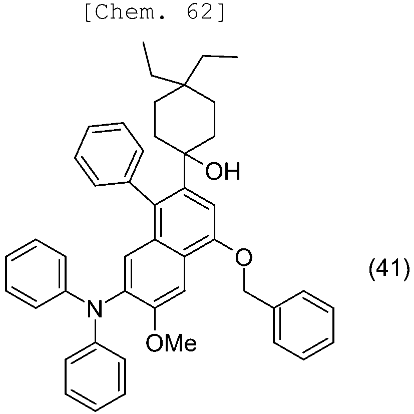

### Fourth Step

To 25.5 g (38.5 mmol) of the compound represented by formula (41) above, 500 mL of THF was added and dissolved, then 7.6 g of 5% Pd/C (water content: 50%) was added, which was reacted in hydrogen gas under pressure at 0.05 to 0.1 MPa. After confirming that the raw materials had been consumed, the Pd/C was filtered off, and, from the obtained organic layer, the solvent was removed to obtain a compound represented by formula (42) below with a yield of 100%.

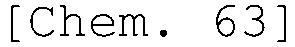

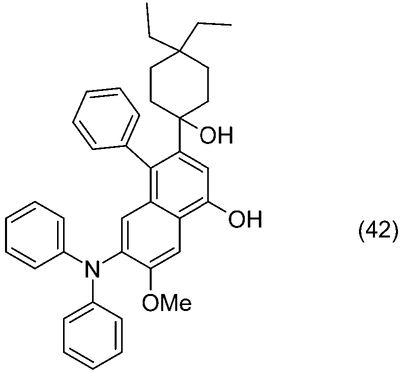

### Fifth Step

To 14.7 g (77.0 mmol) of p-toluenesulfonic acid monohydrate, 750 mL of toluene was added, which was subjected to azeotropic dehydration until the water content in toluene decreased to no more than 150 ppm. Then, into this mixture, 100 mL of a toluene solution containing 22.0 g (38.5 mmol) of the compound represented by formula (42) above was slowly dripped while maintaining the temperature at 85 to 100°C, and after the dripping, the mixture was refluxed. After confirming that the raw materials had been consumed, the mixture was cooled to room temperature, to which 500 mL of water was added, which was subjected to liquid separation. This operation was repeated three times, the solvent was removed from the obtained organic layer, and the organic layer was purified by a chromatography on silica gel to obtain a naphthol derivative represented by formula (43) below with a yield of 83%.

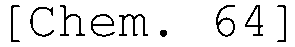

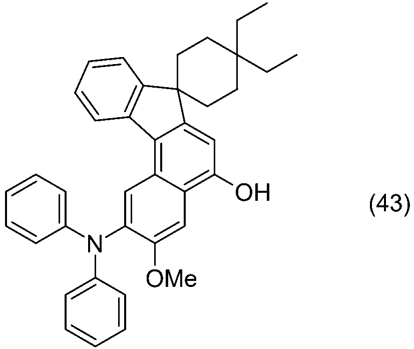

### Fifth Step

The same reaction as in the fourth step of Example 1 was performed except that 4-bromo-4'-methylbenzophenone was used instead of 4-bromo-4'-methoxybenzophenone and 4-(methylamino)toluene was used instead of N-methylaniline, to obtain a propargyl alcohol represented by formula (44) below with a yield of 89%.

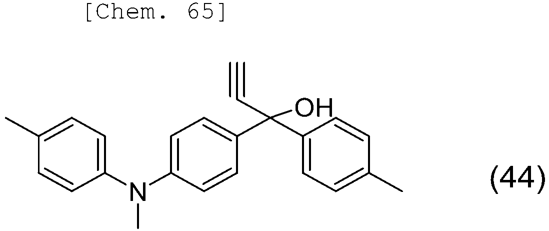

### Sixth Step

3.05 g (5.5 mmol) of the naphthol derivative represented by formula (43) above, 6.00 g of WAKOGEL C300, and 60 mL of toluene were mixed, and heated to 100°C. To this mixture, 10 mL of toluene solution containing 2.26 g (6.6 mmol) of the propargyl alcohol represented by formula (44) above was dripped so that the internal temperature was at 95 to 100°C. After confirming that the raw materials had been consumed, the mixture was filtrated, and from the obtained organic layer, the solvent was removed, and the organic layer was purified by a chromatography on silica gel to obtain a photochromic compound represented by formula (45) below with a yield of 77%.

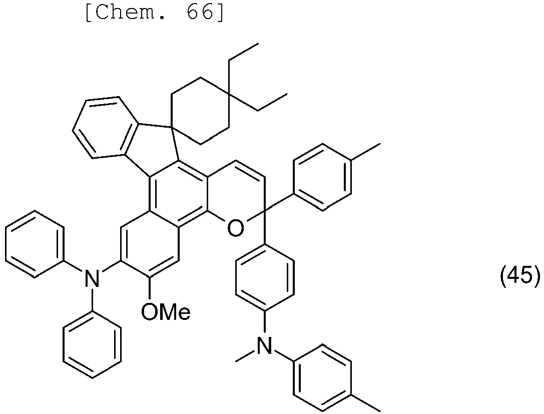

The photochromic compound represented by formula (45) above had elemental analysis values of C: 86.29%, H: 6.87%, and N: 3.21%, which were considerably consistent with the C₆₃H₆₀N₂O₂ calculated values of C: 86.26%, H: 6.89%, and N: 3.19%.

In a proton nuclear magnetic resonance spectroscopy, the photochromic compound represented by formula (45) showed 27H peaks attributed to a methyl group and a 4,4-diethylcyclohexyl ring group at around δ0.5 to 3.0 ppm, a 3H peak attributed to a methoxy group at around δ3.0 to 5.0 ppm, and 30H peaks attributed to aromatic protons and protons of an alkene at around δ5.0 to 9.0 ppm.

Furthermore, in a ¹³C-nuclear magnetic resonance spectroscopy, the photochromic compound represented by formula (45) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 3)

The same reaction as in the first step of Example 1 was performed except that 4,4'-dimethoxybenzophenone was used instead of 3-bromo-4-methoxybenzophenone, and the same reaction as in the sixth step of Example 1 was performed except that iodopropane was used instead of iodomethane, to obtain a photochromic compound represented by formula (46) below with a yield of 82%.

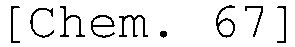

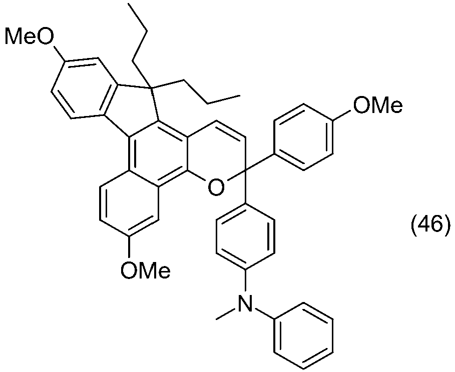

The photochromic compound represented by formula (46) above had elemental analysis values of C: 82.11%, H: 6.76%, and N: 2.01%, which were considerably consistent with the C₄₈H₄₇NO₄ calculated values of C: 82.14%, H: 6.75%, and N: 2.00%.

In a proton nuclear magnetic resonance spectroscopy, the photochromic compound represented by formula (46) showed 17H peaks attributed to a methyl group and a propyl group at around δ0.5 to 3.0 ppm, a 9H peak attributed to a methoxy group at around δ3.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (46) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 4)

### First Step

The same reaction as in the fourth step of Example 1 was performed except that 4-bromo-4'-fluorobenzophenone was used instead of 4-bromo-4'-methoxybenzophenone and N-ethylaniline was used instead of N-methylaniline, to obtain a propargyl alcohol represented by formula (47) below with a yield of 91%.

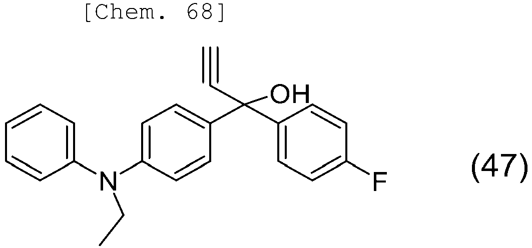

### Second Step

The same reaction as in the first step of Example 1 was performed except that 4-methoxybenzophenone was used instead of 3-bromo-4-methoxybenzophenone, and the same reaction as in the sixth step of Example 1 was performed except that iodohexane was used instead of iodomethane, to obtain a photochromic compound represented by formula (48) below with a yield of 79%.

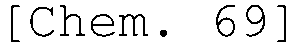

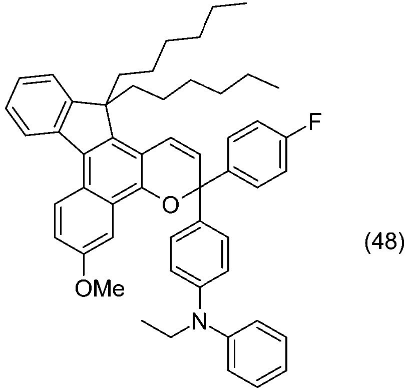

The photochromic compound represented by formula (48) above had elemental analysis values of C: 83.96%, H: 7.43%, and N: 1.84%, which were considerably consistent with the C₅₃H₅₆FNO₄ calculated values of C: 83.98%, H: 7.45%, and N: 1.85%.

In a proton nuclear magnetic resonance spectroscopy, the photochromic compound represented by formula (48) showed 31H peaks attributed to an ethyl group and a hexyl group at around δ0.5 to 3.0 ppm, a 3H peak attributed to a methoxy group at around δ3.0 to 5.0 ppm, and 22H peaks attributed to aromatic protons and protons of an alkene at around δ5.0 to 9.0 ppm.

Furthermore, in a ¹³C-nuclear magnetic resonance spectroscopy, the photochromic compound represented by formula (48) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 5)

### First Step

The same reaction as in the fourth step of Example 1 was performed except that 4-bromo-4'-propylbenzophenone was used instead of 4-bromo-4'-methoxylbenzophenone, to obtain a propargyl alcohol represented by formula (49) below with a yield of 92%.

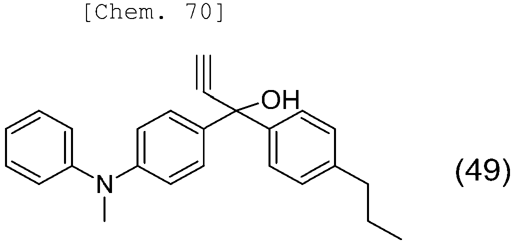

### Second Step

The same reaction as in the fifth step of Example 2 was performed except that a naphthol derivative represented by formula (50) below synthesized by referring to the method described in Patent Document 10 was used instead of the naphthol derivative represented by formula (43) above and the propargyl alcohol represented by formula (49) above was used instead of the propargyl alcohol represented by formula (44) above, to obtain a photochromic compound represented by formula (51) below with a yield of 82%.

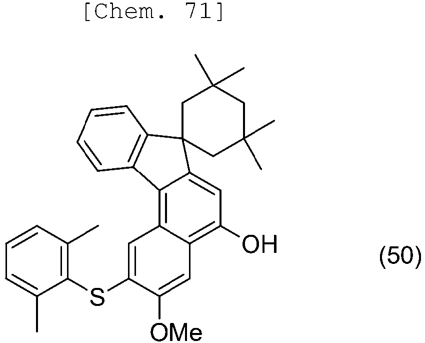

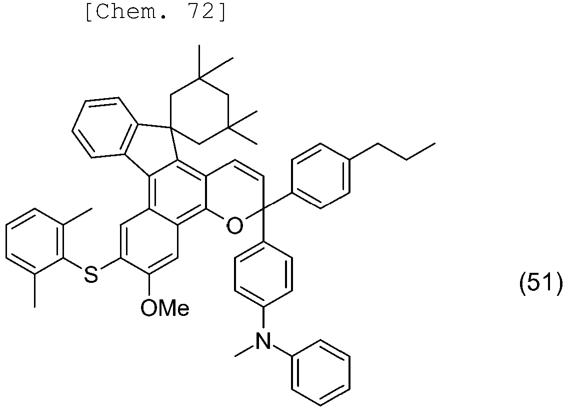

The photochromic compound represented by formula (51) above had elemental analysis values of C: 83.77%, H: 7.15%, N: 1.64%, and S: 3.75%, which were considerably consistent with the C₆₀H₆₁NO₂S calculated values of C: 83.78%, H: 7.15%, N: 1.63%, and S: 3.73%.

In a proton nuclear magnetic resonance spectroscopy, the photochromic compound represented by formula (51) showed 34H peaks attributed to a methyl group, a propyl group, and a 3,3,5,5-tetramethylcyclohexyl ring group at around δ0.5 to 3.0 ppm, a 3H peak attributed to a methoxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (51) showed a peak attributed to carbon of an aromatic ring at around 5110 to 160 ppm, a peak attributed to carbon of an alkene at around δ80 to 140 ppm, and a peak attributed to carbon of an alkyl at δ20 to 60 ppm.

### (Example 6)

### First Step

The same reaction as in the fourth step of Example 1 was performed except that N-cyclohexylaniline was used instead of N-methylaniline, to obtain a propargyl alcohol represented by formula (52) below with a yield of 89%.

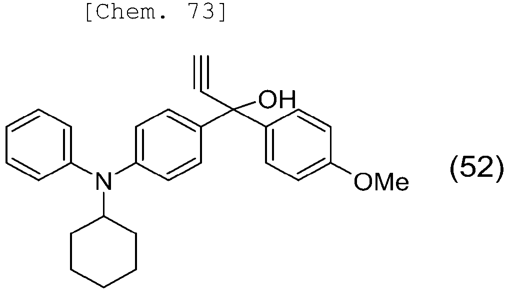

### Second Step

The same reaction as in the first step of Example 1 was performed except that 3,4-dimethoxybenzophenone was used instead of 3-bromo-4-methoxybenzophenone, the same reaction as in the fifth step of Example 1 was performed except that the propargyl alcohol represented by formula (52) above was used instead of the propargyl alcohol represented by formula (35) above, and the same reaction as in the sixth step of Example 1 was performed except that 1-bromo-4-fluorobutane was used instead of iodomethane, to obtain a photochromic compound represented by formula (53) below with a yield of 69%.

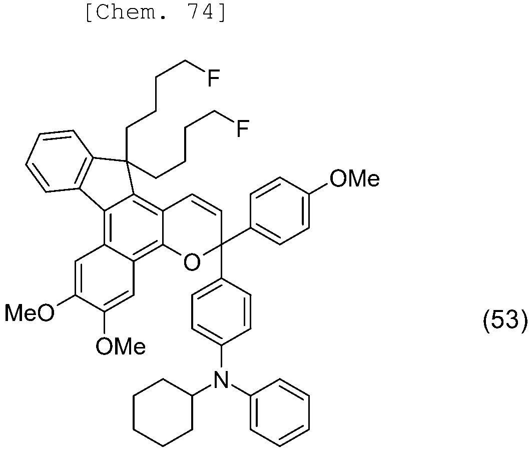

The photochromic compound represented by formula (53) above had elemental analysis values of C: 79.18%, H: 6.88%, and N: 1.66%, which were considerably consistent with the C₅₅H₅₇F₂NO₄ calculated values of C: 79.20%, H: 6.89%, and N: 1.68%.

In a proton nuclear magnetic resonance spectroscopy, the photochromic compound represented by formula (53) showed 27H peaks attributed to a cyclohexyl group and a 4-fluorobutyl group at around δ0.5 to 3.0 ppm, a 9H peak attributed to a methoxy group at around 63.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (53) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 7)

### First Step

The same reaction as in the fourth step of Example 1 was performed except that 4-bromo-3'-fluoro-4'-methoxybenzophenone was used instead of 4-bromo-4'-methoxylbenzophenone, to obtain a propargyl alcohol represented by formula (54) below with a yield of 91%.

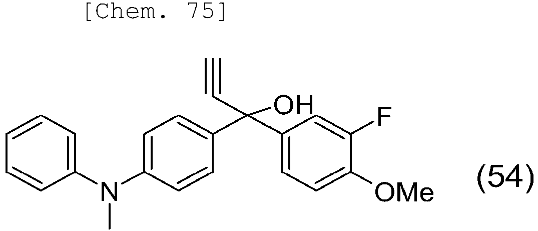

### Second Step

The same reaction as in the third step of Example 1 was performed except that 2,4-dimethoxyphenylboronic acid was used instead of 4-morpholinophenylboronic acid, the same reaction as in the fifth step of Example 1 was performed except that the propargyl alcohol represented by formula (54) above was used instead of the propargyl alcohol represented by formula (35) above, and the same reaction as in the sixth step of Example 1 was performed except that iodopropane was used instead of iodomethane, to obtain a photochromic compound represented by formula (55) below with a yield of 73%.

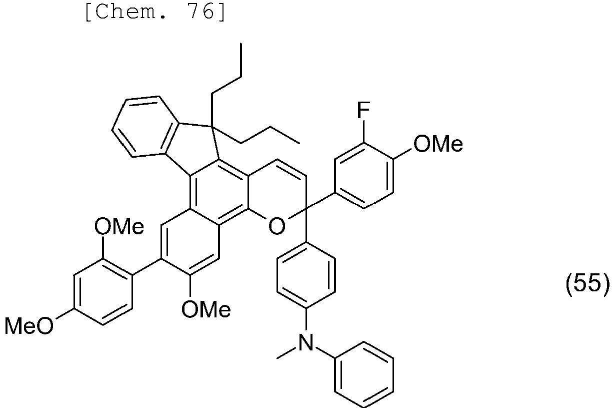

The photochromic compound represented by formula (55) above had elemental analysis values of C: 79.95%, H: 6.34%, and N: 1.69%, which were considerably consistent with the C₅₅H₅₂FNO₅ calculated values of C: 79.97%, H: 6.35%, and N: 1.70%.

In a proton nuclear magnetic resonance spectroscopy, the photochromic compound represented by formula (55) showed 17H peaks attributed to a methyl group and a propyl group at around δ0.5 to 3.0 ppm, a 12H peak attributed to a methoxy group at around 63.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (55) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 8)

### First Step

The same reaction as in the fourth step of Example 1 was performed except that 4-bromo-3',4'-dimethoxybenzophenone was used instead of 4-bromo-4'-methoxylbenzophenone and 4-fluoro-N-methylaniline was used instead of N-methylaniline, to obtain a propargyl alcohol represented by formula (56) below with a yield of 91%.

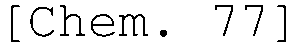

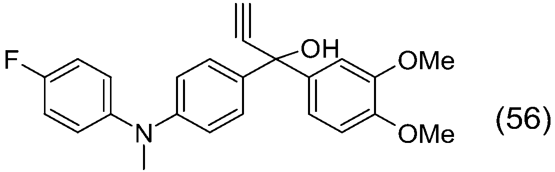

### Second Step

The same reaction as in the first step of Example 1 was performed except that 4,4'-dimethylbenzophenone was used instead of 3-bromo-4-methoxylbenzophenone, the same reaction as in the fifth step of Example 1 was performed except that the propargyl alcohol represented by formula (56) above was used instead of the propargyl alcohol represented by formula (35) above, and the same reaction as in the sixth step of Example 1 was performed except that 1-bromo-2-(2-methoxyethoxy)ethane was used instead of iodomethane, to obtain a photochromic compound represented by formula (57) below with a yield of 68%.

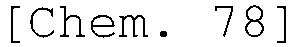

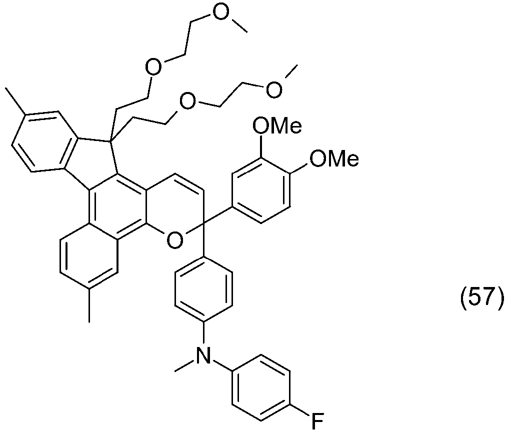

The photochromic compound represented by formula (57) above had elemental analysis values of C: 75.95%, H: 6.74%, and N: 1.69%, which were considerably consistent with the C₅₃H₅₆FNO₇ calculated values of C: 75.96%, H: 6.74%, and N: 1.67%.

In a proton nuclear magnetic resonance spectroscopy, the photochromic compound represented by formula (57) showed 13H peaks attributed to a methyl group and a 2-(2-methoxyethoxy)ethyl group at around δ0.5 to 3.0 ppm, a 24H peak attributed to a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (57) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 9)

### First Step

The same reaction as in the fourth step of Example 1 was performed except that 4-bromo-4'-propoxybenzophenone was used instead of 4-bromo-4'-methoxybenzophenone and N-(2,2,2-trifluoroehtyl)aniline was used instead of N-methylaniline, to obtain a propargyl alcohol represented by formula (58) below with a yield of 87%.

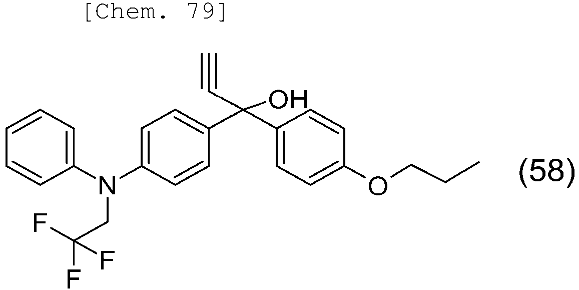

The same reaction as in the fifth step of Example 2 was performed except that a naphthol derivative represented by formula (59) below was used instead of the naphthol derivative represented by formula (43) above and the propargyl alcohol represented by formula (58) above was used instead of the propargyl alcohol represented by formula (44) above, to obtain a photochromic compound represented by formula (60) below with a yield of 74%.

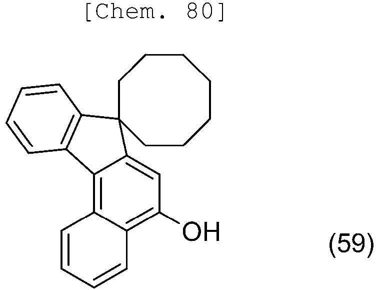

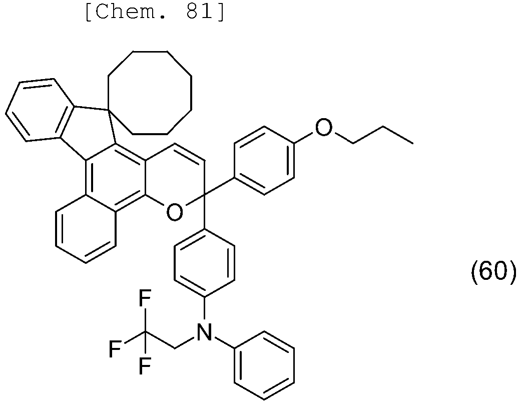

The photochromic compound represented by formula (60) above had elemental analysis values of C: 80.09%, H: 6.16%, and N: 1.85%, which were considerably consistent with the C₅₀H₄₆F₃NO₂ calculated values of C: 80.08%, H: 6.18%, and N: 1.87%.

In a proton nuclear magnetic resonance spectroscopy, the photochromic compound represented by formula (60) showed 21H peaks attributed to a cyclooctane ring group, a (2,2,2-trifluoroethyl group, and a propyloxy group at around δ0.5 to 3.0 ppm, a 2H peak attributed to a propyloxy group at around δ3.0 to 5.0 ppm, and 23H peaks attributed to aromatic protons and protons of an alkene at around 65.0 to 9.0 ppm.

Furthermore, in a ¹³C-nuclear magnetic resonance spectroscopy, the photochromic compound represented by formula (60) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 10)

### First Step

The same reaction as in the fifth step of Example 2 was performed except that N-butylaniline was used instead of 4-(methylamino)toluene, to obtain a propargyl alcohol represented by formula (61) below with a yield of 91%.

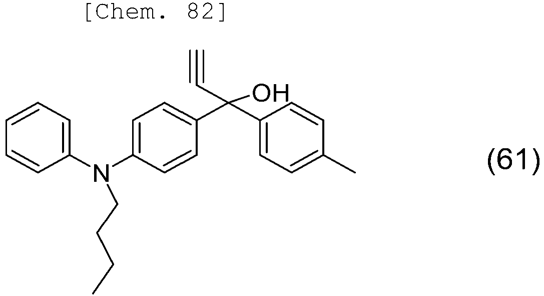

### Second Step

The same reaction as in the first step of Example 2 was performed except that morpholine was used instead of diphenylamine, the same reaction as in the third step of Example 2 was performed except that diethylketone was used instead of 4,4-diethylcyclohexanone, and the same reaction as in the fifth step of Example 2 was performed except that the propargyl alcohol represented by formula (61) above was used instead of the propargyl alcohol represented by formula (44) above, to obtain a photochromic compound represented by formula (62) below with a yield of 77%.

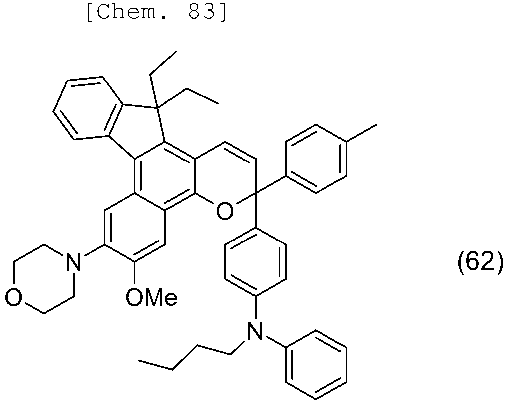

The photochromic compound represented by formula (62) above had elemental analysis values of C: 82.74%, H: 7.19%, and N: 3.72%, which were considerably consistent with the C₅₂H₃₄N₂O₃ calculated values of C: 82.72%, H: 7.21%, and N: 3.71%.

In a proton nuclear magnetic resonance spectroscopy, the photochromic compound represented by formula (62) showed 26H peaks attributed to a methyl group, an ethyl group, a butyl group, and a morpholino group at around δ0.5 to 3.0 ppm, 7H peaks attributed to a methoxy group and a morpholino group at around δ3.0 to 5.0 ppm, and 21H peaks attributed to aromatic protons and protons of an alkene at around 65.0 to 9.0 ppm.

Furthermore, in a ¹³C-nuclear magnetic resonance spectroscopy, the photochromic compound represented by formula (62) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 11)

### First Step

The same reaction as in the fourth step of Example 1 was performed except that 4-methoxy-N-methylaniline was used instead of N-methylaniline, to obtain a propargyl alcohol represented by formula (63) below with a yield of 93%.

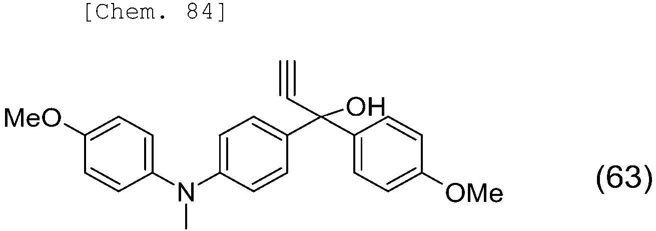

### Second Step

The same reaction as in the first step of Example 1 was performed except that 4-bromo-4'-methoxybenzophenone was used instead of 3-bromo-4-methoxybenzophenone, the same reaction as in the fifth step of Example 1 was performed except that the propargyl alcohol represented by formula (63) above was used instead of the propargyl alcohol represented by formula (35) above, and the same reaction as in the sixth step of Example 1 was performed except that 1-bromo-4-methoxybutane was used instead of iodomethane, to synthesize a photochromic compound represented by formula (64) below.

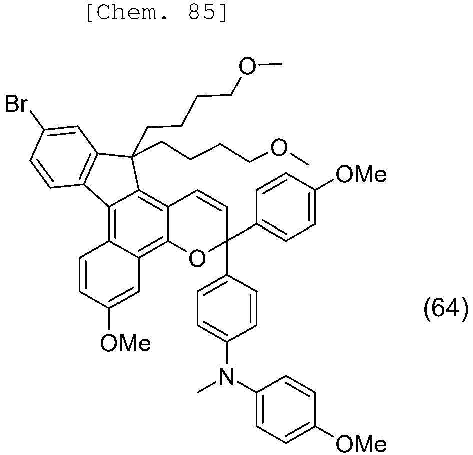

### Third Step

By referring to the method described in Patent Document 10, the photochromic compound represented by formula (64) above was reacted with 2,6-dimethylbenzenethiol to obtain a photochromic compound represented by formula (65) below with a yield of 86%.

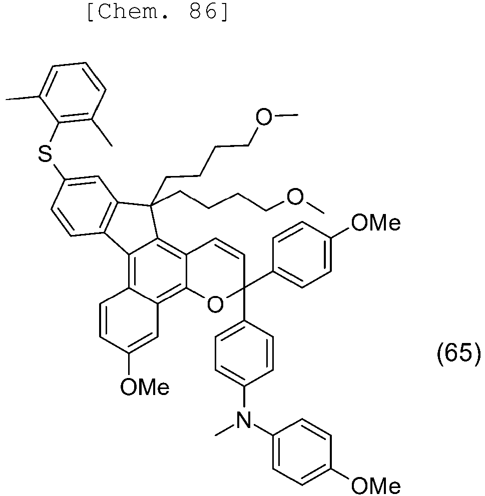

The photochromic compound represented by formula (65) above had elemental analysis values of C: 77.83%, H: 6.86%, N: 1.50%, and S: 3.44%, which were considerably consistent with the C₆₀H₆₃NO₆S calculated values of C: 77.81%, H: 6.86%, N: 1.51%, and S: 3.46%.

In a proton nuclear magnetic resonance spectroscopy, the photochromic compound represented by formula (65) showed 21H peaks attributed to a methyl group and a 4-methoxybutyl group at around δ0.5 to 3.0 ppm, 19H peaks attributed to a methoxy group and a 4-methoxybutyl group at around δ3.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (65) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 12)

### First Step

The same reaction as in the first step of Example 1 was performed except that 3-bromo-4-methoxy-4'-methylbenzophenone was used instead of 3-bromo-4-methoxybenzophenone, the same reaction as in the third step of Example 1 was performed except that 2,4,6-trimethoxyphenylboronic acid was used instead of 4-morpholinophenylboronic acid, and the same reaction as in the sixth step of Example 1 was performed except that 1-bromo-3-methylthiopropane was used instead of iodomethane, to obtain a photochromic compound represented by formula (66) below with a yield of 74%.

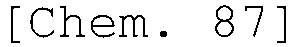

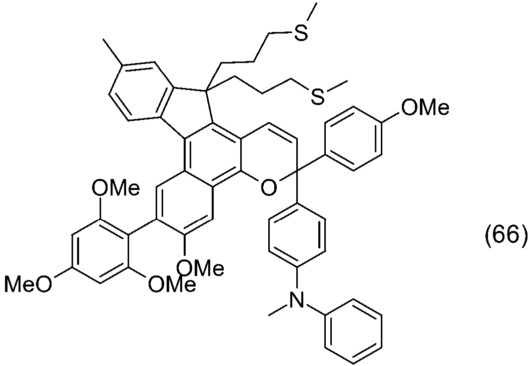

The photochromic compound represented by formula (66) above had elemental analysis values of C: 75.07%, H: 6.50%, N: 1.48%, and S: 6.80%, which were considerably consistent with the C₅₉H₆₁NO₆S₂ calculated values of C: 75.05%, H: 6.51%, N: 1.48%, and S: 6.79%.

In a proton nuclear magnetic resonance spectroscopy, the photochromic compound represented by formula (66) showed 24H peaks attributed to a methyl group and a 3-methylthiopropyl group at around δ0.5 to 3.0 ppm, a 15H peak attributed to a methoxy group at around δ3.0 to 5.0 ppm, and 22H peaks attributed to aromatic protons and protons of an alkene at around δ5.0 to 9.0 ppm.

Furthermore, in a ¹³C-nuclear magnetic resonance spectroscopy, the photochromic compound represented by formula (66) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 13)

### First Step

The same reaction as in the fourth step of Example 1 was performed except that 4-bromo-4'-phenoxybenzophenone was used instead of 4-bromo-4'-methoxylbenzophenone, to obtain a propargyl alcohol represented by formula (67) below with a yield of 86%.

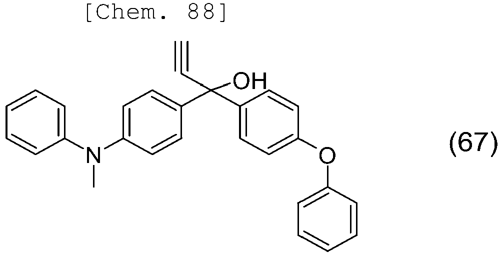

### Second Step

The same reaction as in the second step of Example 6 was performed except that 4-bromo-3',4'-dimethoxybenzophenone was used instead of 3,4-dimethoxybenzophenone, 1,1,1-trifluoro-4-iodobutane was used instead of 1-bromo-4-fluorobutane, and the propargyl alcohol represented by formula (67) above was used instead of the propargyl alcohol represented by formula (52) above, to obtain a photochromic compound represented by formula (68) below with a yield of 82%.

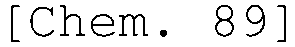

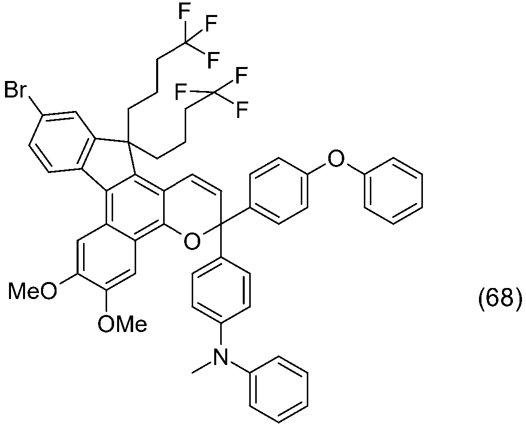

### Third Step

The same reaction as in the third step of Example 1 was performed except that the photochromic compound represented by formula (68) above was used instead of the naphthol derivative represented by formula (32) above and 4-trifluoromethylphenylboronic acid was used instead of 4-morpholinophenylboronic acid, to obtain a photochromic compound represented by formula (69) below with a yield of 89%.

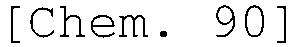

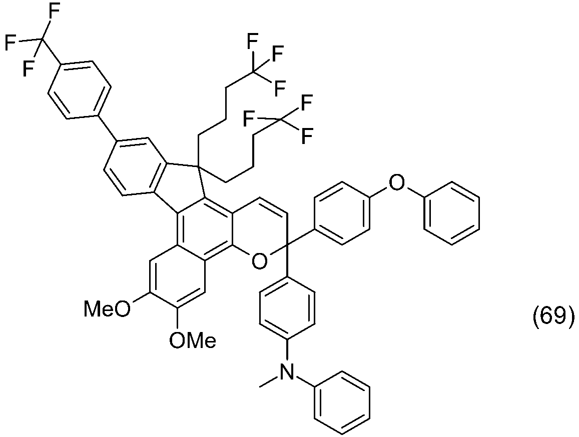

The photochromic compound represented by formula (69) above had elemental analysis values of C: 71.30%, H: 4.80%, and N: 1.32%, which were considerably consistent with the C₆₂H₅₀F₉NO₄ calculated values of C: 71.32%, H: 4.83%, and N: 1.34%.

In a proton nuclear magnetic resonance spectroscopy, the photochromic compound represented by formula (69) showed 15H peaks attributed to a methyl group and a 1,1,1-trifluorobutyl group at around δ0.5 to 3.0 ppm, a 6H peak attributed to a methoxy group at around δ3.0 to 5.0 ppm, and 29H peaks attributed to aromatic protons and protons of an alkene at around δ5.0 to 9.0 ppm.

Furthermore, in a ¹³C-nuclear magnetic resonance spectroscopy, the photochromic compound represented by formula (69) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 14)

### First Step

The same reaction as in the fourth step of Example 1 was performed except that 4-bromo-3',4'-ethylenedioxybenzophenone was used instead of 4-bromo-4'-methoxybenzophenone and N-(2-methoxyethyl)aniline was used instead of N-methylaniline, to obtain a propargyl alcohol represented by formula (70) below with a yield of 86%.

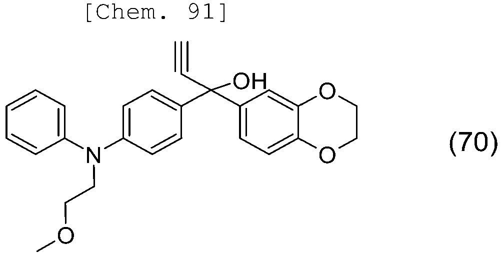

### Second Step

The same reaction as in the fifth step of Example 1 was performed except that a naphthol derivative represented by formula (71) below was used instead of the naphthol derivative represented by formula (33) above and the propargyl alcohol represented by formula (70) above was used instead of the propargyl alcohol represented by formula (35) above, to obtain a photochromic compound represented by formula (72) below with a yield of 84%.

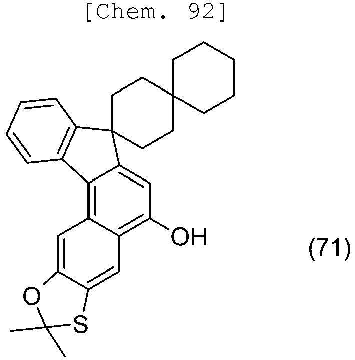

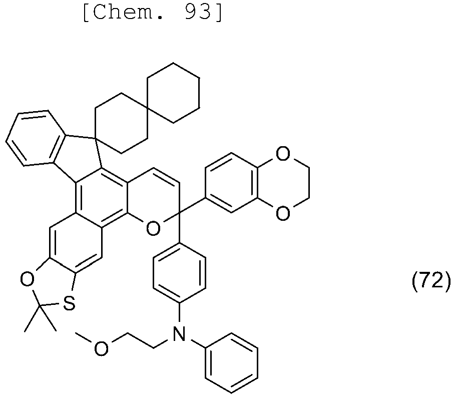

The photochromic compound represented by formula (72) above had elemental analysis values of C: 78.77%, H: 6.48%, N: 1.63%, and S: 3.76, which were considerably consistent with the C₅₆H₅₅NO₅S calculated values of C: 78.75%, H: 6.49%, N: 1.64%, and S: 3.75%.

In a proton nuclear magnetic resonance spectroscopy, the photochromic compound represented by formula (72) showed 26H peaks attributed to a methyl group, a 2-methoxyethyl group, and a spiro[5,5]undecane ring group at around 50.5 to 3.0 ppm, 9H peaks attributed to a 2-methoxyethyl group and an ethylenedioxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (72) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 15)

### First Step

The same reaction as in the fifth step of Example 2 was performed except that N-methylaniline was used instead of 4-(methylamino)toluene, to obtain a propargyl alcohol represented by formula (73) below with a yield of 90%.

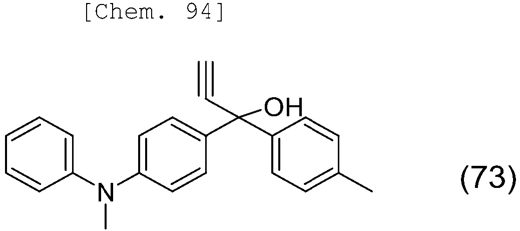

### Second Step

The same reaction as in the second step of Example 6 was performed except that 4-bromo-3'-methyl-4'-methoxybenzophenone was used instead of 3,4-dimethoxybenzophenone, iodobutane was used instead of 1-bromo-4-fluorobutane, and the propargyl alcohol represented by formula (73) above was used instead of the propargyl alcohol represented by formula (52) above, to obtain a photochromic compound represented by formula (74) below with a yield of 76%.

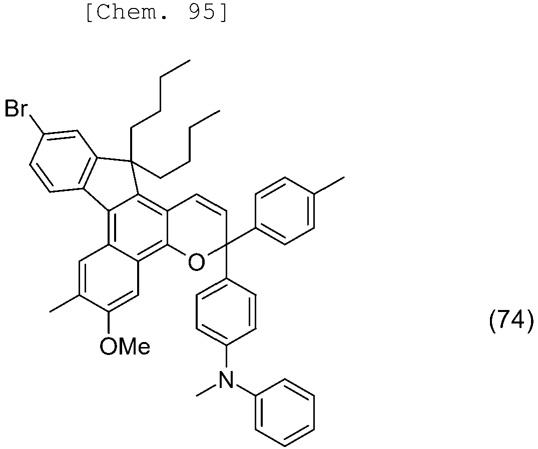

### Third Step

To 35 mL of THF, 3.34 g (4.3 mmol) of the compound represented by formula (74) above was dissolved, and cooled the internal temperature to -78°C. Into the mixture, 3.3 mL of n-BuLi (1.6 mol/L hexane solution) was slowly dripped while maintaining the temperature at -70°C or lower. After confirming that the raw materials had been consumed, 1.5 g (6.5 mmol) of diphenylmethylchlorosilane dissolved in 10 mL of THF was slowly dripped into the mixture while maintaining the temperature at -70°C or lower. After the dripping, the mixture was slowly heated to room temperature. The mixture was stirred at room temperature for 2 hours, then 50 mL of water and 50 mL of toluene were added to the mixture, which was subjected to liquid separation. The mixture was repeatedly washed with water until a pH of a water layer reached 7 to 8. From the obtained organic layer, the solvent was removed, and the organic layer was purified by a chromatography on silica gel to obtain a photochromic compound represented by formula (75) below with a yield of 48%.

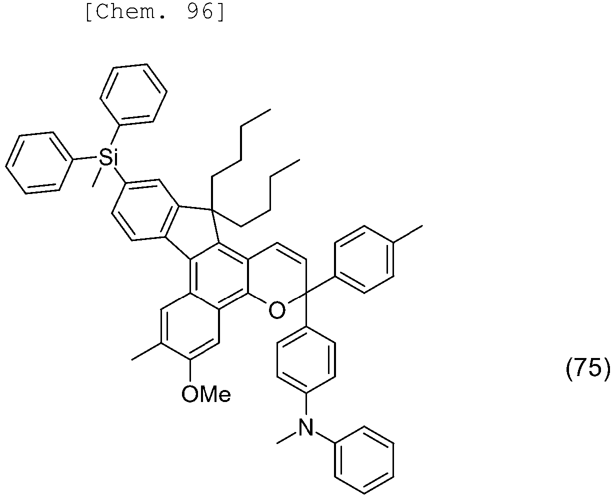

The photochromic compound represented by formula (75) above had elemental analysis values of C: 84.59%, H: 7.11%, and N: 1.55%, which were considerably consistent with the C₆₃H₆₃NO₂Si calculated values of C: 84.61%, H: 7.10%, and N: 1.57%.

In a proton nuclear magnetic resonance spectroscopy, the photochromic compound represented by formula (75) showed 30H peaks attributed to a methyl group and a butyl group at around δ0.5 to 3.0 ppm, a 3H peak attributed to a methoxy group at around δ3.0 to 5.0 ppm, and 30H peaks attributed to aromatic protons and protons of an alkene at around 65.0 to 9.0 ppm.

Furthermore, in a ¹³C-nuclear magnetic resonance spectroscopy, the photochromic compound represented by formula (75) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 16)

### First Step

The same reaction as in the fourth step of Example 1 was performed except that 4-bromo-3'-methyl-4'-methoxybenzophenone was used instead of 4-bromo-4'-methoxybenzophenone, to obtain a propargyl alcohol represented by formula (76) below with a yield of 88%.

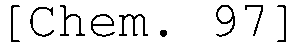

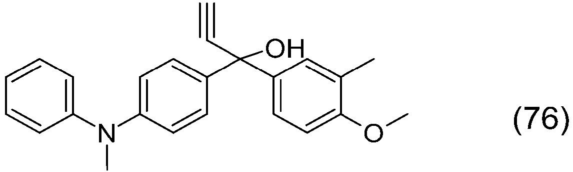

### Second Step

The same reaction as in the first step of Example 2 was performed except that (4-methoxyphenyl) [(4-trifluoromethoxy)phenyl]methanone was used instead of 4-bromo-4'-methoxybenzophenone, and the same reaction as in the fifth step of Example 2 was performed except that the propargyl alcohol represented by formula (76) above was used instead of the propargyl alcohol represented by formula (44) above, to obtain a photochromic compound represented by formula (77) below with a yield of 78%.

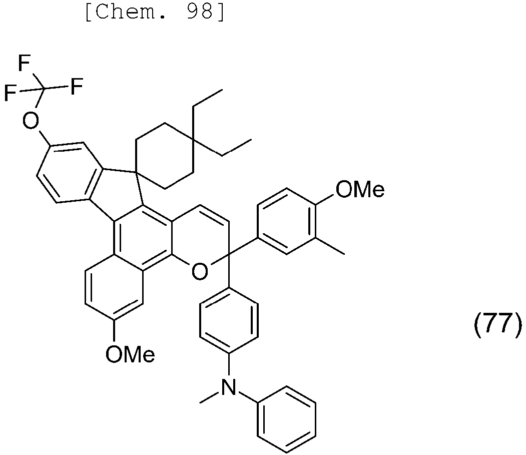

The photochromic compound represented by formula (77) above had elemental analysis values of C: 77.08%, H: 6.21%, and N: 1.71%, which were considerably consistent with the C₅₂H₅₀F₃NO₄ calculated values of C: 77.11%, H: 6.22%, and N: 1.73%.

In a proton nuclear magnetic resonance spectroscopy, the photochromic compound represented by formula (77) showed 24H peaks attributed to a methyl group and a 4,4-diethylcyclohexyl group at around δ0.5 to 3.0 ppm, a 6H peak attributed to a methoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (77) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 17)

### First Step

The same reaction as in the first step of Example 1 was performed except that 3-bromo-4-methyl-4'-methylbenzophenone was used instead of 3-bromo-4-methoxybenzophenone, the same reaction as in the third step of Example 1 was performed except that 4-methoxyphenylboronic acid was used instead of 4-morpholinophenylboronic acid, the same reaction as in the fifth step of Example 1 was performed except that the propargyl alcohol represented by formula (58) above was used instead of the propargyl alcohol represented by formula (35) above, and the same reaction as in the sixth step of Example 1 was performed except that iodoethane was used instead of iodomethane, to obtain a photochromic compound represented by formula (78) below with a yield of 70%.

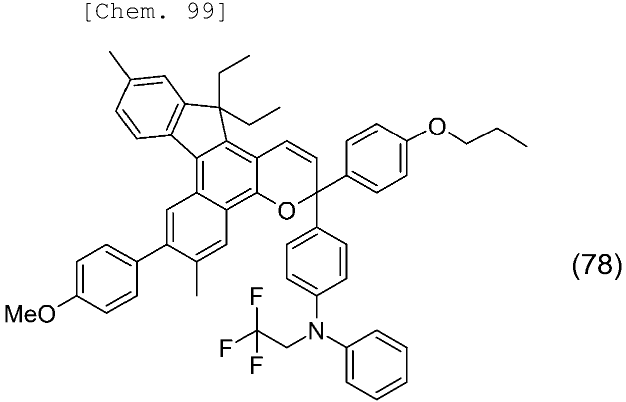

The photochromic compound represented by formula (78) above had elemental analysis values of C: 79.66%, H: 6.20%, and N: 1.66%, which were considerably consistent with the C₅₆H₅₂F₃NO₃ calculated values of C: 79.69%, H: 6.21%, and N: 1.66%.

In a proton nuclear magnetic resonance spectroscopy, the photochromic compound represented by formula (78) showed 23H peaks attributed to a methyl group, an ethyl group, a 1,1,1-trifluoroethyl group, and a propyloxy group at around δ0.5 to 3.0 ppm, 5H peaks attributed to a methoxy group and a propyloxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (78) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 18)

### First Step

The same reaction as in the first step of Example 1 was performed except that 3-bromo-4-methybenzophenone was used instead of 3-bromo-4-methoxybenzophenone, the same reaction as in the third step of Example 1 was performed except that 4-methylphenylboronic acid was used instead of 4-morpholinophenylboronic acid, the same reaction as in the fifth step of Example 1 was performed except that the propargyl alcohol represented by formula (49) above was used instead of the propargyl alcohol represented by formula (35) above, and the same reaction as in the sixth step of Example 1 was performed except that 1-iodo-2-methoxyethane was used instead of iodomethane, to obtain a photochromic compound represented by formula (79) below with a yield of 76%.

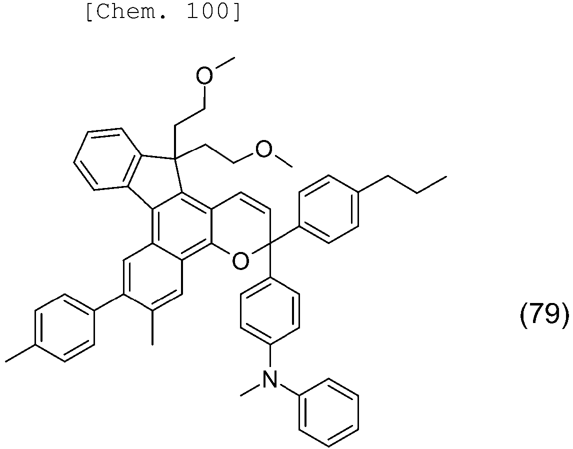

The photochromic compound represented by formula (79) above had elemental analysis values of C: 85.12%, H: 7.04%, and N: 1.77%, which were considerably consistent with the C₅₆H₅₅NO₃ calculated values of C: 85.13%, H: 7.02%, and N: 1.77%.

In a proton nuclear magnetic resonance spectroscopy, the photochromic compound represented by formula (79) showed 20H peaks attributed to a methyl group, a propyl group, and a 2-methoxyethyl group at around δ0.5 to 3.0 ppm, a 10H peak attributed to a 2-methoxyethyl group at around δ3.0 to 5.0 ppm, and 25H peaks attributed to aromatic protons and protons of an alkene at around δ5.0 to 9.0 ppm.

Furthermore, in a ¹³C-nuclear magnetic resonance spectroscopy, the photochromic compound represented by formula (79) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 19)

The same reaction as in the third step of Example 1 was performed except that 2,4-dimethoxyphenylboronic acid was used instead of 4-morpholinophenylboronic acid, the same reaction as in the fifth step of Example 1 was performed except that the propargyl alcohol represented by formula (73) above was used instead of the propargyl alcohol represented by formula (35) above, and the same reaction as in the sixth step of Example 1 was performed except that iodopropane was used instead of iodomethane, to obtain a photochromic compound represented by formula (80) below with a yield of 72%.

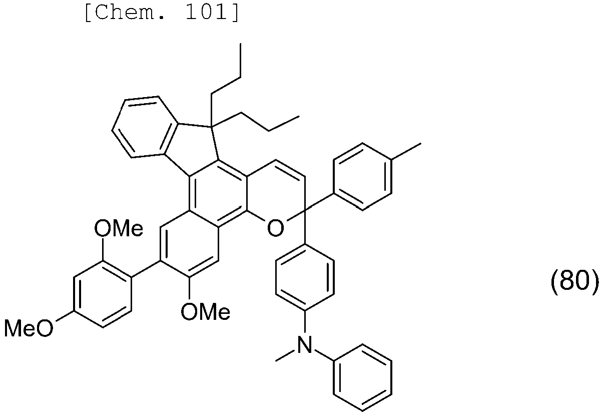

The photochromic compound represented by formula (80) above had elemental analysis values of C: 83.39%, H: 6.75%, and N: 1.78%, which were considerably consistent with the C₅₅H₅₃NO₄ calculated values of C: 83.41%, H: 6.74%, and N: 1.77%.

In a proton nuclear magnetic resonance spectroscopy, the photochromic compound represented by formula (80) showed 20H peaks attributed to a methyl group and a propyl group at around δ0.5 to 3.0 ppm, a 9H peak attributed to a methoxy group at around 63.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (80) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 20)

The same reaction as in the second step of Example 13 was performed except that 1-iodo-3-methoxypropane was used instead of 1,1,1-trifluoro-4-iodobutane and the propargyl alcohol represented by formula (70) above was used instead of the propargyl alcohol represented by formula (67) above, and the same reaction as in the third step of Example 13 was performed except that 4-t-butylphenylboronic acid was used instead of 4-trifluoromethylphenylboronic acid, to obtain a photochromic compound represented by formula (81) below with a yield of 75%.

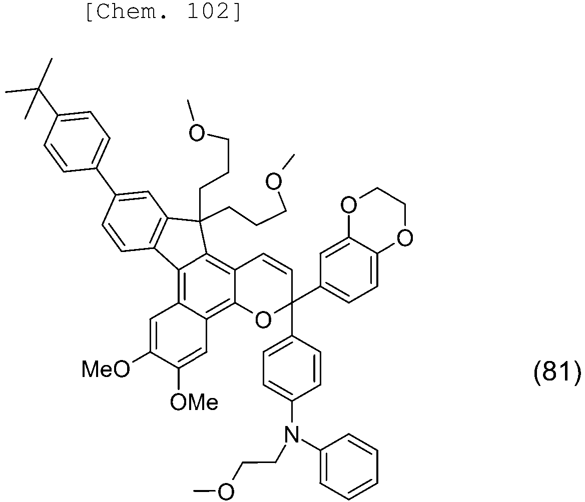

The photochromic compound represented by formula (81) above had elemental analysis values of C: 78.32%, H: 7.00%, and N: 1.44%, which were considerably consistent with the C₆₃H₆₇NO₈ calculated values of C: 78.31%, H: 6.99%, and N: 1.45%.

In a proton nuclear magnetic resonance spectroscopy, the photochromic compound represented by formula (81) showed 19H peaks attributed to a t-butyl group, a 3-methoxypropyl group, and a 2-methoxyethyl group at around δ0.5 to 3.0 ppm, 25H peaks attributed to a methoxy group, a 3-methoxypropyl group, a 2-methoxyethyl group, and an ethylenedioxy group at around δ3.0 to 5.0 ppm, and 23H peaks attributed to aromatic protons and protons of an alkene at around 65.0 to 9.0 ppm.

Furthermore, in a ¹³C-nuclear magnetic resonance spectroscopy, the photochromic compound represented by formula (81) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 21)

### First Step

The same reaction as in the second step of Example 11 was performed except that the propargyl alcohol represented by formula (54) above was used instead of the propargyl alcohol represented by formula (63) above and iodobutane was used instead of 1-bromo-4-methoxybutane, to synthesize a photochromic compound represented by formula (82) below.

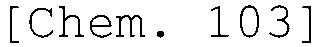

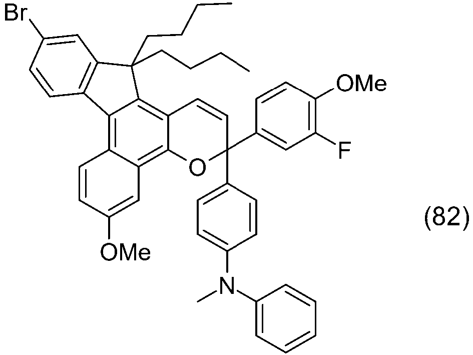

### Second Step

The same reaction as in the third step of Example 1 was performed except that the photochromic compound represented by formula (82) above was used instead of the naphthol derivative represented by formula (32) above and 4-isopropoxyphenylboronic acid was used instead of 4-morpholinophenylboronic acid, to obtain a photochromic compound represented by formula (83) below with a yield of 89%.

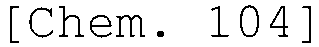

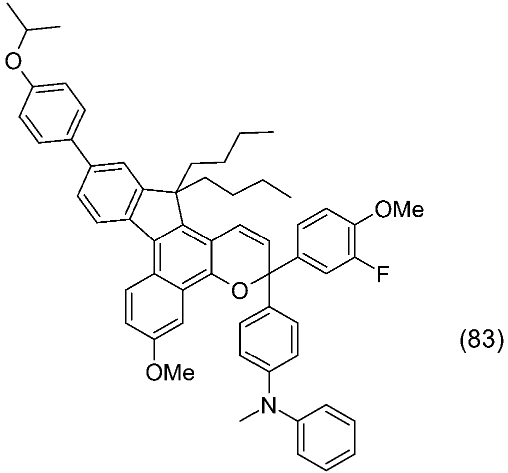

The photochromic compound represented by formula (83) above had elemental analysis values of C: 81.77%, H: 6.85%, and N: 1.64%, which were considerably consistent with the C₅₈H₅₈FNO₄ calculated values of C: 81.75%, H: 6.86%, and N: 1.64%.

In a proton nuclear magnetic resonance spectroscopy, the photochromic compound represented by formula (83) showed 27H peaks attributed to a butyl group, a methyl group, and an isopropoxy group at around δ0.5 to 3.0 ppm, 7H peaks attributed to a methoxy group and an isopropoxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (83) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 22)

### First Step

The same reaction as in the third step of Example 2 was performed except that 4-heptanone was used instead of 4,4-diethylcyclohexanone, and the same reaction as in the sixth step of Example 2 was performed except that the propargyl alcohol represented by formula (73) above was used instead of the propargyl alcohol represented by formula (44) above, to obtain a photochromic compound represented by formula (84) below with a yield of 86%.

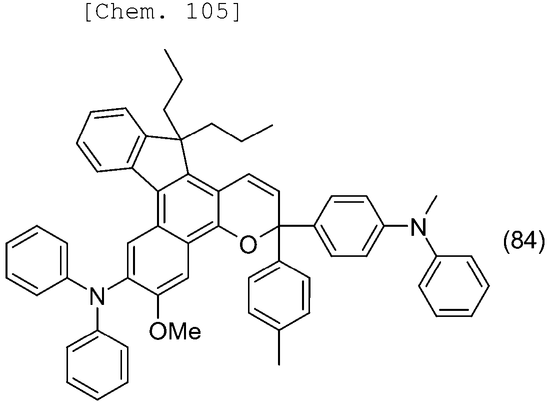

The photochromic compound represented by formula (84) above had elemental analysis values of C: 86.08%, H: 6.61%, and N: 3.41%, which were considerably consistent with the C₅₉H₅₄N₂O₂ calculated values of C: 86.10%, H: 6.61%, and N: 3.40%.

In a proton nuclear magnetic resonance spectroscopy, the photochromic compound represented by formula (84) showed 20H peaks attributed to a methyl group and a propyl group at around δ0.5 to 3.0 ppm, a 3H peak attributed to a methoxy group at around 63.0 to 5.0 ppm, and 31H peaks attributed to aromatic protons and protons of an alkene at around δ5.0 to 9.0 ppm.

Furthermore, in a ¹³C-nuclear magnetic resonance spectroscopy, the photochromic compound represented by formula (84) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 23)

The same reaction as in the third step of Example 1 was performed except that 4-diphenylaminophenylboronic acid was used instead of 4-morpholinophenylboronic acid, the same reaction as in the fifth step of Example 1 was performed except that the propargyl alcohol represented by formula (67) above was used instead of the propargyl alcohol represented by formula (35) above, and the same reaction as in the sixth step of Example 1 was performed except that 1-iodo-2-methoxyethane was used instead of iodomethane, to obtain a photochromic compound represented by formula (85) below with a yield of 71%.

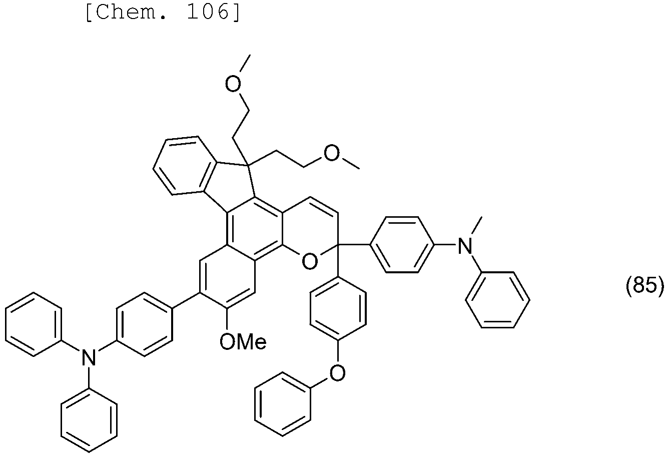

The photochromic compound represented by formula (85) above had elemental analysis values of C: 83.33%, H: 5.98%, and N: 2.76%, which were considerably consistent with the C₇₀H₆₀N₂O₅ calculated values of C: 83.31%, H: 5.99%, and N: 2.78%.

In a proton nuclear magnetic resonance spectroscopy, the photochromic compound represented by formula (85) showed 7H peaks attributed to a methyl group and a 2-methoxyethyl group at around δ0.5 to 3.0 ppm, 13H peaks attributed to a methoxy group and a 2-methoxyethyl group at around δ3.0 to 5.0 ppm, and 40H peaks attributed to aromatic protons and protons of an alkene at around δ5.0 to 9.0 ppm.

Furthermore, in a ¹³C-nuclear magnetic resonance spectroscopy, the photochromic compound represented by formula (85) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 24)

The same reaction as in the third step of Example 1 was performed except that 4-methylphenylaminophenylboronic acid was used instead of 4-morpholinophenylboronic acid, the same reaction as in the fifth step of Example 1 was performed except that the propargyl alcohol represented by formula (73) above was used instead of the propargyl alcohol represented by formula (35) above, and the same reaction as in the sixth step of Example 1 was performed except that iodopropane was used instead of iodomethane, to obtain a photochromic compound represented by formula (86) below with a yield of 77%.

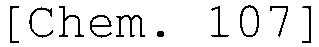

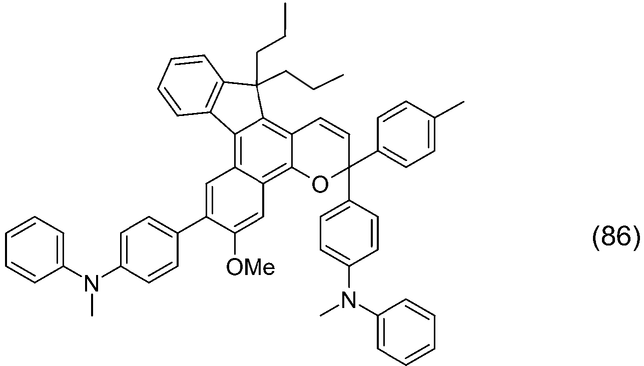

The photochromic compound represented by formula (86) above had elemental analysis values of C: 86.09%, H: 6.75%, and N: 3.33%, which were considerably consistent with the C₆₀H₅₆N₂O₂ calculated values of C: 89.09%, H: 6.74%, and N: 3.35%.

In a proton nuclear magnetic resonance spectroscopy, the photochromic compound represented by formula (86) showed 23H peaks attributed to a methyl group and a propyl group at around δ0.5 to 3.0 ppm, a 3H peak attributed to a methoxy group at around δ3.0 to 5.0 ppm, and 30H peaks attributed to aromatic protons and protons of an alkene at around δ5.0 to 9.0 ppm.

Furthermore, in a ¹³C-nuclear magnetic resonance spectroscopy, the photochromic compound represented by formula (86) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 25)

### First Step

The same reaction as in the fourth step of Example 1 was performed except that N-ethyl-4-methoxyaniline was used instead of N-methylaniline, to obtain a propargyl alcohol represented by formula (87) below with a yield of 89%.

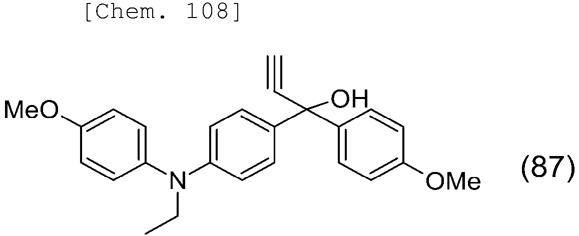

### Second Step

The same reaction as in the fifth step of Example 1 was performed except that the propargyl alcohol represented by formula (87) above was used instead of the propargyl alcohol represented by formula (35) above, to obtain a photochromic compound represented by formula (88) below with a yield of 81%.

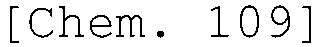

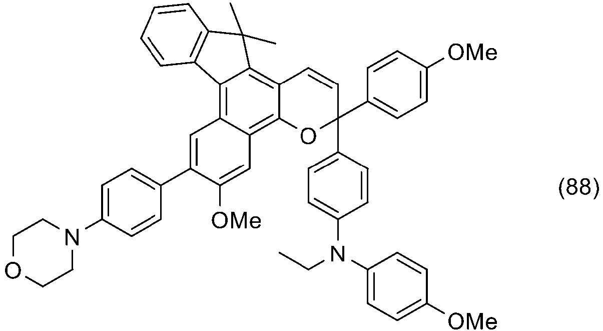

The photochromic compound represented by formula (88) above had elemental analysis values of C: 80.44%, H: 6.38%, and N: 3.42%, which were considerably consistent with the C₅₅H₅₂N₂O₅ calculated values of C: 80.46%, H: 6.38%, and N: 3.41%.

In a proton nuclear magnetic resonance spectroscopy, the photochromic compound represented by formula (88) showed 15H peaks attributed to a methyl group, an ethyl group, and a morpholino group at around 50.5 to 3.0 ppm, 13H peaks attributed to a methoxy group and a morpholino group at around δ3.0 to 5.0 ppm, and 24H peaks attributed to aromatic protons and protons of an alkene at around 65.0 to 9.0 ppm.

Furthermore, in a ¹³C-nuclear magnetic resonance spectroscopy, the photochromic compound represented by formula (88) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Physical Property Evaluation for Photochromic Plastic Lens Prepared by Coating Method)

### (Example 26)

### (Preparation of Curable Composition)

First, the photochromic compound obtained in Example 1, a photoinitiator, and a polymerizable compound were mixed to obtain a curable composition.

As the polymerizable compound, a combination of the following radical polymerizable monomers was used.

Polyethylene glycol dimethacrylate (average molecular weight: 736): 42 parts by mass
Polyethylene glycol dimethacrylate (average molecular weight: 536): 12 parts by mass
Trimethylolpropane trimethacrylate: 38 parts by mass γ-methacryloyloxypropyl trimethoxysilane: 2 parts by mass Glycidyl methacrylate: 1 part by mass

Note that the photochromic compound was added to the curable composition so that the amount of the photochromic compound was 0.25 mmol based on 100 g of the total amount of the radical-polymerizable monomers.

The following additives were used.

Phenylbis(2,4,6-trimethylbenzoyl)phosphine oxide (photoinitiator: Omnirad 819): 0.3 parts by mass Ethylene bis(oxyethylene) bis[3-(5-tert-butyl-4-hydroxy-m-tolyl)propionate] (stabilizer, Irganox 245): 1 part by mass Bis(1,2,2,6,6-pentamethyl-4-piperidyl) sebacate: 3 parts by mass
Leveling agent (L7001) manufactured by Dow Corning Toray Co.,Ltd.: 0.1 part by mass

Note that each compounding ratio of the above additives is based on 100 parts by mass of the total amount of the radical-polymerizable monomers.

### (Production of Optical Article)

This curable composition was subjected to a lamination method using a polymerization to obtain a photochromic laminate, as described below.

First, a thiourethane-based plastic lens having a central thickness of 2 mm and a refractive index of 1.60 was prepared as the optical substrate. Note that this thiourethane-based plastic lens was pre-treated by alkaline etching with a 10% sodium hydroxide aqueous solution at 50°C for 5 minutes, followed by sufficient washing with distilled water.

A surface of the above-described plastic lens was coated with a moisture-curable primer (product name: TR-SC-P, manufactured by Tokuyama Corporation) at a rotation frequency of 70 rpm for 15 seconds, followed by 1000 rpm for 10 seconds, using a spin coater (1H-DX2, manufactured by MIKASA CO., LTD), Subsequently, about 2 g of the photochromic curable composition obtained above was spin-coated at a rotation frequency of 60 rpm for 40 seconds, followed by 600 rpm for 10 to 20 seconds, to form a photochromic coating layer with a film thickness of 40 µm.

The lens with the surface coated with the photochromic curable composition (photochromic coating layer) was irradiated with light using a metal halide lamp with an output power of 200 mW/cm² in a nitrogen gas atmosphere for 90 seconds to cure the coating film. Subsequently, the lens was further heated at 110°C for 1 hour to prepare a photochromic laminate having a photochromic layer.

### (Examples 27 to 50)

In the same manner as in Example 26, photochromic laminates were prepared using the photochromic compounds obtained in Examples 2 to 25.

### (Comparative Examples 1 to 8)

In the same manner as in Example 26, each photochromic laminate was obtained using each of the photochromic compounds represented by formulas (A) to (H) below.

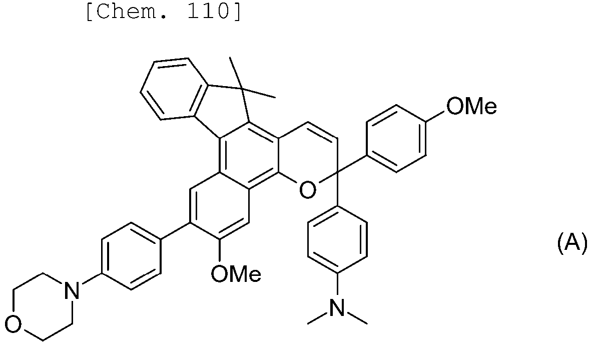

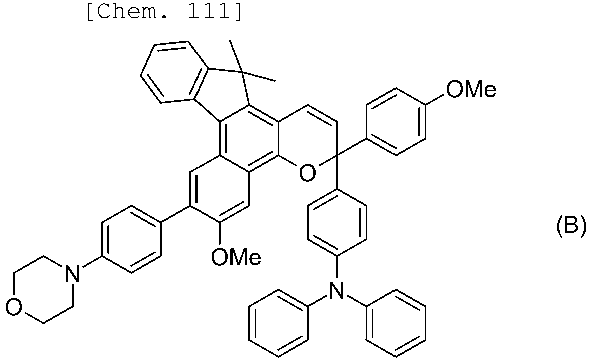

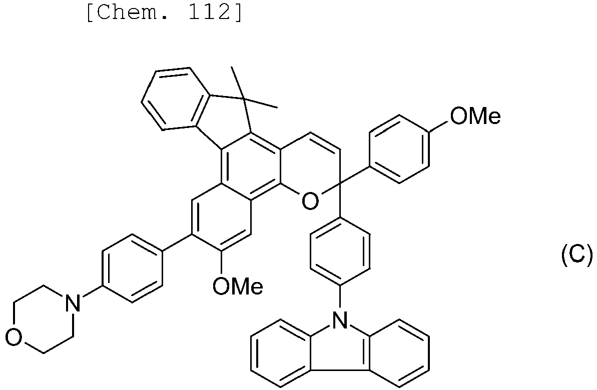

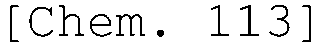

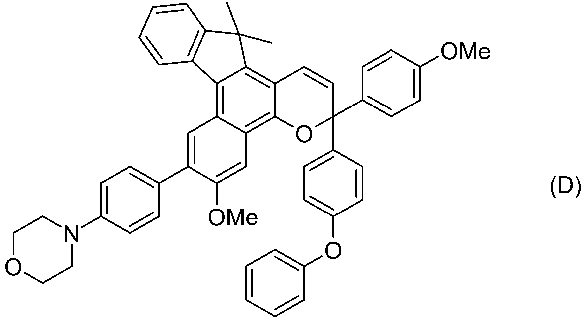

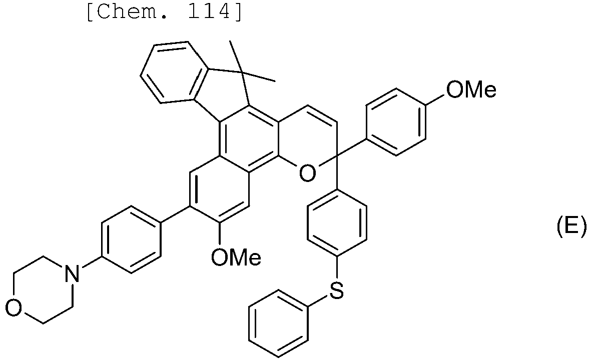

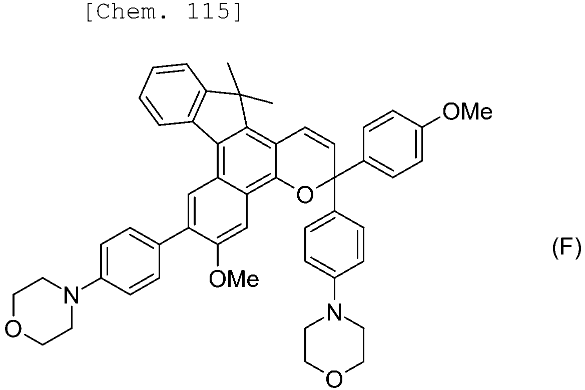

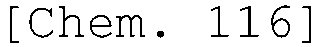

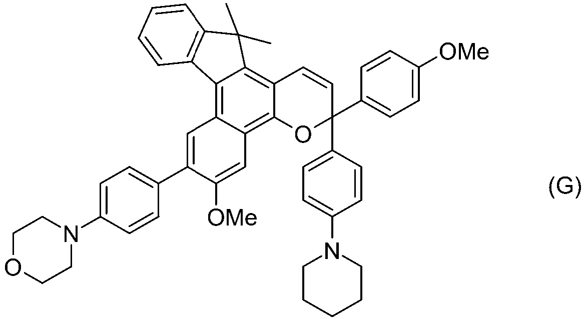

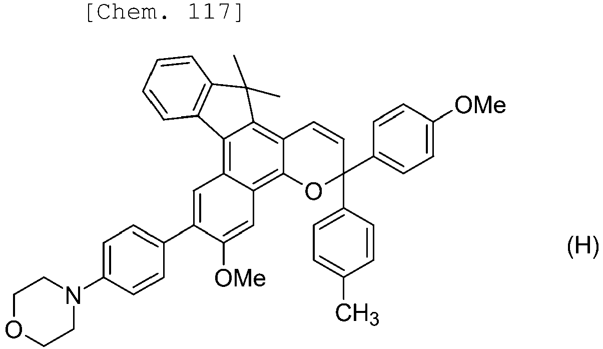

### (Synthesis of Compound A)

The same reaction as in Example 1 was performed except that 4-dimethylamino-4'-methoxybenzophenone was used instead of benzophenone represented by formula (34) above.

### (Synthesis of Compound B)

The same reaction as in Example 1 was performed except that 4-diphenylamino-4'-methoxybenzophenone was used instead of benzophenone represented by formula (34) above.

### (Synthesis of Compound C)

The same reaction as in Example 1 was performed except that [4-(9H-carbazol-9-yl)phenyl]-(4-methoxyphenyl)methanone was used instead of benzophenone represented by formula (34) above.

### (Synthesis of Compound D)

The same reaction as in Example 1 was performed except that 4-phenoxy-4'-methoxybenzophenone was used instead of benzophenone represented by formula (34) above.

### (Synthesis of Compound E)

The same reaction as in Example 1 was performed except that (4-methoxyphenyl)-(4-phenylsulfanylphenyl)methanone was used instead of benzophenone represented by formula (34) above.

### (Synthesis of Compound F)

The same reaction as in Example 1 was performed except that 4-methoxyphenyl)-[(4-morpholin-1-yl)phenyl]methanone was used instead of benzophenone represented by formula (34) above.

### (Synthesis of Compound G)

The same reaction as in Example 1 was performed except that (4-methoxyphenyl)-[(4-piperidine-1-yl)phenyl]methanone was used instead of benzophenone represented by formula (34) above.

### (Synthesis of Compound H)

The same reaction as in Example 1 was performed except that 4-methoxy-4'-methylbenzophenone was used instead of benzophenone represented by formula (34) above.

### <Evaluation Method>

The obtained photochromic laminates were evaluated by a method described below.

### (1) Photochromic Properties

[1] Maximum Absorption Wavelength (λmax):
   The maximum absorption wavelength after color development, measured using a spectrophotometer (instantaneous multichannel photodetector MCPD3000) manufactured by Otsuka Electronics Co., Ltd. was used as an indicator for the color tone during the color development.
[2] 23°C Color Optical Density (A₂₃):
   A difference between an absorbancy measured after light irradiation at 23°C for 300 seconds {ε(300)} and an absorbancy without light irradiation ε(0) at the maximum absorption wavelength was used as an indicator for the color optical density. It is considered that, the higher this value, the superior the photochromic property.
[3] 23°C color fading half-life [τ1/2 (sec.)]:
   A time required for the absorbancy of the sample at the maximum absorption wavelength to decrease to 1/2 of {ε(300)-ε(0)} when the light irradiation was terminated after the light irradiation at 23°C for 300 seconds, was used as an indicator for the color fading speed. The shorter this time, the higher the color fading speed.
[4] Residual rate (A₁₂₀/A₀×100): The obtained photochromic plastic lens was subjected to accelerated deterioration using a xenon weather meter X25 manufactured by Suga Test Instruments Co., Ltd. for 120 hours. Subsequently, the color optical density was evaluated before and after the test, then a color optical density before the test (A₀) and a color optical density after the test (A₁₂₀) were measured, and a ratio (A₁₂₀/A₀) was defined as a residual rate. The residual rate was used as an indicator for a color developing durability. The higher the residual rate, the higher the color developing durability. The results of Examples 26, 50, and Comparative Examples 1 to 8 are summarized in Table 1, and the results of Examples 27 to 49 are summarized in Tables 2 and 3.

### [Table 1]

**[table 1]**

| | Compound No. | Maximum absorption wavelength (nm) | A23 (-) | 23°C color fading half-life (sec) | Residual rate (%) |
|---|---|---|---|---|---|
| Example 26 | Example 1 | 502 | 1.09 | 76 | 65% |
| | Formula (37) | 601 | 1.09 | 77 | 65% |
| Example 50 | Example 25 | 505 | 0.84 | 68 | 63% |
| | Formula (88) | 605 | 0.94 | 68 | 63% |
| Comparative Example 1 | Formula (A) | 503 | 0.89 | 57 | 43% |
| | | 600 | 0.87 | 57 | 43% |
| Comparative Example 2 | Formula (B) | 504 | 1.29 | 189 | 56% |
| | | 587 | 1.23 | 190 | 56% |
| Comparative Example 3 | Formula (C) | 475 | 1. 61 | 393 | 33% |
| Comparative Example 4 | Formula (D) | 468 | 1.36 | 174 | 59% |
| | | 558 | 0.97 | 178 | 59% |
| Comparative Example 5 | Formula (E) | 479 | 1.28 | 187 | 55% |
| | | 560 | 0.92 | 188 | 54% |
| Comparative Example 6 | Formula (F) | 490 | 1.10 | 89 | 54% |
| | | 586 | 1.01 | 90 | 54% |
| Comparative Example 7 | Formula (G) | 500 | 1.02 | 74 | 48% |
| | | 595 | 1.05 | 75 | 48% |
| Comparative Example 8 | Formula (H) | 471 | 1.34 | 202 | 57% |
| | | 554 | 0.94 | 204 | 57% |

### [Table 2]

**[table 2]**

| | Compound No. | Maximum absorption wavelength (nm) | A23 (-) | 23°C color fading half-life (sec) | Residual rate (%) |
|---|---|---|---|---|---|
| Example 27 | Example 2 | 522 | 1.36 | 73 | 67% |
| | Formula (45) | | | | |
| Example 28 | Example 3 | 470 | 0.57 | 71 | 55% |
| | Formula (46) | 612 | 1.14 | 71 | 55% |
| Example 29 | Example 4 | 489 | 0.59 | 90 | 57% |
| | Formula (48) | 601 | 1.05 | 88 | 57% |
| Example 30 | Example 5 | 509 | 0.54 | 48 | 68% |
| | Formula (51) | 591 | 0.66 | 47 | 68% |
| Example 31 | Example 6 | 481 | 0.69 | 59 | 70% |
| | Formula (53) | 593 | 0.72 | 60 | 63% |
| Example 32 | Example 7 | 496 | 0.72 | 70 | 61% |
| | Formula (55) | 603 | 0.96 | 70 | 61% |
| Example 33 | Example 8 | 474 | 0.49 | 74 | 60% |
| | Formula (57) | 597 | 1.19 | 74 | 60% |
| Example 34 | Example 9 | 578 | 0.94 | 36 | 62% |
| | Formula (60) | | | | |
| Example 35 | Example 10 | 512 | 1.35 | 117 | 60% |
| | Formula (62) | 598 | 1.08 | 115 | 64% |
| Example 36 | Example 11 | 477 | 0.48 | 38 | 60% |
| | Formula (65) | 614 | 0.95 | 38 | 60% |
| Example 37 | Example 12 | 478 | 0.80 | 78 | 65% |
| | Formula (66) | 614 | 1.07 | 78 | 65% |
| Example 38 | Example 13 | 489 | 0.86 | 62 | 65% |
| | Formula (69) | 605 | 0.94 | 63 | 60% |

### [Table 3]

**[table 3]**

| | Compound No. | Maximum absorption wavelength (nm) | A23 (-) | 23°C color fading half-life (sec) | Residual rate (%) |
|---|---|---|---|---|---|
| Example 39 | Example 14 | 492 | 056 | 52 | 63% |
| | Formula (72) | 586 | 0.64 | 52 | 63% |
| Example 40 | Example 15 | 487 | 0.89 | 113 | 58% |
| | Formula (75) | 602 | 1.25 | 112 | 58% |
| Example 41 | Example 16 | 472 | 0.44 | 48 | 57% |
| | Formula (77) | 584 | 0.85 | 48 | 57% |
| Example 42 | Example 17 | 484 | 0.54 | 48 | 56% |
| | Formula (78) | 592 | 1.05 | 49 | 56% |
| Example 43 | Example 18 | 587 | 1.12 | 48 | 60% |
| | Formula (79) | | | | 60% |
| Example 44 | Example 19 | 494 | 0.84 | 93 | 64% |
| | Formula (80) | 604 | 1.12 | 93 | 64% |
| Example 45 | Example 20 | 486 | 0.75 | 77 | 67% |
| | Formula (81) | 608 | 0.85 | 76 | 63% |
| Example 46 | Example 21 | 489 | 0.57 | 72 | 60% |
| | Formula (83) | 609 | 1.21 | 71 | 60% |
| Example 47 | Example 22 | 530 | 1.51 | 75 | 68% |
| | Formula (84) | | | | |
| Example 48 | Example 23 | 506 | 0.99 | 48 | 60% |
| | Formula (85) | 609 | 1.04 | 48 | 60% |
| Example 49 | Example 24 | 504 | 1.27 | 89 | 62% |
| | Formula (86) | 599 | 1.27 | 90 | 62% |

### Example 51

### First Step

The same reaction as in the first step of Example 1 was performed except that (3,4-dimethoxyphenyl)-[4-(trifluoromethyl)phenyl]methanone was used instead of 3-bromo-4-methoxybenzophenone, and the same reaction as in the sixth step of Example 1 was performed except that 13-bromo-2,5,8,11-tetraoxatridecane was used instead of iodomethane, to obtain a photochromic compound represented by formula (89) below with a yield of 61%.

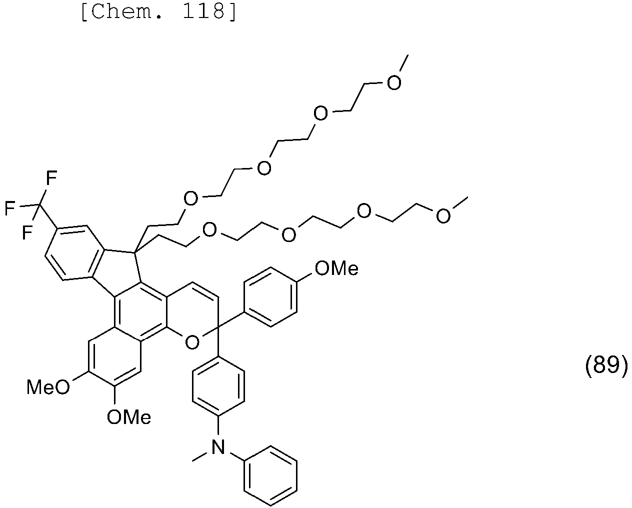

The photochromic compound represented by formula (89) above had elemental analysis values of C: 68.70%, H: 6.61%, and N: 1.31%, which were considerably consistent with the C₆₁H₇₀F₃NO₁₂ calculated values of C: 68.72%, H: 6.62%, and N: 1.31%. In a proton nuclear magnetic resonance spectroscopy, the photochromic compound represented by formula (89) showed 7H peaks attributed to a methyl group and a 2,5,8,11-tetraoxatridecane group at around δ0.5 to 3.0 ppm, 43H peaks attributed to a methoxy group and a 2,5,8,11-tetraoxatridecane group at around δ3.0 to 5.0 ppm, and 20H peaks attributed to aromatic protons and protons of an alkene at around δ5.0 to 9.0 ppm. Furthermore, in a ¹³C-nuclear magnetic resonance spectroscopy, the photochromic compound represented by formula (89) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 52

### First Step

By referring to the method described in Patent Document 8, the same reaction as in Example 2 was performed except that a benzophenone represented by formula (90) below obtained by reacting 4-bromobenzoyl chloride with 2,2-dimethyl-1,3-benzothiol was used, to synthesize a calboxylic acid compound represented by formula (91) below.

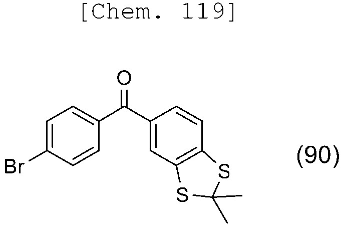

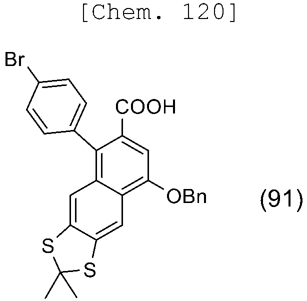

### Second Step

The same reaction as in the third step of Example 1 was performed except that the compound represented by formula (91) above was used instead of the compound represented by formula (32) above and (2,3,4-trifluorophenyl)boronic acid was used instead of 4-morpholinophenylboronic acid, to synthesize a calboxylic acid compound represented by formula (92) below with a yield of 91%.

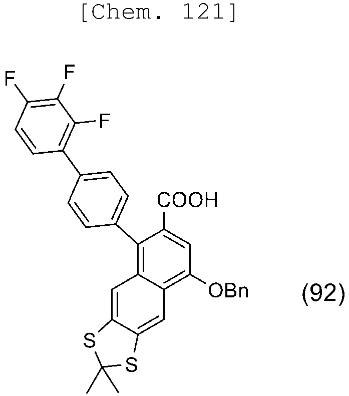

### Third Step

The same reaction as in the second step of Example 2 was performed except that the compound represented by formula (92) above was used instead of the compound represented by formula (39) above, the same reaction as in the third step of Example 2 was performed except that cyclododecanone was used instead of 4,4-diethylcyclohexanone, and the same reaction as in the fifth step of Example 2 was performed except that the propargyl alcohol represented by formula (35) above was used instead of the propargyl alcohol represented by formula (44) above, to obtain a photochromic compound represented by formula (93) below with a yield of 56%.

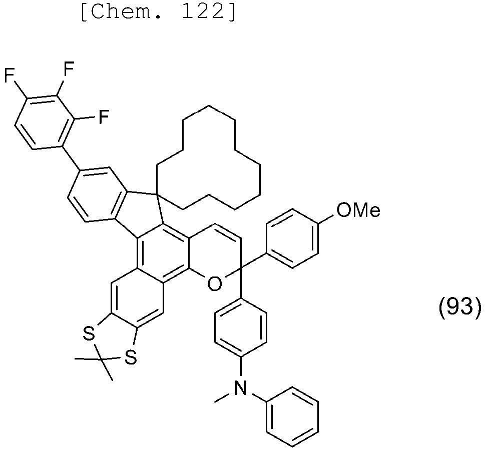

The photochromic compound represented by formula (93) above had elemental analysis values of C: 76.30%, H: 5.96%, N: 1.47%, and S: 6.80%, which were considerably consistent with the C₆₀H₅₆F₃NO₂S₂ calculated values of C: 76.32%, H: 5.98%, N: 1.48%, and S: 6.79%. In a proton nuclear magnetic resonance spectroscopy, the photochromic compound represented by formula (93) showed 31H peaks attributed to a methyl group and a cyclododecanone ring group at around δ0.5 to 3.0 ppm, a 3H peak attributed to a methoxy group at around δ3.0 to 5.0 ppm, and 22H peaks attributed to aromatic protons and protons of an alkene at around δ5.0 to 9.0 ppm. Furthermore, in a ¹³C-nuclear magnetic resonance spectroscopy, the photochromic compound represented by formula (93) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 53

### First Step

The same reaction as in the fifth step of Example 1 was performed except that the naphthol compound represented by formula (32) above was used instead of the naphthol compound represented by formula (33) above and the propargyl alcohol represented by formula (54) above was used instead of the propargyl alcohol represented by formula (35) above, and the same reaction as in the sixth step of Example 1 was performed except that 1-(2-iodoethoxy)butane was used instead of iodomethane, to obtain a photochromic compound represented by formula (94) below with a yield of 64%.

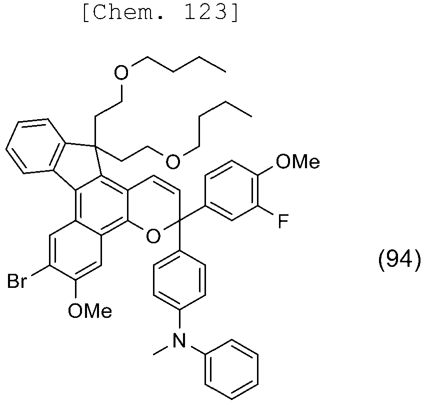

### Second Step

By referring to the method described in Patent Document 11, the photochromic compound represented by formula (94) above was reacted with benzamide. After the reaction, the reactant was purified by a chromatography on silica gel to obtain a photochromic compound represented by formula (95) below with a yield of 64%.

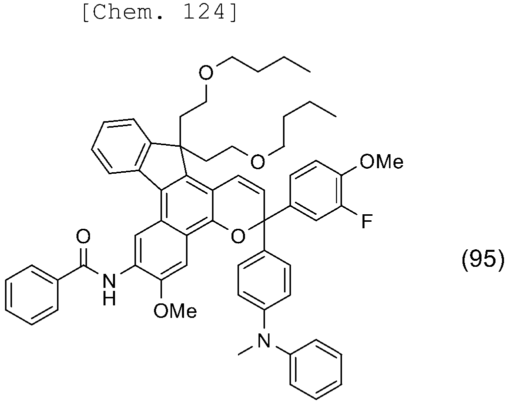

The photochromic compound represented by formula (95) above had elemental analysis values of C: 77.88%, H: 6.65%, and N: 3.02%, which were considerably consistent with the C₆₀H₆₁FN₂O₆ calculated values of C: 77.90%, H: 6.65%, and N: 3.03%. In a proton nuclear magnetic resonance spectroscopy, the photochromic compound represented by formula (95) showed 21H peaks attributed to a methyl group and an ethylbutoxy group at around δ0.5 to 3.0 ppm, 15H peaks attributed to a methoxy group, an ethylbutoxy group, and an amide group at around δ3.0 to 5.0 ppm, and 25H peaks attributed to aromatic protons and protons of an alkene at around δ5.0 to 9.0 ppm. Furthermore, in a ¹³C-nuclear magnetic resonance spectroscopy, the photochromic compound represented by formula (95) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 54

### First Step

The same reaction as in the first step of Example 12 was performed except that 3-bromo-4-methoxy-4'-methoxbenzophenone was used instead of 3-bromo-4-methoxy-4'-methylbenzophenone, 4-thiomorpholinophenylboronic acid was used instead of 2,4,6-trimethoxyphenylboronic acid, and 1,1,1-trifluoro-3-iodopropane was used instead of 1-bromo-3-methylthiopropane, to obtain a photochromic compound represented by formula (96) below with a yield of 55%.

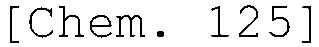

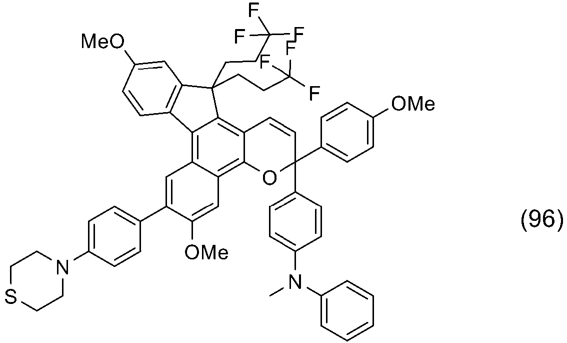

The photochromic compound represented by formula (96) above had elemental analysis values of C: 70.55%, H: 5.30%, N: 2.85%, and S: 3.24%, which were considerably consistent with the C₅₈H₅₂F6N₂O₄S calculated values of C: 70.57%, H: 5.31%, N: 2.84%, and S: 3.25%. In a proton nuclear magnetic resonance spectroscopy, the photochromic compound represented by formula (96) showed 19H peaks attributed to a methyl group, a 1,1,1-trifluoropropyl group, and a thiomorpholino group at around δ0.5 to 3.0 ppm, a 9H peak attributed to a methoxy group at around δ3.0 to 5.0 ppm, and 24H peaks attributed to aromatic protons and protons of an alkene at around δ5.0 to 9.0 ppm. Furthermore, in a ¹³C-nuclear magnetic resonance spectroscopy, the photochromic compound represented by formula (96) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 55

### First Step

To 200 mL of dimethylformamide, 27.7 g (100.1 mmol) of (4-chlorophenyl) [4-(2-hydroxyethoxy)phenyl]methanone and 15.0 g (220.2 mmol) of imidazole were dissolved, and cooled the internal temperature to 2°C. Into the mixture, 80 mL of dimethylformamide containing 16.6 g (110.1 mmol) of t-butyldimethylchlorosilane was dripped while maintaining the internal temperature at 5°C or lower. The mixture was stirred at 5°C or lower until the raw materials were consumed, and then 300 mL of toluene and 150 mL of water were added to the mixture, which was subjected to liquid separation. To the mixture, 300 mL of water was added, and the liquid separation was repeated twice. The obtained organic layer was concentrated and then purified by a chromatography on silica gel to obtain a benzophenone compound represented by formula (97) below with a yield of 94%.

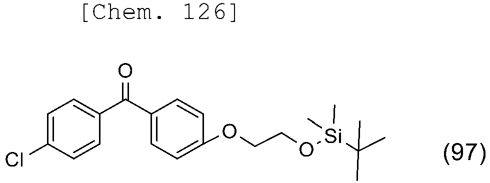

### Second Step

The same reaction as in the fourth step of Example 1 was performed except that the compound represented by formula (97) above was used instead of 4-bromo-4'-methoxybenzophenone, to obtain a propargyl alcohol represented by formula (98) below with a yield of 80%.

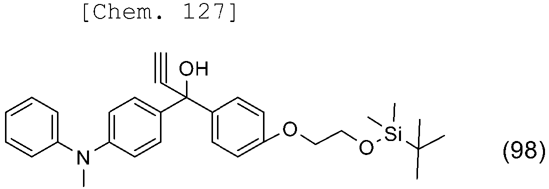

### Third Step

The same reaction as in the first step of Example 2 was performed except that 3-bromo-4-methoxy-4'-mehtylbenzophenone was used instead of 3-bromo-4-methoxybenzophenone and benzomorpholine was used instead of diphenylamine, the same reaction as in the third step of Example 2 was performed except that diethylketone was used instead of diethylcyclohexanone, and the same reaction as in the fifth step of Example 2 was performed except that the propargyl alcohol represented by formula (98) above was used instead of the propargyl alcohol represented by formula (44) above, to obtain a compound represented by formula (99) below with a yield of 58%.

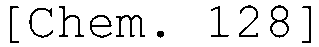

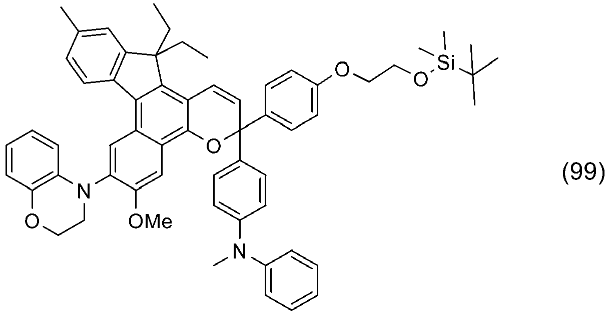

### Fourth Step

To 25 mL of THF, 4.88 g (5.3 mmol) of the compound represented by formula (99) above was dissolved, and cooled the internal temperature to 3°C. Into the mixture, 6 mL of tetrabutylammonium fluoride (1.0 mol/L THF solution) was dripped. The mixture was stirred at an internal temperature of 5°C or lower, and, after confirming that the raw materials had been consumed, 20 mL of water and 20 mL of toluene were added to the mixture, which was subjected to liquid separation. The liquid separation was repeated until the pH of the water layer reached around 7. The obtained organic layer was concentrated and then purified by a chromatography on silica gel to obtain a compound represented by formula (100) below with a yield of 97%.

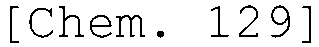

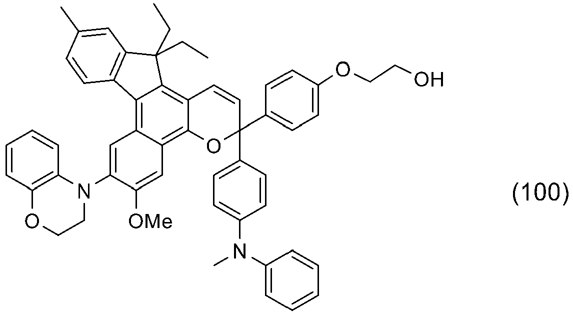

### Fifth Step

By referring to the method described in Patent Document 12, a compound represented by formula (101) below synthesized from polytetramethylene glycol having a number-average molecular weight of 1000 was reacted with the compound represented by formula (100) above to obtain the photochromic compound represented by formula (102) below with a yield of 79%.

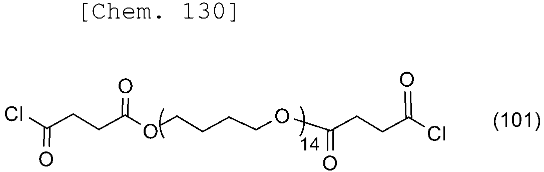

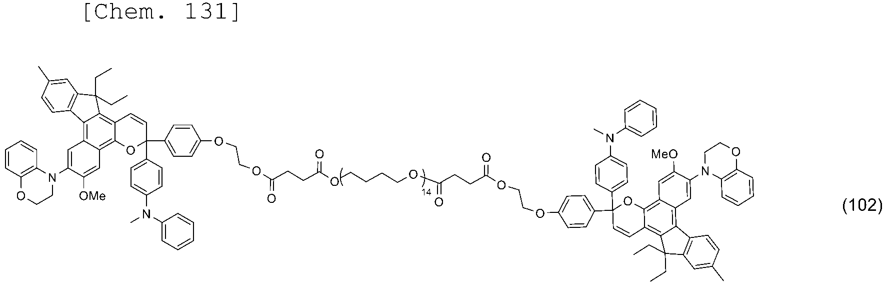

In a proton nuclear magnetic resonance spectroscopy, the photochromic compound represented by formula (102) above showed about 100H peaks attributed to a methyl group, an ethyl group, a benzmorpholino group, a succinic acid group, and a polytetramethylene glycol chain group at around δ0.5 to 3.0 ppm, about 74H peaks attributed to a methoxy group, an ethylene glycol group, a polytetramethylene glycol chain group, and a benzmorpholine group at around δ3.0 to 5.0 ppm, and 48H peaks attributed to aromatic protons and protons of an alkene at around δ5.0 to 9.0 ppm.

### Example 56

### First Step

The same reaction as in the first step of Example 55 was performed except that (4-chlorophenyl) [4-(2-hydroxyethyl)phenyl]methanone was used instead of (4-chlorophenyl) [4-(2-hydroxyethoxy)phenyl]methanone, and the same reaction as in the second step of Example 55 was performed except that 4-methoxy-N-methylaniline was used instead of N-methylaniline, to obtain a propargyl alcohol represented by formula (103) below.

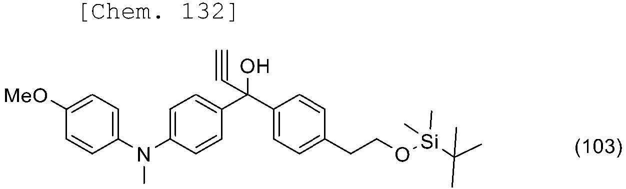

### Second Step

The same reaction as in Example 3 was performed except that (4-methoxyphenyl) (4-phenoxyphenyl)methanone was used instead of 4,4'-dimethoxybenzophonene, 1-iodo-3-methoxypropane was used instead of iodopropane, and the propargyl alcohol represented by formula (103) above was used instead of the propargyl alcohol represented by formula (35) above, to obtain a compound represented by formula (104) below with a yield of 75%.

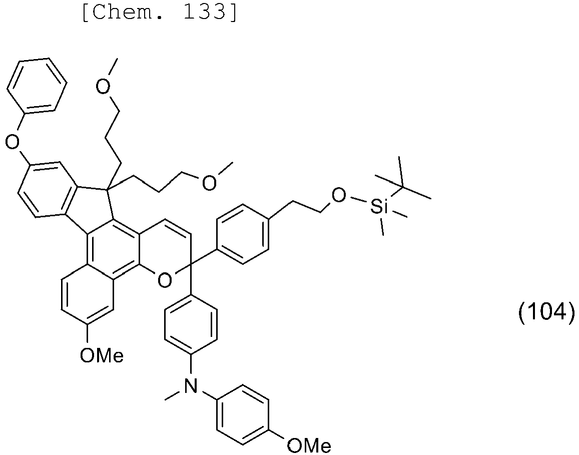

### Third Step

The same reaction as in the fourth step of Example 55 was performed except that the compound represented by formula (104) above was used instead of the compound represented by formula (99) above, and the same reaction as in the fifth step of Example 55 was performed except that polypropylene glycol monobutyl ether having a number-average molecular weight of 1250 was used instead of polytetramethylene glycol having a number-average molecular weight of 1000, to obtain a photochromic compound represented by formula (105) below with a yield of 81%.

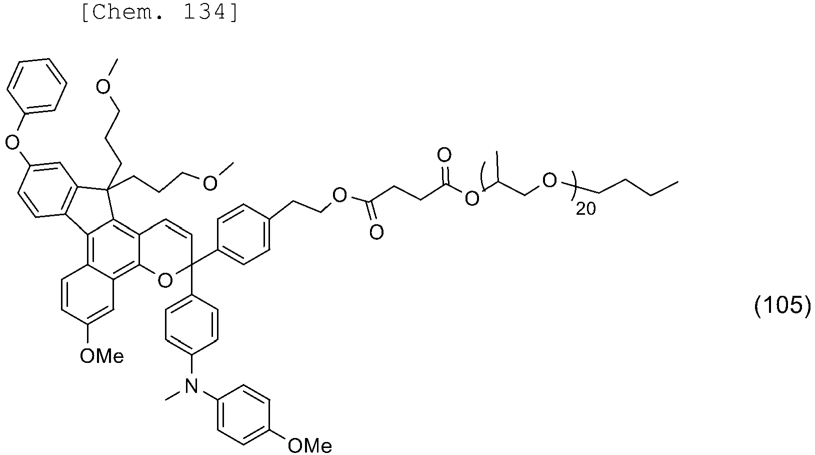

In a proton nuclear magnetic resonance spectroscopy, the photochromic compound represented by formula (105) above showed about 84H peaks attributed to a methyl group, a 3-methoxypropyl group, an ethyl group, a succinic acid group, a polypropylene glycol chain group, and a butyl group at around δ0.5 to 3.0 ppm, about 80H peaks attributed to a methoxy group, a 3-methoxypropyl group, an ethyloxy group, a polypropylene glycol chain group, and a butyloxy group at around δ3.0 to 5.0 ppm, and 25H peaks attributed to aromatic protons and protons of an alkene at around δ5.0 to 9.0 ppm.

### Example 57

### First Step

By referring to the method described in Patent Document 8, the same reaction as in Example 1 was performed except that a compound represented by formula (106) below obtained by reacting 2-bromoanisole with 4-trifluoromethoxybenzoil chloride was used, to synthesize a naphthol derivative represented by formula (107) below.

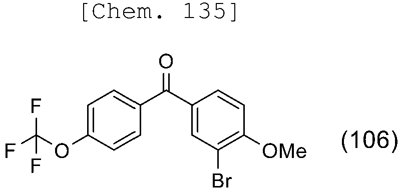

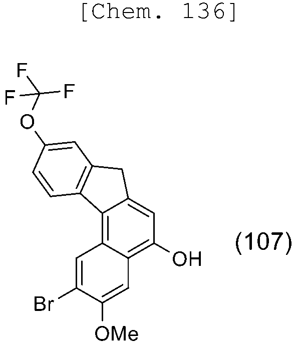

### Second Step

The same reaction as in the fifth step of Example 1 was performed except that the compound represented by formula (107) above was used instead of the compound represented by formula (33) above and a propargyl alcohol represented by formula (108) below was used instead of the propargyl alcohol represented by formula (35) above, and the same reaction as in the sixth step of Example 1 was performed except that iodopropane was used instead of iodomethane, to obtain a compound represented by formula (109) below with a yield of 54%.

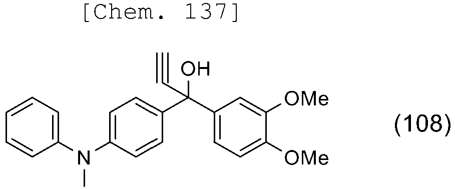

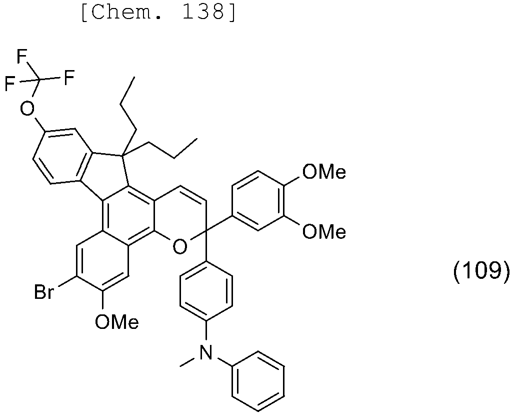

### Third Step

By referring to the method described in Patent Document 10, the compound represented by formula (109) above was reacted with 2,6-dimethylbenzenethiol to obtain a compound represented by formula (110) below with a yield of 88%.

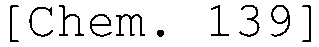

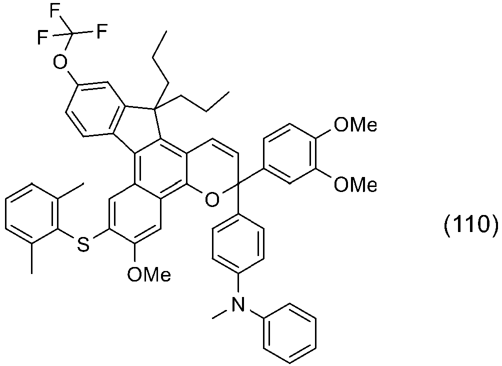

The photochromic compound represented by formula (110) above had elemental analysis values of C: 74.25%, H: 5.88%, N: 1.51%, and S: 3.46%, which were considerably consistent with the C₅₇H₅₄F₃NO₅S calculated values of C: 74.24%, H: 5.90%, N: 1.52%, and S: 3.48%. In a proton nuclear magnetic resonance spectroscopy, the photochromic compound represented by formula (110) showed 23H peaks attributed to a methyl group and a propyl group at around δ0.5 to 3.0 ppm, a 9H peak attributed to a methoxy group at around δ3.0 to 5.0 ppm, and 22H peaks attributed to aromatic protons and protons of an alkene at around δ5.0 to 9.0 ppm. Furthermore, in a ¹³C-nuclear magnetic resonance spectroscopy, the photochromic compound represented by formula (110) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 58

### First Step

The same reaction as in the first step of Example 57 was performed except that 4-phenylbenzoyl chloride was used instead of 4-trifluoromethoxybenzoyl chloride, to synthesize a naphthol derivative represented by formula (111) below.

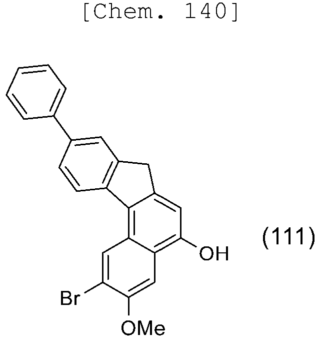

### Second Step

The same reaction as in the fourth step of Example 1 was performed except that a propargyl alcohol represented by formula (112) below synthesized using (4-bromophenyl) [4-[2-(2-methoxyethoxy)ethoxy]phenyl]methanone instead of 4-bromo-4'-methoxybenzophenone was used instead of the propargyl alcohol represented by formula (108) above and 1-bromo-4-methoxybutane was used instead of iodopropane, to obtain a compound represented by formula (113) below with a yield of 59%.

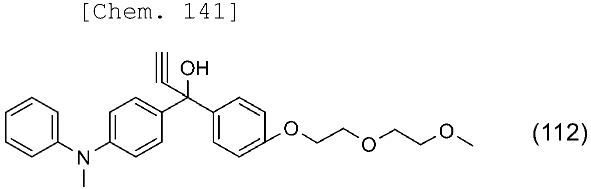

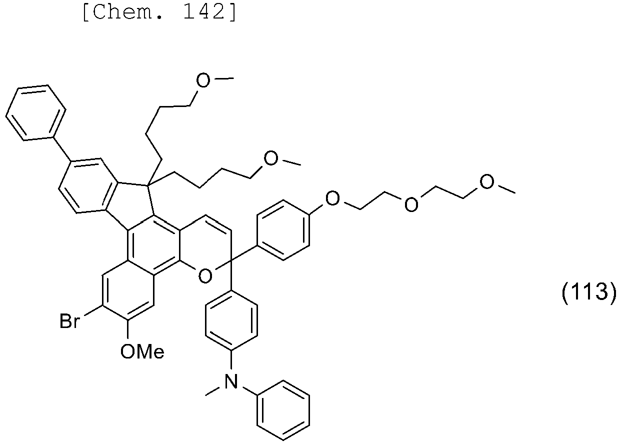

### Third Step

The same reaction as in the first step of Example 2 was performed except that the compound represented by formula (113) above was used instead of the compound represented by formula (38) above and thiomorpholine-1,1-dioxide was used instead of diphenylamine, to obtain a photochromic compound represented by formula (114) below with a yield of 72%.

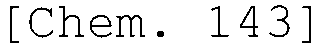

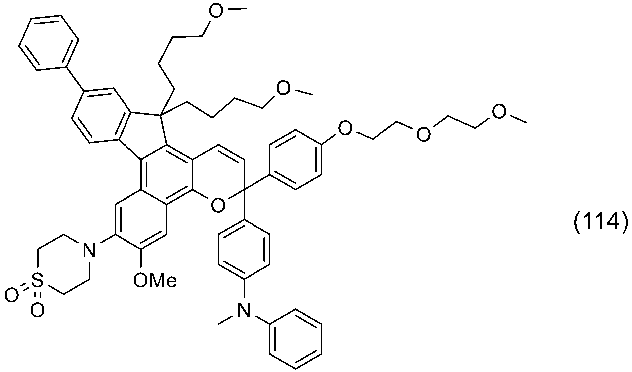

The photochromic compound represented by formula (114) above had elemental analysis values of C: 73.85%, H: 6.84%, N: 2.66%, and S: 3.01%, which were considerably consistent with the C₆₅H₇₂N₂O₉S calculated values of C: 73.84%, H: 6.86%, N: 2.65%, and S: 3.03%. In a proton nuclear magnetic resonance spectroscopy, the photochromic compound represented by formula

(114) showed 19H peaks attributed to a methyl group, a 4-methoxybutyl group, and a thiomorpholine-1,1-dioxide group at around δ0.5 to 3.0 ppm, 28H peaks attributed to a methoxy group, a 4-methoxybutyl group, a thiomorpholine-1,1-dioxide group, and a 2-(2-methoxyethoxy)ethoxy group at around δ3.0 to 5.0 ppm, and 25H peaks attributed to aromatic protons and protons of an alkene at around δ5.0 to 9.0 ppm. Furthermore, in a ¹³C-nuclear magnetic resonance spectroscopy, the photochromic compound represented by formula (114) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 59

### First Step

The same reaction as in the second step of Example 57 was performed except that the propargyl alcohol represented by formula (98) above was used instead of the propargyl alcohol represented by formula (108) above and 1,1,1-trifluoro-3-iodopropane was used instead of iodopropane, to obtain a compound represented by formula (115) below with a yield of 63%.

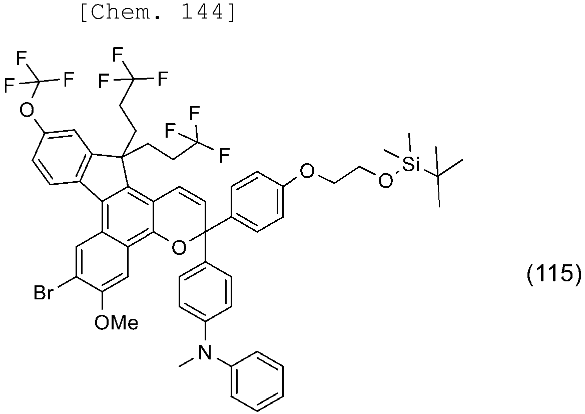

### Second Step

The same reaction as in the third step of Example 1 was performed except that the compound represented by formula

(115) above was used instead of the compound represented by formula (32) above and 2,4-dimethoxyphenylboronic acid was used instead of 4-morpholinophenylboronic acid, to obtain a compound represented by formula (116) below with a yield of 91%.

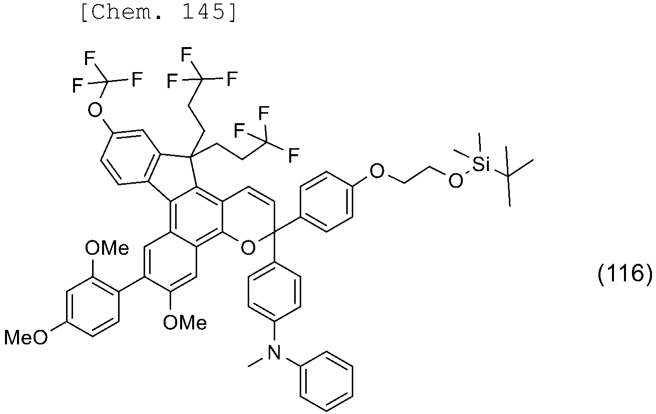

### Third Step

The same reaction as in the fourth step of Example 55 was performed except that the compound represented by formula (116) above was used instead of the compound represented by formula (99) above, and the same reaction as in the fifth step of Example 55 was performed except that sebacoyl chloride was used instead of the compound represented by formula (101) above, to obtain a photochromic compound represented by formula (117) below with a yield of 89%.

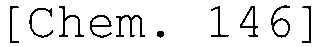

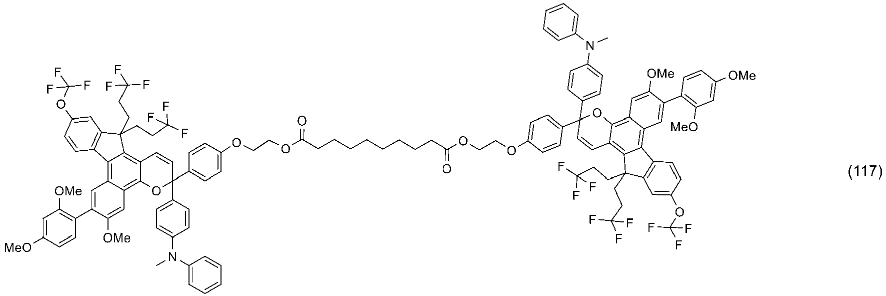

The photochromic compound represented by formula (117) above had elemental analysis values of C: 66.88%, H: 4.69%, and N: 1.25%, which were considerably consistent with the C₁₂₄H₁₁₀F₁₈N₂O₁₆ calculated values of C: 66.90%, H: 4.98%, and N: 1.26%. In a proton nuclear magnetic resonance spectroscopy, the photochromic compound represented by formula (117) showed 38H peaks attributed to a methyl group, a 1,1,1-trifluoropropyl group, and a sebacic acid group at around δ0.5 to 3.0 ppm, 26H peaks attributed to a methoxy group and an ethylene glycol group at around δ3.0 to 5.0 ppm, and 46H peaks attributed to aromatic protons and protons of an alkene at around δ5.0 to 9.0 ppm. Furthermore, in a ¹³C-nuclear magnetic resonance spectroscopy, the photochromic compound represented by formula (117) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 60

### First Step

The same reaction as in the first step of Example 57 was performed except that 4-trifluoromethylbenzoyl chloride was used instead of 4-trifluoromethoxybenzoyl chloride, to synthesize a compound represented by formula (118) below with a yield of 86%.

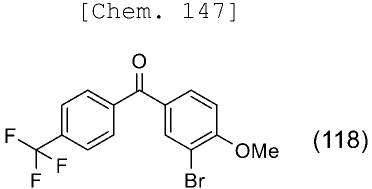

### Second Step

The same reaction as in the first step of Example 2 was performed except that the compound represented by formula (118) above was used instead of 3-bromo-4-methoxybenzophenone and N-methylaniline was used instead of diphenylamine, and the same reaction as in the third step of Example 2 was performed except that 4,4-dimethylcyclohexanone was used instead of 4,4-diethylcyclohexanone and the propargyl alcohol represented by formula (103) above was used instead of the propargyl alcohol represented by formula (44) above, to synthesize a compound represented by formula (119) below.

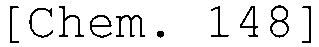

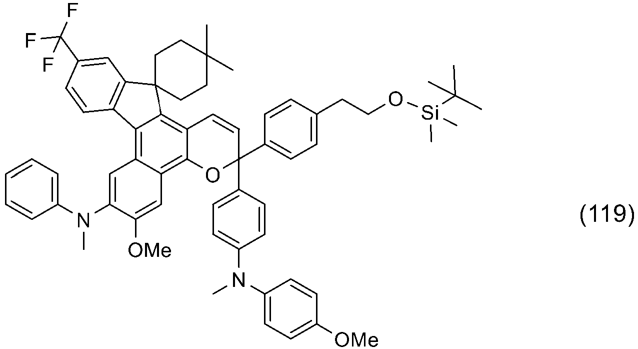

### Third Step

The same reaction as in the fourth step of Example 55 was performed except that the compound represented by formula (119) above was used instead of the compound represented by formula (99) above, to synthesize a compound represented by formula (120) below with a yield of 92%.

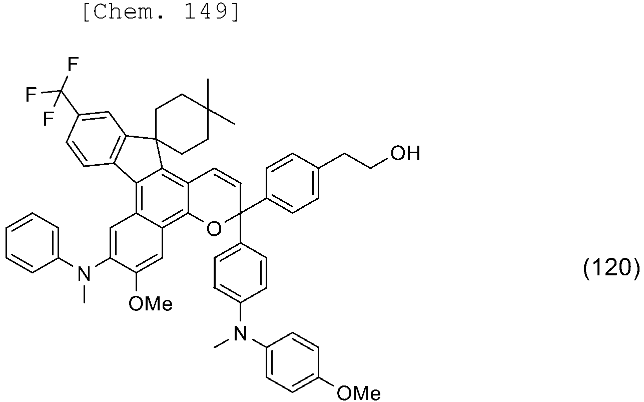

### Fourth Step

The same reaction as in the first step of Example 55 was performed except that the compound represented by formula (120) above was used instead of (4-chlorophenyl) [4-(2-hydroxyethoxy)phenyl]methanone and [tris(trimethylsiloxy)silylethyl]dimethylchlorosilane was used instead of t-butyldimethylchlorosilane, to obtain a photochromic compound represented by formula (121) below with a yield of 92%.

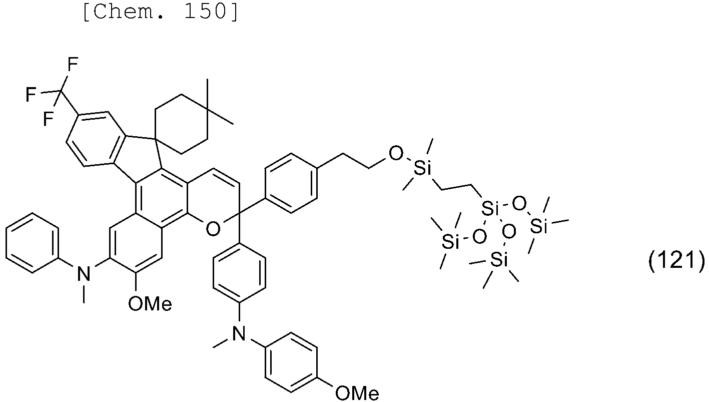

The photochromic compound represented by formula (121) above had elemental analysis values of C: 66.50%, H: 7.17%, and N: 2.22%, which were considerably consistent with the C₇₁H₉₁F₃N₂O₇Si₅ calculated values of C: 66.52%, H: 7.16%, and N: 2.19%. In a proton nuclear magnetic resonance spectroscopy, the photochromic compound represented by formula (121) showed 59H peaks attributed to a methyl group, a [tris(trimethylsiloxy)silylethyl]silyl group, and a 4,4-dimethylcyclohexyl group at around δ0.5 to 3.0 ppm, 8H peaks attributed to a methoxy group and an ethyloxysilyl group at around δ3.0 to 5.0 ppm, and 24H peaks attributed to aromatic protons and protons of an alkene at around δ5.0 to 9.0 ppm. Furthermore, in a ¹³C-nuclear magnetic resonance spectroscopy, the photochromic compound represented by formula (121) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 61

The same reaction as in the first step of Example 2 was performed except that 3-methoxybenzophenone was used instead of 3-bromo-4-methoxybenzophenone, the same reaction as in the third step of Example 2 was performed except that 3,3,5,5-tetramethylcyclohexanone was used instead of 4,4-diethylcyclohexanone, and the same reaction as in the fifth step of Example 2 was performed except that the propargyl alcohol represented by formula (49) above was used instead of the propargyl alcohol represented by formula (44) above, to obtain a photochromic compound represented by formula (122) below with a yield of 67%.

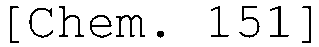

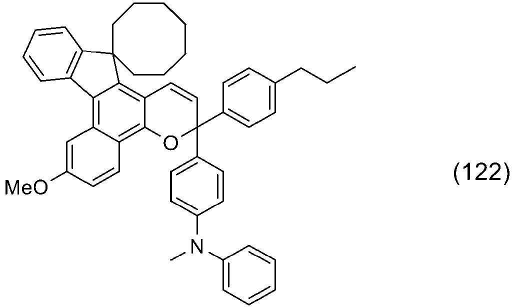

The photochromic compound represented by formula (122) above had elemental analysis values of C: 86.27%, H: 7.08%, and N: 2.03%, which were considerably consistent with the C₅₀H₄₉NO₂ calculated values of C: 86.29%, H: 7.10%, and N: 2.01%. In a proton nuclear magnetic resonance spectroscopy, the photochromic compound represented by formula (122) showed 24H peaks attributed to a methyl group, a propyl group, and a cyclooctyl group at around δ0.5 to 3.0 ppm, a 3H peak attributed to a methoxy group at around δ3.0 to 5.0 ppm, and 22H peaks attributed to aromatic protons and protons of an alkene at around δ5.0 to 9.0 ppm. Furthermore, in a ¹³C-nuclear magnetic resonance spectroscopy, the photochromic compound represented by formula (122) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 62

### First Step

The same reaction as in the fourth step of Example 1 was performed except that (4-bromophenyl) (2,3-dihydro-5-benzofuranyl)methanone was used instead of 4-bromo-4'-methoxybenzophenone, to obtain a propargyl alcohol represented by formula (123) below with a yield of 79%.

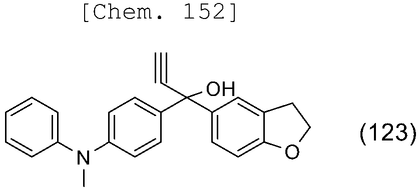

### Second Step

The same reaction as in the first step of Example 1 was performed except that 4-bromo-3-methoxybenzophenone was used instead of 3-bromo-4-methoxybenzophenone, the same reaction as in the third step of Example 1 was performed except that 2,4-dimethoxyphenylboronic acid was used instead of 4-morpholinophenylboronic acid, the same reaction as in the fifth step of Example 1 was performed except that the propargyl alcohol represented by formula (123) above was used instead of the propargyl alcohol represented by formula (35) above, and the same reaction as in the sixth step of Example 1 was performed except that 1-bromo-4-methoxybutane was used instead of iodomethane, to obtain a photochromic compound represented by formula (124) below with a yield of 52%.

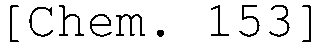

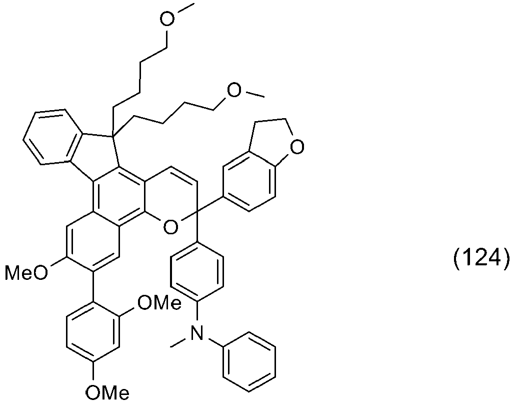

The photochromic compound represented by formula (124) above had elemental analysis values of C: 79.36%, H: 6.77%, and N: 1.52%, which were considerably consistent with the C₆₀H₆₁NO₇ calculated values of C: 79.35%, H: 6.77%, and N: 1.54%. In a proton nuclear magnetic resonance spectroscopy, the photochromic compound represented by formula (124) showed 17H peaks attributed to a methyl group, a 4-methoxybutyl group, and a dihydrobenzofuran group at around δ0.5 to 3.0 ppm, 21H peaks attributed to a methoxy group, a 4-methoxybutyl group, and a dihydrobenzofuran group at around δ3.0 to 5.0 ppm, and 23H peaks attributed to aromatic protons and protons of an alkene at around δ5.0 to 9.0 ppm. Furthermore, in a ¹³C-nuclear magnetic resonance spectroscopy, the photochromic compound represented by formula (124) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Examples 63 to 74)

### <Physical Property Evaluation for Photochromic Plastic Lens Prepared by Coating Method>

In the same manner as in Example 26, photochromic laminates in Examples 63 to 74 were prepared using the photochromic compounds obtained in Examples 51 to 62. Note that, in Examples 67 and 71 using the compound in Example 55 (compound represented by formula (101) above) and the compound in Example 59 (compound represented by formula (117) above) respectively, the addition amount of the photochromic compound was 0.125 mmol based on 100 g of the total amount of the radical-polymerizable monomers. The results are presented in Table 4.

### [Table 4]

**[table 4]**

| | Compound No. | Maximum absorption wavelength (nm) | A23 (-) | 23°C color fading half-life (sec) | Residual rate (%) |
|---|---|---|---|---|---|
| Example 63 | Example 51 | 482 | 0.44 | 20 | 60% |
| | Formula (89) | 603 | 0.43 | 20 | 60% |
| Example 64 | Example 52 | 488 | 0.60 | 45 | 58% |
| | Formula (93) | 604 | 0.67 | 45 | 56% |
| Example 65 | Example 53 | 500 | 0.73 | 56 | 58% |
| | Formula (95) | 605 | 0.91 | 56 | 60% |
| Example 66 | Example 54 | 501 | 0.72 | 37 | 67% |
| | Formula (96) | 628 | 1.03 | 38 | 67% |
| Example 67 | Example 55 | 545 | 1.18 | 41 | 62% |
| | Formula (101) | | | | |
| Example 68 | Example 56 | 469 | 0.75 | 96 | 60% |
| | Formula (105) | 592 | 1.47 | 95 | 58% |
| Example 69 | Example 57 | 493 | 0.82 | 54 | 65% |
| | Formula (110) | 615 | 0.85 | 55 | 65% |
| Example 70 | Example 58 | 498 | 1.12 | 52 | 60% |
| | Formula (114) | 617 | 1.12 | 53 | 60% |
| Example 71 | Example 59 | 491 | 0.47 | 14 | 63% |
| | Formula (117) | 600 | 0.68 | 13 | 63% |
| Example 72 | Example 60 | 523 | 0.79 | 40 | 62% |
| | Formula (121) | 582 | 0.84 | 42 | 64% |
| Example 73 | Example 61 | 487 | 1.18 | 156 | 59% |
| | Formula (122) | 564 | 1.87 | 154 | 57% |
| Example 74 | Example 62 | 477 | 0.97 | 87 | 61% |
| | Formula (124) | 587 | 1.08 | 88 | 61% |

### <Physical Property Evaluation for Photochromic Layer Prepared by Binder Method>

### (Example 75)

A binder sheet was prepared using the following method. The binder sheet consisted of a first optical sheet, a first adhesive layer, a photochromic layer, a second adhesive layer, and a second optical sheet, which are laminated in this order. The first optical sheet and the second optical sheet were made of a polycarbonate sheet with a thickness of 400 µm.

### (Preparation of Photochromic Layer Forming Composition)

A 2L four-necked flask equipped with agitation blades, a cooling tube, a thermometer, and a nitrogen gas introducing tube was charged with 315 parts by mass of polycarbonate diol having a number-average molecular weight of 1000, 100 parts by mass of isophorone diisocyanate, and 72 parts by mass of toluene, and the mixture was reacted under nitrogen atmosphere at 100°C for 7 hours to synthesize a urethane prepolymer having an isocyanate group on its terminal. After completion of the urethane prepolymer reaction, the reaction liquid was cooled to approximately 0°C, then dissolved in 205 parts by mass of t-butyl alcohol and 382 parts by mass of diethyl ketone, and then the liquid temperature was maintained at 0°C. Subsequently, into this reaction liquid, a mixture of 21.3 parts by mass of bis-(4-aminocyclohexyl)methane as a chain extender and 20 parts by mass of diethyl ketone was dripped over within 30 minutes, and the mixture was reacted at 0°C for 1 hour. Then, into the mixture, 8.1 parts by mass of 1,2,2,6,6-pentamethyl-4-aminopiperidine was dripped, and the mixture was reacted at 0°C for 1 hour to obtain a diethyl ketone solution of a terminal non-reactive urethaneurea resin.

To 100 parts by mass of the obtained terminal non-reactive urethaneurea resin solution, the photochromic compound in Example 1 (represented by formula (28) above), 6.3 parts by mass of 4,4'-methylenebis(cyclohexyl isocyanate) isomer mixture (polyisocyanate compound), furthermore 0.4 part by mass of ethylene bis(oxyethylene)bis[3-(5-tert-butyl-4-hydroxy-m-tolyl)propionate] as an antioxidant, and 0.06 part by mass of DOW CORNING TORAY L-7001 as a surfactant were added, and the mixture was stirred and mixed at room temperature to obtain a photochromic layer forming composition.

Note that the addition amount of the photochromic compound was 0.25 mmol based on 100 parts by mass of the terminal non-reactive urethane urea resin.

### (Preparation of Adhesive Layer Composition)

A 5L separable flask (four-necked) equipped with agitation blades, a cooling tube, a thermometer, and a nitrogen gas introducing tube was charged with 400 parts by mass of polycarbonate diol having a number-average molecular weight of 1000, 175 parts by mass of isophorone diisocyanate, and 120 parts by mass of toluene, and the mixture was reacted under nitrogen atmosphere at 110°C for 7 hours to synthesize a urethane prepolymer having an isocyanate group on its terminal. After completion of the urethane prepolymer reaction, the reaction liquid was cooled to approximately 20°C, then dissolved in 2500 parts by mass of propylene glycol monomethyl ether, and then the liquid temperature was maintained at 20°C. Subsequently, into this reaction liquid, 60 parts by mass of isophorone diamine as a chain extender was dripped, and the mixture was reacted at 20°C for 1 hour. Then, into this mixture, 3 parts by mass of n-butylamine was dripped, and the mixture was reacted at 20°C for 1 hour to obtain a propylene glycol-monomethyl ether solution of a terminal non-reactive urethaneurea resin.

To 500 parts by mass of the obtained terminal non-reactive urethaneurea resin solution, 0.2 part by mass of DOW CORNING TORAY L-7001 as a surfactant was added, and the mixture was stirred and mixed at room temperature to obtain an adhesive layer composition.

### (Production of Binder Sheet)

Using a coater (manufactured by TESTER SANGYO CO., LTD.), the adhesive layer composition was applied onto one main face of the first optical sheet at a coating speed of 0.5 m/min, and dried at a drying temperature of 110°C for 3 minutes to obtain a first optical sheet having a 5 µm-thick first coating film. In the same manner as above, the adhesive layer composition was coated onto one main face of the second optical sheet to obtain a second optical sheet having a second coating film.

Subsequently, using a coater (manufactured by TESTER SANGYO CO., LTD.), a photochromic layer forming composition was applied onto a 50 µm-thick OPP film (stretched polypropylene film) at a coating speed of 0.3 m/min, and dried at a drying temperature of 100°C for 5 minutes. Thereby, a third coating film was obtained. Subsequently, the third coating film and the first optical sheet were bonded to each other so that the third coating film was in contact with the first coating film. From this structure, the OPP film was peeled off, and the second optical sheet and the third coating film were bonded to each other so that the exposed the other main face of the third coating film was in contact with the second coating film. Subsequently, the resulting laminate was allowed to stand under vacuum at 40°C for 24 hours, then heated at 110°C for 60 minutes, then humidified at 60°C and 100% RH for 24 hours, and finally allowed to stand under vacuum at 40°C for 24 hours to obtain a binder sheet. The obtained binder sheet was evaluated in the same manner as in Example 26. The results are presented in Table 4.

### (Examples 76 to 83, and Comparative Examples 9 to 13)

Binder sheets were prepared by the same manner as in Example 50 using the photochromic compounds presented in Table 5.

### [Table 5]

**[table 5]**

| | Compound No. | Maximum absorption wavelength (nm) | A23 (-) | 23°C color fading half-life (sec) | Residual rate (%) |
|---|---|---|---|---|---|
| Example 75 | Example 1 | 502 | 0.97 | 90 | 89% |
| | Formula (37) | 603 | 1.06 | 91 | 90% |
| Example 76 | Example 25 | 505 | 0.88 | 76 | 87% |
| | Formula (88) | 606 | 0.97 | 77 | 87% |
| Comparative Example 9 | Formula (A) | 503 | 0.89 | 68 | 63% |
| | | 599 | 0.88 | 69 | 63% |
| Comparative Example 10 | Formula (B) | 503 | 1.31 | 220 | 79% |
| | | 588 | 1.28 | 222 | 79% |
| Comparative Example 11 | Formula (C) | 475 | 1.6 | 447 | 56% |
| Comparative Example 12 | Formula (F) | 490 | 1.12 | 105 | 77% |
| | | 585 | 1.04 | 106 | 76% |
| Comparative Example 13 | Formula (G) | 500 | 1.05 | 86 | 71% |
| | | 594 | 1.08 | 87 | 71% |
| Example 77 | Example 10 | 516 | 1.35 | 129 | 91% |
| | Formula (62) | 594 | 1.12 | 128 | 91% |
| Example 78 | Example 11 | 479 | 0.51 | 43 | 90% |
| | Formula (65) | 612 | 0.97 | 43 | 89% |
| Example 79 | Example 19 | 492 | 0.88 | 100 | 91% |
| | Formula (80) | 604 | 1.14 | 99 | 91% |
| Example 80 | Example 23 | 506 | 1.02 | 52 | 89% |
| | Formula (85) | 609 | 1.06 | 52 | 89% |
| Example 81 | Example 53 | 502 | 0.75 | 60 | 87% |
| | Formula (95) | 607 | 0.96 | 60 | 89% |
| Example 82 | Example 57 | 493 | 0.85 | 61 | 91% |
| | Formula (110) | 618 | 0.87 | 61 | 90% |
| Example 83 | Example 60 | 524 | 0.84 | 42 | 88% |
| | Formula (121) | 582 | 0.87 | 43 | 89% |

### <Physical Property Evaluation for Photochromic Cured Product Prepared by Kneading Method>

### (Example 84)

### (Preparation of Curable Composition)

First, the photochromic compound obtained in Example 1, additives, and a polymerizable compound were mixed to obtain a curable composition.

As the polymerizable compound, a combination of the following polymerizable monomers was used.
1,3-bis(isocyanatomethyl)cyclohexane: 36.7 parts by mass Pentaerythritol tetrakis(3-mercaptopropionate): 39.4 parts by mass
Polyoxyethylene polyoxypropylene lauryl ether (Wonder Surf 140 manufactured by Aoki Oil Industrial Co., Ltd.): 17.4 parts by mass
1-decanethiol: 2.8 parts by mass
RX-1 adjusted according to the method described in Patent Document 13: 3.8 parts by mass

Note that the photochromic compound was added to the curable composition so that the amount of the photochromic compound was 0.106 mmol based on 100 g of the total amount of the polymerizable monomer. The following additives were used: Dimethyltin dichloride
: 0.05 part by mass
Irganox 245: 0.1 part by mass
2-ethylhexyl 4-methoxycinnamate: 0.6 part by mass

### (Production of Cured Product)

The prepared curable composition was thoroughly defoamed, then injected into a glass mold having a 1 mm gap, and polymerized by cast polymerization. The polymerization was carried out in an air furnace while gradually raising the temperature from 27°C to 120°C over 18 hours. After the polymerization, the cured product was removed from the glass mold to obtain a photochromic cured product having a thickness of 1 mm. The obtained photochromic cured product was evaluated in the same manner as in Example 26. The results are presented in Table 6.

(Examples 85 to 99, and Comparative Example 14 to 18) Photochromic cured products were prepared using photochromic compounds presented in Tables 6 and 7 in the same manner as in Example 84. The results of Examples 84 and 85 and Comparative Examples 14 to 18 are summarized in Table 6, and the results of Examples 86 to 99 are summarized in Table 7.

### [Table 6]

**[table 6]**

| | Compound No. | Maximum absorption wavelength (nm) | A23 (-) | 23°C color fading half-life (sec) | Residual rate (%) |
|---|---|---|---|---|---|
| Example 84 | Example 1 | 503 | 0.96 | 112 | 72% |
| | Formula (37) | 610 | 1.06 | 112 | 72% |
| Example 85 | Example 25 | 505 | 0.88 | 96 | 68% |
| | Formula (88) | 613 | 0.96 | 95 | 68% |
| Comparative Example 14 | Formula (A) | 503 | 0.92 | 81 | 50% |
| | | 607 | 0.89 | 81 | 50% |
| Comparative Example 15 | Formula (B) | 506 | 1.33 | 268 | 61% |
| | | 593 | 1.27 | 267 | 61% |
| Comparative Example 16 | Formula (C) | 475 | 1. 68 | 561 | 39% |
| Comparative Example 17 | Formula (F) | 493 | 1.14 | 129 | 58% |
| | | 591 | 1.05 | 129 | 59% |
| Comparative Example 18 | Formula (G) | 502 | 1.05 | 107 | 54% |
| | | 602 | 1.07 | 108 | 54% |

### [Table 7]

**[table 7]**

| | Compound No. | Maximum absorption wavelength (nm) | A23 (-) | 23°C color fading half-life (sec) | Residual rate (%) |
|---|---|---|---|---|---|
| Example 86 | Example 2 | 526 | 1.41 | 105 | 72% |
| | Formula (45) | | | | |
| Example 87 | Example 3 | 472 | 0.59 | 99 | 61% |
| | Formula (46) | 619 | 1.16 | 99 | 61% |
| Example 88 | Example 5 | 511 | 0.56 | 68 | 75% |
| | Formula (51) | 598 | 0.69 | 67 | 75% |
| Example 89 | Example 7 | 499 | 0.75 | 99 | 68% |
| | Formula (55) | 610 | 0.99 | 100 | 68% |
| Example 90 | Example 8 | 476 | 0.51 | 105 | 68% |
| | Formula (57) | 604 | 1.24 | 105 | 68% |
| Example 91 | Example 10 | 519 | 1.41 | 165 | 68% |
| | Formula (62) | 600 | 1. 09 | 163 | 70% |
| Example 92 | Example 14 | 496 | 0.59 | 72 | 71% |
| | Formula (71) | 590 | 0.65 | 72 | 70% |
| Example 93 | Example 16 | 475 | 0.47 | 68 | 64% |
| | Formula (77) | 593 | 0.86 | 68 | 64% |
| Example 94 | Example 19 | 496 | 0.87 | 130 | 73% |
| | Formula (80) | 610 | 1.15 | 131 | 73% |
| Example 95 | Example 21 | 490 | 0.62 | 102 | 68% |
| | Formula (83) | 616 | 1.25 | 103 | 68% |
| Example 96 | Example 24 | 505 | 1.32 | 124 | 65% |
| | Formula (86) | 607 | 1.31 | 125 | 65% |
| Example 97 | Example 51 | 486 | 0.46 | 30 | 68% |
| | Formula (89) | 612 | 0.45 | 30 | 68% |
| Example 98 | Example 57 | 496 | 0.86 | 74 | 72% |
| | Formula (110) | 625 | 0.88 | 74 | 72% |
| Example 99 | Example 62 | 479 | 1.02 | 125 | 67% |
| | Formula (124) | 594 | 1.11 | 126 | 67% |

Preferable aspects of the present invention will be described below.
[1] A photochromic compound having a skeleton represented by formula (1) below: 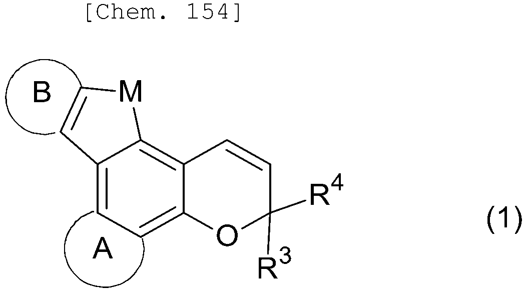 in which, in formula (1) above,
   M is C, Si, or Ge,
   R³ is an aryl group or heteroaryl group substituted with a group represented by formula (1a) below, 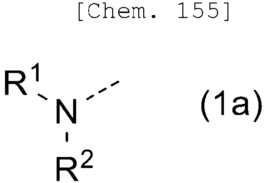 in formula (1a) above,
   R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
   R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below,

      -Q¹-(X¹Q²)a-X²Q³ (2a)

      in formula (2a) above,
      Q¹ is an alkylene group or a haloalkylene group,
      Q² is an alkylene group or a haloalkylene group,
      Q³ is an alkyl group or a haloalkyl group,
      X¹ and X² are each independently O, S, NR⁷⁰⁰, PR⁷⁰¹, or P(=O), R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
      a is an integer of 0, or 1 or more and 3 or less,
      R⁴ is a substituted or unsubstituted aryl group or a substituted or unsubstituted heteroaryl group, and ring A and ring B are each independently a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[2] The photochromic compound as described in [1], in which the photochromic compound has a skeleton represented by formula (2) below: 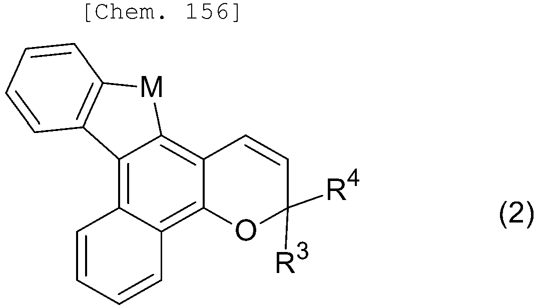 in which, in formula (2) above, M, R³, and R⁴ are each the same as those in formula (1) above.
[3] The photochromic compound as described in [1] or [2], in which the photochromic compound has a skeleton represented by formula (3) below: 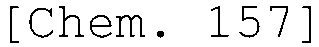 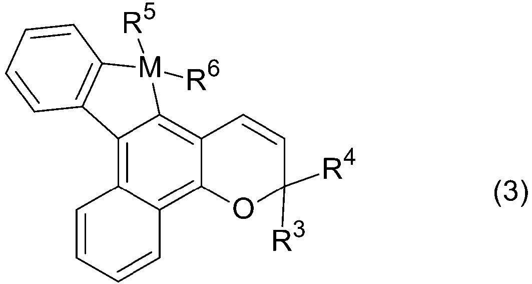 in which, in formula (3) above, M, R³, and R⁴ are each the same as those in formula (1) above,
   R⁵ and R⁶ are each independently a hydrogen atom, a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) below, 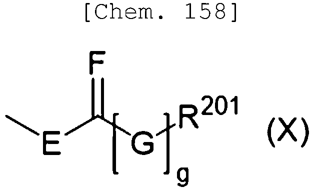 in formula (X),
   E is an oxygen atom or NR¹⁰¹, and R¹⁰¹ is a hydrogen atom or an alkyl group,
   F is an oxygen atom or a sulfur atom,
   G is an oxygen atom, a sulfur atom, or NR²⁰², R²⁰² is a hydrogen atom, an alkyl group, a cycloalkyl group, an aryl group, or a heteroaryl group,
   g is 0 or 1,
   R²⁰¹ is a hydrogen atom, an alkyl group, a cycloalkyl group, an aryl group, or a heteroaryl group,
   when G is an oxygen atom or a sulfur atom, R²⁰¹ is a group other than a hydrogen atom,

      L¹-R⁴⁰⁰ (X3)
   in formula (X3) above,
   R⁴⁰⁰ is a hydrogen atom, an alkyl group, an aryl group, a polymerizable group, a photochromic group, or a silyl group having, as a substituent, an alkyl group, an alkoxy group, or an aryl group,
   L¹ is a group represented by formula (X2) below, 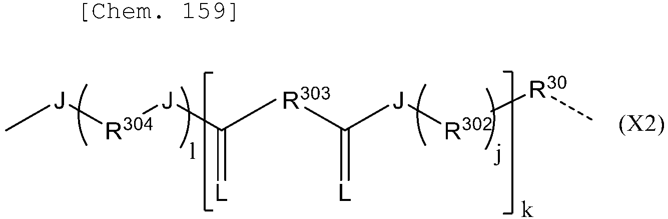 in formula (X2) above, R³⁰ is a group represented by formula (X2a) below, 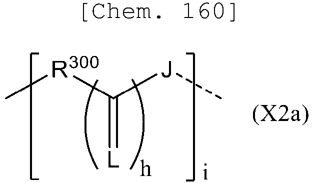 in formulas (X2) and (X2a) above,
   J represents a divalent group, each independently being directly bonded, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group or a silylene group having an alkyl group or an aryl group as a substituent,
   R³⁰², R³⁰³, and R³⁰⁴ are each independently an alkylene group, h, j, k, and l are each independently an integer of 0 or 1, i is an integer of 1 to 200, and when i is 2 or more, a plurality of R³⁰ groups may be the same as or different from each other, the dashed lines indicate bonds with R⁴⁰⁰, and R⁵ and R⁶, together with M, may constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring.
[4] The photochromic compound as described in [3], in which R⁵ and R⁶ are each independently a hydrogen atom, a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (X) above, or a group represented by formula (X3) above.
[5] The photochromic compound as described in [3], in which the photochromic compound is represented by formula (4) below: 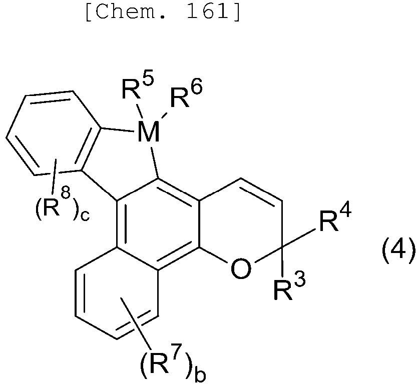 in which, in formula (4) above, R³, R⁴, R⁵, R⁶, and M are each the same as those in formula (3) above,
   R⁷ and R⁸ are each independently a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted haloalkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) above, or a group represented by formula (X3) above,
   b is an integer of 0 to 4, and c is an integer of 0 to 4, when b is 2 to 4, a plurality of R⁷ groups may be the same as or different from each other,
   when c is 2 to 4, a plurality of R⁸ groups may be the same as or different from each other,
   when b is 2 to 4 and there are a R⁷ groups adjacent to each other, the two adjacent R⁷ groups, together with carbon atoms to which the R⁷ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, and when c is 2 to 4 and there are a R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[6] The photochromic compound as described in [5], in whichi the photochromic compound is represented by formula (5) below: 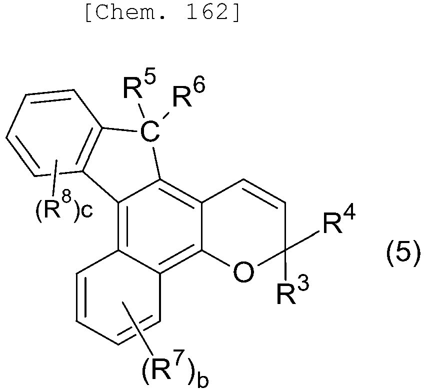 in which, in formula (5) above, R³, R⁴, R⁵, R⁶, R⁷, R⁸, b, and c are each the same as those in formula (4) above.
[7] The photochromic compound as described in [6], in which the photochromic compound is represented by formula (6) below: 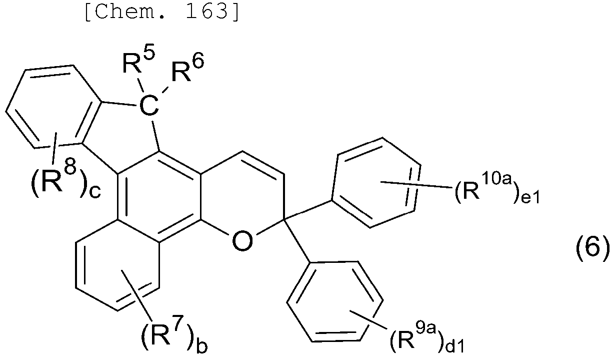 in which, in formula (6) above, R⁵, R⁶, R⁷, R⁸, b, and c are each the same as those in formula (5) above,
   R^{9a} is a group represented by formula (1a) above, a hydrogen atom, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   d1 is an integer of 1 to 5, when d1 is 1, R^{9a} is a group represented by formula (1a) above,
   when d1 is 2 to 5, at least one of a plurality of R^{9a} groups is a group represented by formula (1a) above, the plurality of R^{9a} groups may be the same as or different from each other,
   when there are R^{9a} groups adjacent to each other, except for the group represented by formula (1a) above, the two adjacent R^{9a} groups, together with carbon atoms to which the R^{9a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   R^{10a} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   e1 is an integer of 0 to 5,
   when e1 is 2 to 5, the plurality of R^{10a} may be the same as or different from each other, and when there are R^{10a} groups adjacent to each other, the two adjacent R^{10a} groups, together with carbon atoms to which the R^{10a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[8] The photochromic compound as described in [7], in which the photochromic compound is represented by formula (7) below: 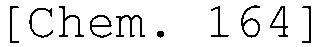 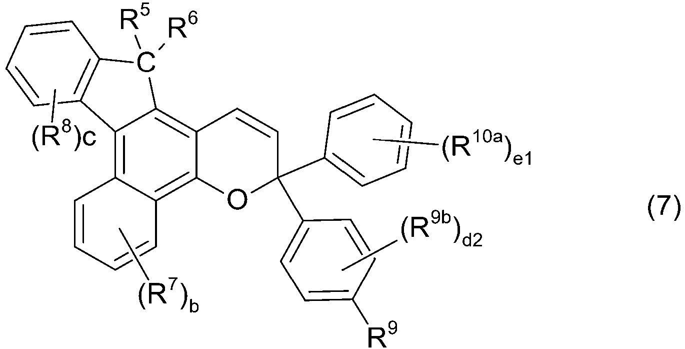 in which, in formula (7) above, R⁵, R⁶, R⁷, R⁸, R^{10a}, b, c, and e1 are each the same as those in formula (6) above,
   R⁹ is a group represented by formula (1a) above,
   R^{9b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   d2 is an integer of 0 to 4,
   when d2 is 2 to 4, a plurality of R^{9b} groups may be the same as or different from each other, and
   when there are R^{9b} groups adjacent to each other, the two adjacent R^{9b} groups, together with carbon atoms to which the R^{9b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[9] The photochromic compound as described in [8], in which the photochromic compound is represented by formula (8) below: 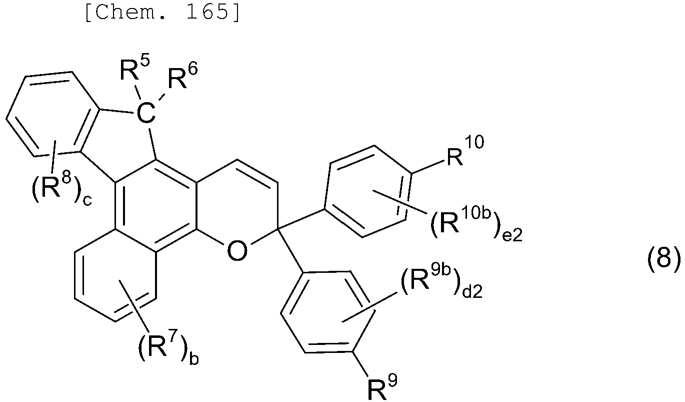 in which, in formula (8) above, R⁵, R⁶, R⁷, R⁸, R⁹, R^{9b}, b, c, and d2 are each the same as those in formula (7) above,
   R¹⁰ is an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a substituted or unsubstituted silyl group, a substituted or unsubstituted oxysilyl group, a group represented by formula (2a) above, or a group represented by formula (X3) above,
   R^{10b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   e2 is an integer of 0 to 4,
   when e2 is 2 to 4, a plurality of R^{10b} groups may be the same as or different from each other, and
   when there are R^{10b} groups adjacent to each other, the two adjacent R^{10b} groups, together with carbon atoms to which the R^{10b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[10] The photochromic compound as described in any one of [1] to [9], in which R¹ is a substituted or unsubstituted phenyl group.
[11] The photochromic compound as described in any one of [1] to [10], in which R² is a substituted or unsubstituted alkyl group having 1 or more and 10 or less carbon atoms, a substituted or unsubstituted cycloalkyl group having 5 or more and 10 or less carbon atoms, a substituted or unsubstituted haloalkyl group having 1 or more and 10 or less carbon atoms, or a group represented by formula (2a) above.
[12] A curable composition including the photochromic compound as described in any one of [1] to [11], and at least one selected from a group consisting of a radical polymerizable monomer, a cationic polymerizable monomer, a compound having a polymerizable group, and a (thio)urethane(urea) polymer.
[13] A cured product of the curable composition as described in [12].
[14] An optical article including the cured product as described in [13].
[15] A lens including the photochromic compound as described in any one of [1] to [11].
[16] Eyeglasses including the lens as described in [15].
[17] A propargyl alcohol compound represented by formula (9) below; 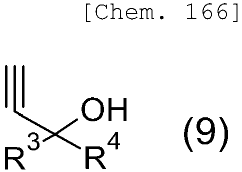 in which, in formula (9) above,
   R³ is an aryl group or a heteroaryl group substituted with a group represented by formula (1a) below, 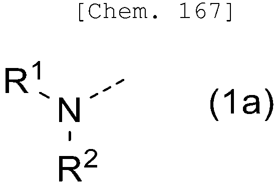 in formula (1a) above,
   R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
   R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below,

      -Q¹-(X¹Q²)a-X²Q³ (2a)
   in formula (2a) above,
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰, PR⁷⁰¹, or P(=O), R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
   a is an integer of 0, or 1 or more and 3 or less, and R⁴ is a substituted or unsubstituted aryl group or a substituted or unsubstituted heteroaryl group.
[18] The propargyl alcohol compound as described in [17], in which the propargyl alcohol compound is represented by formula (10) below: 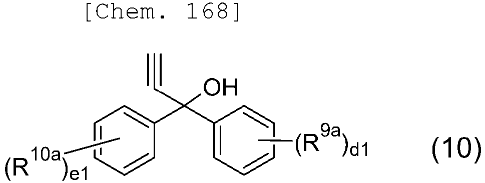 in formula (10) above,
   R^{9a} is a group represented by formula (1a) above, a hydrogen atom, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,

      L¹-R⁴⁰⁰ (X3)
   in formula (X3) above,
   R⁴⁰⁰ is a hydrogen atom, an alkyl group, an aryl group, a polymerizable group, a photochromic group, or a silyl group having, as a substituent, an alkyl group, an alkoxy group, or an aryl group,
   L¹ is a group represented by formula (X2) below, 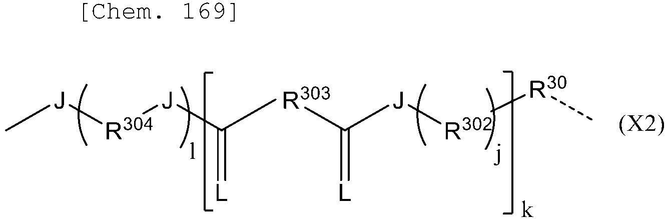 in formula (X2) above, R³⁰ is a group represented by formula (X2a) below, 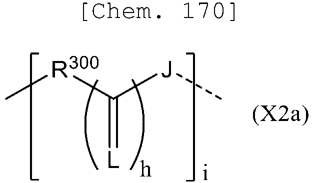 in formulas (X2) and (X2a) above,
   J represents a divalent group, each independently being directly bonded, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group or a silylene group having an alkyl group or an aryl group as a substituent,
   R³⁰², R³⁰³, and R³⁰⁴ are each independently an alkylene group,
   h, j, k, and 1 are each independently an integer of 0 or 1,
   i is an integer of 1 to 200,
   when i is 2 or more, the structures of a plurality of R³⁰ groups may be the same as or different from each other, and the dashed lines indicate bonds with R⁴⁰⁰,
   d1 is an integer of 1 to 5, when d1 is 1, R^{9a} is a group represented by formula (1a) above,
   when d1 is 2 to 5, at least one of a plurality of R^{9a} groups is a group represented by formula (1a) above, and the plurality of R^{9a} groups may be the same as or different from each other, when there are R^{9a} groups adjacent to each other, except for the group represented by formula (1a) above, the two adjacent R^{9a} groups, together with carbon atoms to which the R^{9a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   R^{10a} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   e1 is an integer of 0 to 5,
   when e1 is 2 to 5, the plurality of R^{10a} may be the same as or different from each other, and
   when there are a R^{10a} groups adjacent to each other, the two adjacent R^{10a} groups, together with carbon atoms to which the R^{10a} groups bind, may constitute a substituted or unsubstituted aliphatic ring, a substituted or unsubstituted aliphatic
   heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[19] The propargyl alcohol compound as described in [18], in which the propargyl alcohol compound is represented by formula (11) below: 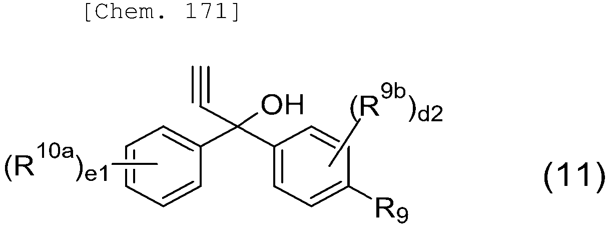 in formula (11) above, R^{10a} and e1 are the same as those in formula (10) above,
   R⁹ is a group represented by formula (1a) above,
   R^{9b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   d2 is an integer of 0 to 4, and
   when d2 is 2 to 4, a plurality of R^{9b} groups may be the same as or different from each other, and when there are R^{9b} groups adjacent to each other, the two adjacent R^{9b} groups, together with carbon atoms to which the R^{9b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[20] The propargyl alcohol compound as described in [19], in which the propargyl alcohol compound is represented by formula (12) below: 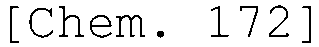 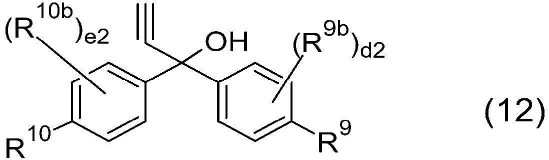 in formula (12) above, R⁹, R^{9b}, and d2 are the same as those in formula (11) above,
   R¹⁰ is an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a substituted or unsubstituted silyl group, a substituted or unsubstituted oxysilyl group, a group represented by formula (2a) above, or a group represented by formula (X3) above,
   R^{10b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
   e2 is an integer of 0 to 4,
   when e2 is 2 to 4, a plurality of R^{10b} groups may be the same as or different from each other, and
   when there are R^{10b} groups adjacent to each other, the two adjacent R^{10b} groups, together with carbon atoms to which the R^{10b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[21] The propargyl alcohol compound as described in any one of [17] to [21], in which R¹ is a substituted or unsubstituted phenyl group, R² is a substituted or unsubstituted alkyl group having 1 or more and 10 or less carbon atoms, a substituted or unsubstituted cycloalkyl group having 5 or more and 10 or less carbon atoms, a substituted or unsubstituted haloalkyl group having 1 or more and 10 or less carbon atoms, or a group represented by formula (2a) above.

## Claims

1. A photochromic compound having a skeleton represented by formula (1) below: 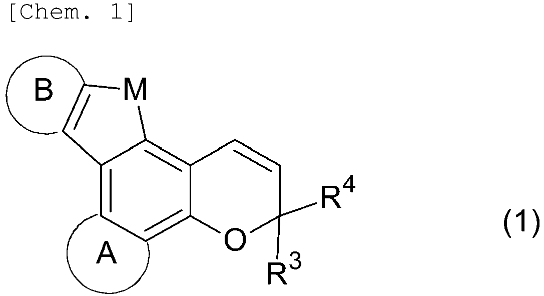 wherein, in formula (1) above,
M is C, Si, or Ge,
R³ is an aryl group or a heteroaryl group substituted with a group represented by formula (1a) below, 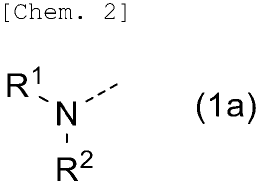
in formula (1a) above,
R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below,
-Q¹-(X¹Q²)a-X²Q³ (2a)
in formula (2a) above,
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰, pR⁷⁰¹, or P(=O),
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
a is an integer of 0, or 1 or more and 3 or less,
R⁴ is a substituted or unsubstituted aryl group or a substituted or unsubstituted heteroaryl group, and
ring A and ring B are each independently a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

2. The photochromic compound according to claim 1, wherein the photochromic compound has a skeleton represented by formula (2) below: 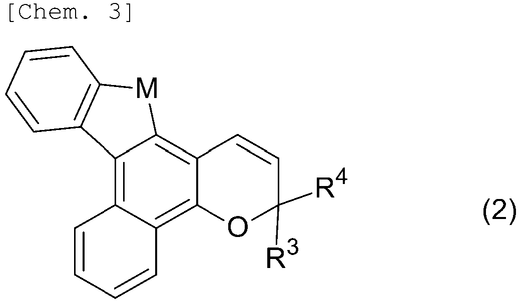 wherein, in formula (2) above, M, R³, and R⁴ are each the same as those in formula (1) above.

3. The photochromic compound according to claim 1 or 2, wherein the photochromic compound has a skeleton represented by formula (3) below: 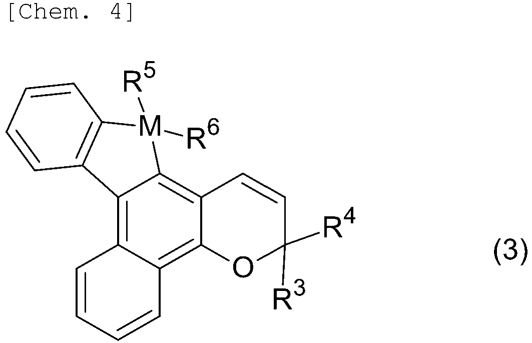
wherein, in formula (3) above, M, R³, and R⁴ are each the same as those in formula (1) above,
R⁵ and R⁶ are each independently a hydrogen atom, a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) below, 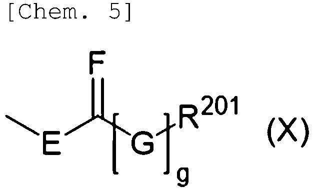
in formula (X),
E is an oxygen atom or NR¹⁰¹, and R¹⁰¹ is a hydrogen atom or an alkyl group,
F is an oxygen atom or a sulfur atom,
G is an oxygen atom, a sulfur atom, or NR²⁰², R²⁰² is a hydrogen atom, an alkyl group, a cycloalkyl group, an aryl group, or a heteroaryl group,
g is 0 or 1,
R²⁰¹ is a hydrogen atom, an alkyl group, a cycloalkyl group, an aryl group, or a heteroaryl group,
when G is an oxygen atom or a sulfur atom, R²⁰¹ is a group other than a hydrogen atom,
L¹-R⁴⁰⁰ (X3)
in formula (X3) above,
R⁴⁰⁰ is a hydrogen atom, an alkyl group, an aryl group, a polymerizable group, a photochromic group, or a silyl group having, as a substituent, an alkyl group, an alkoxy group, or an aryl group,
L¹ is a group represented by formula (X2) below, 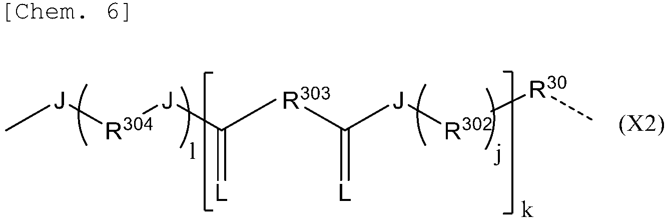
in formula (X2) above, R³⁰ is a group represented by formula (X2a) below, 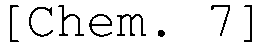 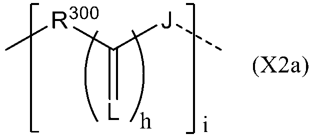
in formulas (X2) and (X2a) above,
J represents a divalent group, each independently being directly bonded, a substituted or unsubstituted methylene group, an oxygen atom, a sulfur atom, or NR³⁰¹, and R³⁰¹ is a hydrogen atom or an alkyl group,
L is an oxygen atom or a sulfur atom,
R³⁰⁰ is an alkylene group or a silylene group having an alkyl group or an aryl group as a substituent,
R³⁰², R³⁰³, and R³⁰⁴ are each independently an alkylene group,
h, j, k, and 1 are each independently an integer of 0 or 1,
i is an integer of 1 to 200, and when i is 2 or more, the plurality of R³⁰ groups may be the same as or different from each other, and the dashed lines indicate bonds with R⁴⁰⁰, and
R⁵ and R⁶, together with M, may constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring.

4. The photochromic compound according to claim 3, wherein R⁵ and R⁶ are each independently a hydrogen atom, a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (X) above, or a group represented by formula (X3) above.

5. The photochromic compound according to claim 3, wherein the photochromic compound is represented by formula (4) below: 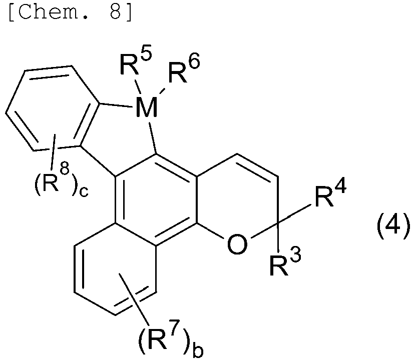
wherein, in formula (4) above, R³, R⁴, R⁵, R⁶, and M are each the same as those in formula (3) above,
R⁷ and R⁸ are each independently a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted haloalkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) above, or a group represented by formula (X3) above,
b is an integer of 0 to 4,
c is an integer of 0 to 4,
when b is 2 to 4, a plurality of R⁷ groups may be the same as or different from each other,
when c is 2 to 4, a plurality of R⁸ groups may be the same as or different from each other,
when b is 2 to 4 and there are R⁷ groups adjacent to each other, the two adjacent R⁷ groups, together with carbon atoms to which the R⁷ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, and
when c is 2 to 4 and there are R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

6. The photochromic compound according to claim 5, wherein the photochromic compound is represented by formula (5) below: 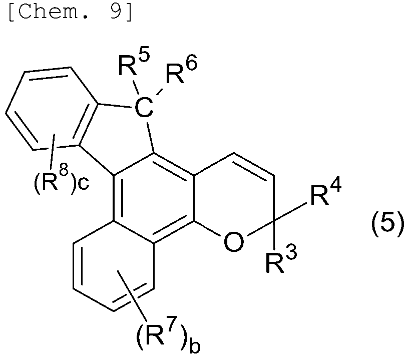 wherein, in formula (5) above, R³, R⁴, R⁵, R⁶, R⁷, R⁸, b, and c are each the same as those in formula (4) above.

7. The photochromic compound according to claim 6, wherein the photochromic compound is represented by formula (6) below: 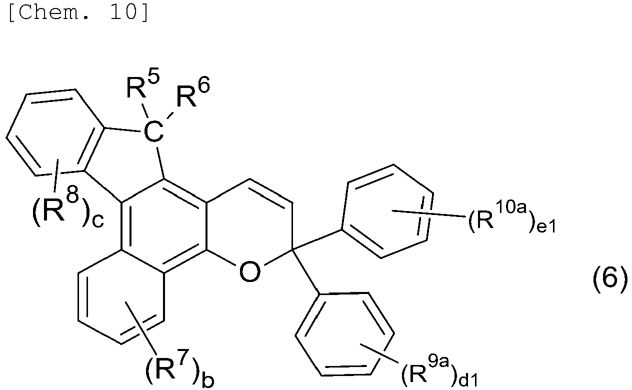
in formula (6) above, R⁵, R⁶, R⁷, R⁸, b, and c are each the same as those in formula (5) above,
R^{9a} is a group represented by formula (1a) above, a hydrogen atom, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
d1 is an integer of 1 to 5,
when d1 is 1, R^{9a} is a group represented by formula (1a) above,
when d1 is 2 to 5, at least one of a plurality of R^{9a} groups is a group represented by formula (1a) above, and the plurality of R^{9a} groups may be the same as or different from each other,
when there are R^{9a} groups adjacent to each other, except for the group represented by formula (1a) above, the two adjacent R^{9a} groups, together with carbon atoms to which the R^{9a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
R^{10a} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
e1 is an integer of 0 to 5,
when e1 is 2 to 5, the plurality of R^{10a} may be the same as or different from each other, and when there are R^{10a} groups adjacent to each other, the two adjacent R^{10a} groups, together with carbon atoms to which the R^{10a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

8. The photochromic compound according to claim 7, wherein the photochromic compound is represented by formula (7) below: 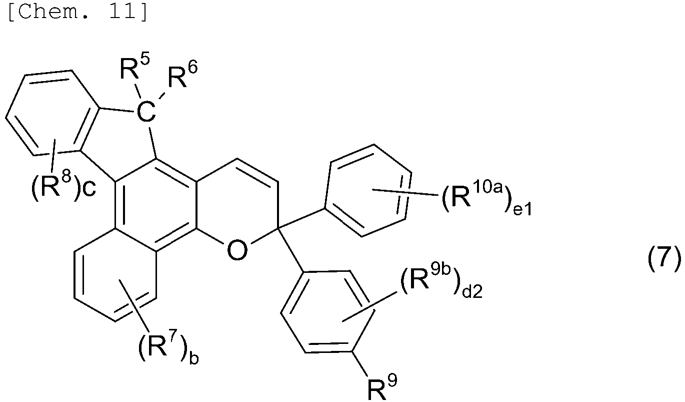
in formula (7) above, R⁵, R⁶, R⁷, R⁸, R^{10a}, b, c, and e1 are each the same as those in formula (6) above,
R⁹ is a group represented by formula (1a) above,
R^{9b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
d2 is an integer of 0 to 4,
when d2 is 2 to 4, a plurality of R^{9b} groups may be the same as or different from each other, and
when there are R^{9b} groups adjacent to each other, the two adjacent R^{9b} groups, together with carbon atoms to which the R^{9b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

9. The photochromic compound according to claim 8, wherein the photochromic compound is represented by formula (8) below: 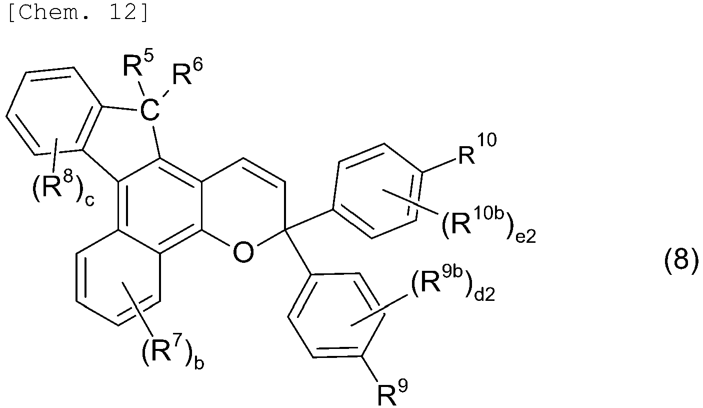
in formula (8) above, R⁵, R⁶, R⁷, R⁸, R⁹, R^{9b}, b, c, and d2 are each the same as those in formula (7) above,
R¹⁰ is an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a substituted or unsubstituted silyl group, a substituted or unsubstituted oxysilyl group, a group represented by formula (2a) above, or a group represented by formula (X3) above,
R^{10b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
e2 is an integer of 0 to 4,
when e2 is 2 to 4, the plurality of R^{10b} groups may be the same as or different from each other, and
when there are R^{10b} groups adjacent to each other, the two adjacent R^{10b} groups, together with carbon atoms to which the R^{10b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

10. The photochromic compound according to claim 1, wherein R¹ is a substituted or unsubstituted phenyl group.

11. The photochromic compound according to claim 1, wherein R² is a substituted or unsubstituted alkyl group having 1 or more and 10 or less carbon atoms, a substituted or unsubstituted cycloalkyl group having 5 or more and 10 or less carbon atoms, a substituted or unsubstituted haloalkyl group having 1 or more and 10 or less atoms, or a group represented by formula (2a) above.

12. A curable composition comprising the photochromic compound according to claim 1, and at least one selected from a group consisting of a radical polymerizable monomer, a cationic polymerizable monomer, a compound having a polymerizable group, and a (thio)urethane(urea) polymer.

13. A cured product of the curable composition according to claim 12.

14. An optical article comprising the cured product according to claim 13.

15. A lens comprising the photochromic compound according to claim 1.

16. Eyeglasses comprising the lens according to claim 15.

17. A propargyl alcohol compound represented by formula (9) below; 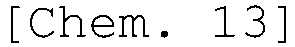 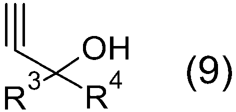 wherein, in formula (9) above,
R³ is an aryl group or heteroaryl group substituted with a group represented by formula (1a) below, 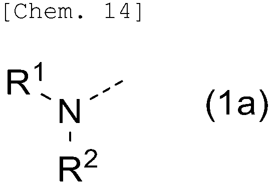
in formula (1a) above,
R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
R² is a hydrogen atom, a halogen atom, a substituted or unsubstituted alkyl group, a substituted or unsubstituted haloalkyl group, a substituted or unsubstituted cycloalkyl group, a substituted or unsubstituted alkenyl group, a substituted or unsubstituted alkynyl group, a substituted or unsubstituted alkoxy group, a substituted or unsubstituted aryloxy group, a substituted or unsubstituted alkylthio group, a substituted or unsubstituted arylthio group, a substituted or unsubstituted aralkyl group, or a group represented by formula (2a) below,
-Q¹-(X¹Q²)a-X²Q³ (2a)
in formula (2a) above,
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰, PR⁷⁰¹, or P(=O),
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
a is an integer of 0, or 1 or more and 3 or less, and
R⁴ is a substituted or unsubstituted aryl group or a substituted or unsubstituted heteroaryl group.

18. The propargyl alcohol compound according to claim 17, wherein the propargyl alcohol compound is represented by formula (10) below: 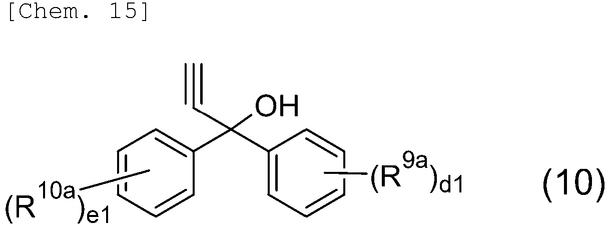 in formula (10) above,
R^{9a} is a group represented by formula (1a) above, a hydrogen atom, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
L¹-R⁴⁰⁰ (X3)
in formula (X3) above,
R⁴⁰⁰ is a hydrogen atom, an alkyl group, an aryl group, a polymerizable group, a photochromic group, or a silyl group having, as a substituent, an alkyl group, an alkoxy group, or an aryl group,
L¹ is a group represented by formula (X2) below, 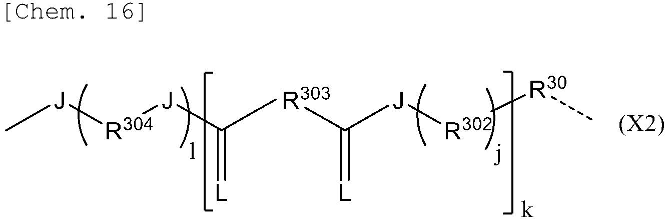
in formula (X2) above, R³⁰ is a group represented by formula (X2a) below, 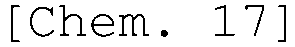 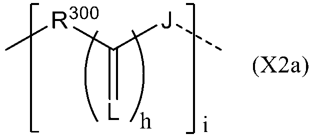
in formulas (X2) and (X2a) above,
J represents a divalent group, each independently being directly bonded, a substituted or unsubstituted methylene group, an oxygen atom, a sulfur atom, or NR³⁰¹, and R³⁰¹ is a hydrogen atom or an alkyl group,
L is an oxygen atom or a sulfur atom,
R³⁰⁰ is an alkylene group or a silylene group having an alkyl group or an aryl group as a substituent,
R³⁰², R³⁰³, and R³⁰⁴ are each independently an alkylene group,
h, j, k, and l are each independently an integer of 0 or 1,
i is an integer of 1 to 200,
when i is 2 or more, the structures of a plurality of R³⁰ groups may be the same as or different from each other, and the dashed lines indicate bonds with R⁴⁰⁰,
d1 is an integer of 1 to 5,
when d1 is 1, R^{9a} is a group represented by formula (1a) above,
when d1 is 2 to 5, at least one of the plurality of R^{9a} groups is a group represented by formula (1a) above, and the plurality of R^{9a} groups may be the same as or different from each other,
when there are R^{9a} groups adjacent to each other, except for the group represented by formula (1a) above, the two adjacent R^{9a} groups, together with carbon atoms to which the R^{9a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
R^{10a} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
e1 is an integer of 0 to 5,
when e1 is 2 to 5, the plurality of R^{10a} may be the same as or different from each other, and
when there are R^{10a} groups adjacent to each other, the two adjacent R^{10a} groups, together with carbon atoms to which the R^{10a} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

19. The propargyl alcohol compound according to claim 18, wherein the propargyl alcohol compound is represented by formula (11) below: 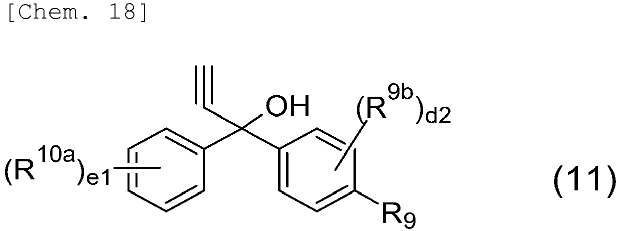
in formula (11) above, R^{10a} and e1 are the same as those in formula (10) above,
R⁹ is a group represented by formula (1a) above,
R^{9b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
d2 is an integer of 0 to 4,
when d2 is 2 to 4, R^{9b} groups may be the same as or different from each other, and
when there are R^{9b} groups adjacent to each other, the two adjacent R^{9b} groups, together with carbon atoms to which the R^{9b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

20. The propargyl alcohol compound according to claim 19, wherein the propargyl alcohol compound is represented by formula (12) below: 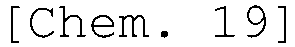 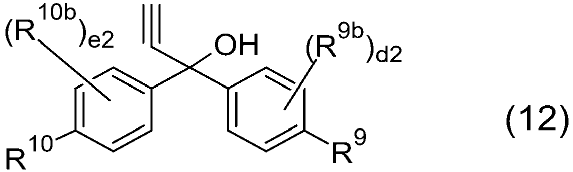
in formula (12) above, R⁹, R^{9b}, and d2 are the same as those in formula (11) above,
R¹⁰ is an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a substituted or unsubstituted silyl group, a substituted or unsubstituted oxysilyl group, a group represented by formula (2a) above, or a group represented by formula (X3) above,
R^{10b} is a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
e2 is an integer of 0 to 4,
when e2 is 2 to 4, a plurality of R^{10b} groups may be the same as or different from each other, and
when there are R^{10b} groups adjacent to each other, the two adjacent R^{10b} groups, together with carbon atoms to which the R^{10b} groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

21. The propargyl alcohol compound according to claim 17, wherein R¹ is a substituted or unsubstituted phenyl group, and R² is a substituted or unsubstituted alkyl group having 1 or more and 10 or less carbon atoms, a substituted or unsubstituted cycloalkyl group having 5 or more and 10 or less carbon atoms, a substituted or unsubstituted haloalkyl group having 1 or more and 10 or less carbon atoms, or a group represented by formula (2a) above.
